# EUROPEAN PATENT APPLICATION

(11) **EP 1 350 847 A1**
(43) Date of publication of application: **08.10.2003**
(21) Application number: 01273171.7
(22) Date of filing: 30.11.2001
(51) Int. Cl.: C12N 15/12, C12N 5/10, A01K 67/027, A61K 31/711, A61K 38/00, A61K 39/00, A61K 39/395, A61K 45/00, A61K 48/00, A61P 35/00, C07K 14/47, C07K 16/18, C07K 19/00, C12Q 1/02, C12Q 1/68

(54) **HUMAN GLIOMA ANTIGEN AND PROCESS FOR PREPARING THE SAME**

(30) Priority: 09.01.2001 JP 2001001965
(71) Applicant: Keio University, Tokyo 108-8345 (JP)
(72) Inventor: TODA, Masahiro, Yokohama-shi, Kanagawa 227-0043 (JP); KAWAKAMI, Yutaka, Yokohama-shi, Kanagawa 221-0861 (JP); KAWASE, Takeshi, Ichikawa-shi, Chiba 272-0804 (JP); IIZUKA, Yukihiko, Suginami-ku, Tokyo 166-0002 (JP)
(74) Representative: Williams, Aylsa
(86) International application number: JP0110505
(87) International publication number: WO02055695

(57) **Abstract**

The present invention provides a human glioma antigen or a method for preparing the same, which can be applied to diagnosis and therapy of glioma. A glioma antigen or a glioma antigen gene is prepared by a process comprising the steps of: total RNA is extracted from a glioma cell line and cDNA was synthesized; λphage cDNA library, which was constructed by infection of E. coli caused by introducing said cDNA into λ phage vector, and the sera of glioma patients are made to react; positive clones to which antibodies in the serum reacted are detected with the use of labeled anti-IgG antibodies; screening is repeated several times for the detected positive clones; antigens are isolated from positive clones confirmed to be antibody-reactive; serum screening is conducted by using the isolated antigens, the sera of glioma patients and healthy persons.

SEQUENCE LISTING

<110> KEIO UNIVERSITY

<120> Human Brain Tumor Antigens and method for preparation

<130> P00000114

<140>
<141>

<150> JP 2001-001965
<151> 2001-01-09

<160> 20

<170> PatentIn Ver. 2.1

<210> 1
<211> 3709
<212> DNA
<213> Homo sapiens

<220>
<221> CDS
<222> (170)..(3532)

<400> 1
ggcacgagtc tatctcatca ataaacattc actgggttct tggtttaagg ggtttgagaa 60
gcttgccacc tcccagcgtt tattattatt tttttctttt gccccgacct ttagcctctg 120
ggccatttgt aatacatatt taatttttag tcacaggaaa taagtaatc atg gtg ctc 178
aac agt ttg gat aag atg att caa ctc cag aaa aac act gcc aac atc 226
agg aat att tgt gtt ttg gct cat gtt gac cat gga aaa act act ctg 274
gct gac tgt ctt ata tct agc aat gga atc atc tcc agc cgc cta gca 322
ggc aag tta agg tac atg gac agc aga gaa gat gaa cag atc cga ggg 370
atc act atg aaa tcc agt gcc att tcc cta cat tat gca aca ggt aat 418
gag gag tac ctg atc aat ctg ata gac tct cca gga cac gtg gac ttt 466
tcc tca gaa gta tca acc gct gtt cgc att tgt gat gga tgc atc att 514
gtg gta gat gct gtg gaa gga gtc tgt cca cag aca cag gca gtt ctg 562
cga caa gct tgg ctt gaa aac atc cgt ccg gtt tta gtg att aat aag 610
att gat cgc ttg ata gtg gaa ctg aaa ttc acc cca caa gag gcc tat 658
tct cac ctc aag aat att tta gaa cag att aat gcg ctc aca ggg act 706
ctt ttt act tct aaa gtc cta gaa gaa aga gca gag agg gag act gaa 754
tcc caa gtg aat cca aat tct gaa caa gga gag caa gta tat gac tgg 802
agc act ggc ttg gag gac aca gat gat tct cac ctt tac ttc tct cca 850
gaa cag gga aat gtg gtg ttt acc agt gca ata gat ggg tgg ggc ttt 898
gga att gag cac ttc gcc aga atc tac agt caa aaa att ggc atc aaa 946
aag gaa gtt ctt atg aaa acc ttg tgg gga gat tac tat ata aat atg 994
aag gct aaa aag atc atg aag ggt gat cag gcc aaa gga aag aaa cct 1042
tta ttt gta cag ttg atc ctg gaa aat ata tgg agt ttg tat gat gct 1090
gtt ttg aaa aag gac aaa gac aaa att gat aaa ata gtg act tct tta 1138
gga tta aaa att gga gcc cgg gag gca cga cat tca gac cct aaa gtt 1186
cag atc aac gcc att tgc agt cag tgg cta ccc ata tcc cat gct gtt 1234
ctt gct atg gtg tgt cag aaa ctt cct agt ccc ctt gat att aca gct 1282
gag aga gtg gag aga ctg atg tgc aca gga tca caa act ttt gac tct 1330
ttt cca cca gaa act caa gca ctg aaa gca gct ttt atg aaa tgt gga 1378
agt gag gac act gct cca gtt att ata ttt gtt tcc aaa atg ttt gca 1426
gtt gat gct aag gcc ttg cct cag aat aag cca agg cct ctc act caa 1474
gaa gaa att gct cag aga cgt gag cgt gca aga caa agg cat gca gag     1522
aag ctt gca gca gca cag gga cag gca ccc ttg gag ccc acc caa gat     1570
ggg agt gcc att gaa aca tgt cca aaa gga gac gag cca aga ggt gac     1618
gag caa cag gtg gaa agt atg acc cct aaa cct gtg ctc cag gaa gaa     1666
aac aac caa gag tct ttt att gca ttt gct cgg gtg ttc agt ggt gtg     1714
gct cga aga gga aag aaa att ttt gtc ttg ggg ccc aaa tac agt cct     1762
ctt gag ttt tta cga agg gta cca tta ggc ttc tca gct cca cca gat     1810
ggc ctc ccc caa gtc ccc cac atg gca tac tgt gct ctg gaa aac ctg     1858
tat ctt ctg atg gga agg gaa ctg gaa tat cta gag gag gta cct cca     1906
gga aat gtg cta gga ata gga ggc ctt caa gat ttt gtg ctg aaa tct     1954
gca aca ctg tgt agc ctg cca tcc tgc cca cca ttt ata cca ctc aac     2002
ttc gaa gcc act cct att gtg aga gtt gct gtt gaa cca aaa cat cca 2050
agt gaa atg cct cag ctc gta aaa gga atg aaa ctg tta aac cag gct 2098
gat ccc tgt gtc cag att tta att cag gaa acg gga gag cac gtt tta 2146
gtc aca gca gga gaa gtc cac ctt cag cga tgc ctg gat gac tta aaa 2194
gaa agg ttt gca aag att cat atc agt gta tct gaa cct att att cca 2242
ttc aga gaa aca atc aca aaa ccc cca aaa gtt gac atg gtc aat gaa 2290
gaa ata ggc aaa cag caa aaa gtt gca gtc ata cac caa atg aaa gaa 2338
gat caa agc aaa atc cct gaa gga atc caa gtt gac tct gac ggg cta 2386
atc acc ata aca act ccc aat aaa ctt gcc acg ctc agt gtt cga gcc 2434
atg ccc ctt cca gaa gaa gtc acc cag att ctg gaa gaa aat agt gat 2482
ttg att cgt tct atg gag cag ttg aca tcc tct ttg aat gag ggt gaa 2530
aat act cac atg att cat cag aag acc caa gag aaa att tgg gaa ttc 2578
aaa gga aaa ctg gag caa cac cta aca ggg aga aga tgg agg aac att 2626
gtt gac caa atc tgg tca ttt ggc cca aga aaa tgt ggg ccc aac ata 2674
cta gtc aat aaa agt gaa gat ttt cag aac tca gta tgg aca ggt cca 2722
gct gac aaa gct tca aaa gaa gcc agt aga tac cga gat ttg ggc aat 2770
agc att gtg agt ggc ttc caa cta gca acc ctc tct ggc ccc atg tgt 2818
gag gag cct ctc atg ggt gtc tgt ttt gtt ctg gaa aaa tgg gac cta 2866
agt aaa ttt gag gaa caa gga gca agt gat ctg gca aaa gag gga cag 2914
gag gaa aat gaa acc tgt tct ggt gga aat gaa aac caa gag cta caa 2962
gat ggc tgc tct gag gcc ttt gag aag agg aca tca cag aaa gga gaa 3010
tct cca ctc act gac tgc tat gga cct ttc tca gga cag cta att gcc 3058
acc atg aaa gaa gca tgt cgc tat gca ctg caa gtg aaa cct cag cgc 3106
ctg atg gca gct atg tac aca tgt gac atc atg gcc act ggt gat gtt 3154
ctc ggt cga gtc tat gct gtc ttg tca aag aga gaa ggt cgg gta ctt     3202
caa gaa gaa atg aaa gaa ggg aca gac atg ttc atc atc aag gct gtg     3250
ctg cct gtt gct gaa agc ttt ggt ttt gct gat gaa atc agg aag agg     3298
aca agt ggc ctg gcc agc cca caa cta gta ttc agc cat tgg gag atc     3346
att ccc agt gac ccc ttc tgg gtg cca act act gag gag gaa tac ttg     3394
cac ttt ggg gag aag gct gac tct gag aac caa gcc cgg aag tac atg     3442
aac gca gta cga aag cgg aag ggg ctt tat gtg gaa gaa aag att gtg     3490
gag cat gca gaa aag cag agg aca ctc agc aaa aat aag tag             3532
ctacctacta ctggtggatt cttttcctta tagtgaattt aaaagtatca tcaaggtttt 3592
aatattggga aaatttcttt ttgccacatt atctctgttt attcactttc aataaagttg 3652
atccatataa atattttaaa aaaaaaaaaa aaaaaaaaaa aaaaaaaaaa aaaaaaa    3709

<210> 2
<211> 1120
<212> PRT
<213> Homo sapiens

<400> 2

<210> 3
<211> 3533
<212> DNA
<213> Homo sapiens

<220>
<221> CDS
<222> (14)..(3478)

<400> 3
   ggcacgagcg gtc atg gag gcg ggc gcc gga gcc ggc gcg gga gcc gcg     49
                  Met Glu Ala Gly Ala Gly Ala Gly Ala Gly Ala Ala
                   1               5                   10
   ggc tgg agc tgc ccg ggc cca gga ccc aca gtg acc act cta ggc tcc     97
   Gly Trp Ser Cys Pro Gly Pro Gly Pro Thr Val Thr Thr Leu Gly Ser
           15                  20                  25
   tat gag gct tcc gag ggc tgt gag agg aag aag ggc caa cgc tgg ggg     145
   Tyr Glu Ala Ser Glu Gly Cys Glu Arg Lys Lys Gly Gln Arg Trp Gly
       30                  35                  40
   tcc ctg gaa cga cgg ggg atg caa gct atg gag ggg gag gtg tta ctc     193
   Ser Leu Glu Arg Arg Gly Met Gln Ala Met Glu Gly Glu Val Leu Leu
   45                  50                  55                  60
   cca gct ctc tat gag gag gaa gag gaa gag gaa gag gag gaa gaa gag     241
   Pro Ala Leu Tyr Glu Glu Glu Glu Glu Glu Glu Glu Glu Glu Glu Glu
                   65                  70                  75
   gtg gaa gaa gaa gaa gaa caa gtg cag aaa ggt ggc agt gtt ggc tct     289
   Val Glu Glu Glu Glu Glu Gln Val Gln Lys Gly Gly Ser Val Gly Ser
               80                  85                  90
   ctg tca gtc aac aag cac cgg gga ctg agc ctc acg gag aca gag ctg     337
   Leu Ser Val Asn Lys His Arg Gly Leu Ser Leu Thr Glu Thr Glu Leu
           95                  100                 105
   gag gag ctg cgg gct cag gtg ctg cag ctg gtg gca gaa ctg gag gag     385
   Glu Glu Leu Arg Ala Gln Val Leu Gln Leu Val Ala Glu Leu Glu Glu
       110                 115                 120
   acc cgg gaa ctg gca ggg cag cat gag gat gac tcc ttg gag cta cag     433
   Thr Arg Glu Leu Ala Gly Gln His Glu Asp Asp Ser Leu Glu Leu Gln
   125                 130                 135                 140
   ggg ctc ctg gag gat gaa cgg cta gcc agc gcc cag cag gca gag gtg 481
   Gly Leu Leu Glu Asp Glu Arg Leu Ala Ser Ala Gln Gln Ala Glu Val
   145 150 155
   ttc acc aag cag atc cag cag ctc caa ggt gag ctg cgt tct cta cgg 529
   Phe Thr Lys Gln Ile Gln Gln Leu Gln Gly Glu Leu Arg Ser Leu Arg
   160 165 170
   gag gag att tcc ctg tta gag cat gag aaa gaa agc gaa ctt aag gaa 577
   Glu Glu Ile Ser Leu Leu Glu His Glu Lys Glu Ser Glu Leu Lys Glu
   175 180 185
   ata gaa cag gaa ttg cat ttg gcc cag gct gag atc cag agt ctg cgg 625
   Ile Glu Gln Glu Leu His Leu Ala Gln Ala Glu Ile Gln Ser Leu Arg
   190 195 200
   caa gca gca gag gat tcc gca act gaa cat gag agt gac ata gca tcc 673
   Gln Ala Ala Glu Asp Ser Ala Thr Glu His Glu Ser Asp Ile Ala Ser
   205 210 215 220
   ctg cag gag gat ctc tgc cgg atg cag aat gaa ctt gaa gac atg gaa 721
   Leu Gln Glu Asp Leu Cys Arg Met Gln Asn Glu Leu Glu Asp Met Glu
   225 230 235
   cgc att cgg gga gat tat gag atg gag atc gcc tcc ctc cgt gca gaa 769
   Arg Ile Arg Gly Asp Tyr Glu Met Glu Ile Ala Ser Leu Arg Ala Glu
   240 245 250
   atg gaa atg aag agc tct gaa cca tcc ggt agt tta ggt ctc tca gat 817
   Met Glu Met Lys Ser Ser Glu Pro Ser Gly Ser Leu Gly Leu Ser Asp
   255 260 265
   tac tct ggg tta caa gaa gaa ctg cag gag ctg cgg gaa cgc tac cat 865
   Tyr Ser Gly Leu Gln Glu Glu Leu Gln Glu Leu Arg Glu Arg Tyr His
   270 275 280
   ttc ctg aat gag gaa tac cgg gcc ctg cag gag agc aac agc agc ctc 913
   Phe Leu Asn Glu Glu Tyr Arg Ala Leu Gln Glu Ser Asn Ser Ser Leu
   285 290 295 300
   acg ggg cag ctt gca gat ctg gag agt gag agg aca cag aga gca aca 961
   Thr Gly Gln Leu Ala Asp Leu Glu Ser Glu Arg Thr Gln Arg Ala Thr
   305 310 315
   gag aga tgg ctg cag tcc caa aca ctg agt atg acg tca gca gag tct 1009
   Glu Arg Trp Leu Gln Ser Gln Thr Leu Ser Met Thr Ser Ala Glu Ser
   320 325 330
   cag act tca gaa atg gat ttc tta gag cct gat cct gaa atg cag ttg 1057
   Gln Thr Ser Glu Met Asp Phe Leu Glu Pro Asp Pro Glu Met Gln Leu
   335 340 345
   tta cgg cag cag cta cgg gat gct gaa gag cag atg cat ggc atg aag 1105
   Leu Arg Gln Gln Leu Arg Asp Ala Glu Glu Gln Met His Gly Met Lys
   350 355 360
   aac aag tgt cag gaa ttg tgt tgt gag ttg gaa gag cta cag cat cat 1153
   Asn Lys Cys Gln Glu Leu Cys Cys Glu Leu Glu Glu Leu Gln His His
   365 370 375 380
   cgc cag gtc agt gag gag gag cag agg cgg ctg cag agg gag ctc aag 1201
   Arg Gln Val Ser Glu Glu Glu Gln Arg Arg Leu Gln Arg Glu Leu Lys
   385 390 395
   tgt gct cag aat gag gtg ctt cgg ttt cag acc tcc cac agt gtc acc 1249
   Cys Ala Gln Asn Glu Val Leu Arg Phe Gln Thr Ser His Ser Val Thr
   400 405 410
   cag gag tta ctg tgc cgg ctg cag aag ctg cac ctc cag cac cag aac 1297
   Gln Glu Leu Leu Cys Arg Leu Gln Lys Leu His Leu Gln His Gln Asn
   415 420 425
   gtc aca tgt gag aag gaa aag ctg ctg gaa cgg cag cag cag ctg cag 1345
   Val Thr Cys Glu Lys Glu Lys Leu Leu Glu Arg Gln Gln Gln Leu Gln
   430 435 440
   gag gag ctg cag tgc cat gag gca gag ctg cag cac ctc agg gat acg 1393
   Glu Glu Leu Gln Cys His Glu Ala Glu Leu Gln His Leu Arg Asp Thr
   445 450 455 460
   gtg gcc tcc ttc aaa gag agc aat gag aag gac aca gag acg cac gct 1441
   Val Ala Ser Phe Lys Glu Ser Asn Glu Lys Asp Thr Glu Thr His Ala
   465 470 475
   cag ctt cag gag atg aag cag ctg tac cag gcc agc aag gac gag ctg 1489
   Gln Leu Gln Glu Met Lys Gln Leu Tyr Gln Ala Ser Lys Asp Glu Leu
   480 485 490
   gag cgg cag aag cac atg tat gac cag ctg gag cag gac ctc ctg ctc 1537
   Glu Arg Gln Lys His Met Tyr Asp Gln Leu Glu Gln Asp Leu Leu Leu
   495 500 505
   tgc cag ctg gag ctg aaa gag ctc aag gcc tcc cac ccc att ccg gag 1585
   Cys Gln Leu Glu Leu Lys Glu Leu Lys Ala Ser His Pro Ile Pro Glu
   510 515 520
   gac aaa gga aag tgt gct aat aag tgt gac aca ctg ctg tcc aga ctg 1633
   Asp Lys Gly Lys Cys Ala Asn Lys Cys Asp Thr Leu Leu Ser Arg Leu
   525 530 535 540
   aca gaa ttg cag gaa aag tac aag gcc agc cag aag gag atg ggg cag 1681
   Thr Glu Leu Gln Glu Lys Tyr Lys Ala Ser Gln Lys Glu Met Gly Gln
   545 550 555
   ctg cag atg gag cag tgt gag ctc ctg gag gat cag agg agg atg cag 1729
   Leu Gln Met Glu Gln Cys Glu Leu Leu Glu Asp Gln Arg Arg Met Gln
   560 565 570
   gag gag cag ggc cag ctg cag gaa gag ctg cac agg ctc aca ctg cca 1777
   Glu Glu Gln Gly Gln Leu Gln Glu Glu Leu His Arg Leu Thr Leu Pro
   575 580 585
   ctg cca aag agt ggc ctc tta ctc aag agt cag gag cta ctc acc aag 1825
   Leu Pro Lys Ser Gly Leu Leu Leu Lys Ser Gln Glu Leu Leu Thr Lys
   590 595 600
   tta gaa gac ctg tgt gag ctg cag ctg ctc tac caa ggc atg cag gag 1873
   Leu Glu Asp Leu Cys Glu Leu Gln Leu Leu Tyr Gln Gly Met Gln Glu
   605 610 615 620
   gaa cag aag aag ctg ata cag aac caa gac tgt gta tta aaa gaa caa 1921
   Glu Gln Lys Lys Leu Ile Gln Asn Gln Asp Cys Val Leu Lys Glu Gln
   625 630 635
   tta gag atc cac gaa gag ctg cga cgt ttc aaa gag tct cat ttc cag 1969
   Leu Glu Ile His Glu Glu Leu Arg Arg Phe Lys Glu Ser His Phe Gln
   640 645 650
   gaa gtg ttg gag aat ccc gat gat tcc aaa ttg gct aag tcc tcc aaa 2017
   Glu Val Leu Glu Asn Pro Asp Asp Ser Lys Leu Ala Lys Ser Ser Lys
   655 660 665
   tgt aat cga aac aag caa tcc aag ctg ctc atg gag cag atg cag gcc 2065
   Cys Asn Arg Asn Lys Gln Ser Lys Leu Leu Met Glu Gln Met Gln Ala
   670 675 680
   ctg cag gtg atg tat gac gcc ggt cag gcg aag cag gag ctc ttg cag 2113
   Leu Gln Val Met Tyr Asp Ala Gly Gln Ala Lys Gln Glu Leu Leu Gln
   685 690 695 700
   caa gag caa ggg agg ctc cta gag gag cgg aag agg ctg cag gca gac 2161
   Gln Glu Gln Gly Arg Leu Leu Glu Glu Arg Lys Arg Leu Gln Ala Asp
   705 710 715
   ttg cag ctc tgc ctg gaa gaa atg cag ctg ctt caa gtc cag tcc cct 2209
   Leu Gln Leu Cys Leu Glu Glu Met Gln Leu Leu Gln Val Gln Ser Pro
   720 725 730
   tct ata aaa atg agc ctt gag tcc tac ggg aag agc tat ggt agc atg 2257
   Ser Ile Lys Met Ser Leu Glu Ser Tyr Gly Lys Ser Tyr Gly Ser Met
   735 740 745
   gtc ccc agc aat gag aac tgt cgc aag act tat gat acc act gtg gat 2305
   Val Pro Ser Asn Glu Asn Cys Arg Lys Thr Tyr Asp Thr Thr Val Asp
   750 755 760
   gac aat gag agc tat tac aag agt tac acc agc acc cag acc agc agc 2353
   Asp Asn Glu Ser Tyr Tyr Lys Ser Tyr Thr Ser Thr Gln Thr Ser Ser
   765 770 775 780
   aag agc ttt ctc aag agc tat gac agc agc acc agt gcc agt gag gcc 2401
   Lys Ser Phe Leu Lys Ser Tyr Asp Ser Ser Thr Ser Ala Ser Glu Ala
   785 790 795
   tat ggg aag agt tac tgc act acc agc aac agc agc att acc tat aag 2449
   Tyr Gly Lys Ser Tyr Cys Thr Thr Ser Asn Ser Ser Ile Thr Tyr Lys
   800 805 810
   aag agt tac ggc agc acc agt agc tct gac acc tgc cag aag agt ttt 2497
   Lys Ser Tyr Gly Ser Thr Ser Ser Ser Asp Thr Cys Gln Lys Ser Phe
   815 820 825
   gtc agc agc tgc act gac gag gaa cct gct gag cct gaa gac atg gag 2545
   Val Ser Ser Cys Thr Asp Glu Glu Pro Ala Glu Pro Glu Asp Met Glu
   830 835 840
   cgc ttt gag gaa atg gtt gtg aaa gtg ctg atc aag ctg cag gcg gtg 2593
   Arg Phe Glu Glu Met Val Val Lys Val Leu Ile Lys Leu Gln Ala Val
   845 850 855 860
   cag gcc atg tac cag ata agc cag gag gaa cac agc cag ctg caa gag 2641
   Gln Ala Met Tyr Gln Ile Ser Gln Glu Glu His Ser Gln Leu Gln Glu
   865 870 875
   cag atg gaa aag tta ctg gcc aag cag aaa gac ctg aag gaa gag ctg 2689
   Gln Met Glu Lys Leu Leu Ala Lys Gln Lys Asp Leu Lys Glu Glu Leu
   880 885 890
   gat gcc tgt gaa agg gag ttc aag gag tgc atg gaa tgc ctt gaa aag 2737
   Asp Ala Cys Glu Arg Glu Phe Lys Glu Cys Met Glu Cys Leu Glu Lys
   895 900 905
   ccc atg gcc ccc cag aac gac aag aat gag atc aaa gaa ctg cag acc 2785
   Pro Met Ala Pro Gln Asn Asp Lys Asn Glu Ile Lys Glu Leu Gln Thr
   910 915 920
   aag ctg cgg gag ctg cag ctg caa tac cag gct agc atg gat gag cag 2833
   Lys Leu Arg Glu Leu Gln Leu Gln Tyr Gln Ala Ser Met Asp Glu Gln
   925 930 935 940
   ggg cag ctt ctg gta gtg cag gag cag ctg gag ggg cag ctg cag tgc 2881
   Gly Gln Leu Leu Val Val Gln Glu Gln Leu Glu Gly Gln Leu Gln Cys
   945 950 955
   tgc cag gag gag ctc cgc cag ctc agg gag aag agg cct tct gtt gtc 2929
   Cys Gln Glu Glu Leu Arg Gln Leu Arg Glu Lys Arg Pro Ser Val Val
   960 965 970
   aaa gaa gcc cgg ggg aag aat gct aat aag aac atg aac aag aat gcc 2977
   Lys Glu Ala Arg Gly Lys Asn Ala Asn Lys Asn Met Asn Lys Asn Ala
   975 980 985
   aat ggg gtt aaa atg aaa aag gtg acc aag cca tgc tcg gat act tct 3025
   Asn Gly Val Lys Met Lys Lys Val Thr Lys Pro Cys Ser Asp Thr Ser
   990 995 1000
   gag agc gac ctt gag acc aga aag aag atc agg agg aaa atg aag agg 3073
   Glu Ser Asp Leu Glu Thr Arg Lys Lys Ile Arg Arg Lys Met Lys Arg
   1005 1010 1015 1020
   aca aag agg aag agg aga agg aag aag aca gtg aag agg agg aag atg 3121
   Thr Lys Arg Lys Arg Arg Arg Lys Lys Thr Val Lys Arg Arg Lys Met
   1025 1030 1035
   acg ccg act ctt ccc ttg aaa gtc ccg aag aaa ata acc ccc tca gac 3169
   Thr Pro Thr Leu Pro Leu Lys Val Pro Lys Lys Ile Thr Pro Ser Asp
   1040 1045 1050
   ttt ccg aga gca aaa aga aca tgt ttg ggt tgt gga agc cta tgg tat 3217
   Phe Pro Arg Ala Lys Arg Thr Cys Leu Gly Cys Gly Ser Leu Trp Tyr
   1055 1060 1065
   tct tgg cta ttg cag ctg tgg ctc tgt atg tgt tac cca aca tgc gac 3265
   Ser Trp Leu Leu Gln Leu Trp Leu Cys Met Cys Tyr Pro Thr Cys Asp
   1070                1075                1080
   agc agg agt cag agt tct gcc tca tgg agt gat ggc aga cct tgg cca      3313
   Ser Arg Ser Gln Ser Ser Ala Ser Trp Ser Asp Gly Arg Pro Trp Pro
   1085                1090                1095                1100
   gcg cga ggg cag atc ccc agt ggc cac cac cct cag ctt tgg gca gga      3361
   Ala Arg Gly Gln Ile Pro Ser Gly His His Pro Gln Leu Trp Ala Gly
                   1105                1110                1115
   cac act gtg cca gaa ccc tcc cca tat gtt cca tgt gtc ccc atc tcc      3409
   His Thr Val Pro Glu Pro Ser Pro Tyr Val Pro Cys Val Pro Ile Ser
               1120                1125                1130
   tca gcc tca gtc acc cag gct gaa aag gct tgt ggg gag cgg ctg act      3457
   Ser Ala Ser Val Thr Gln Ala Glu Lys Ala Cys Gly Glu Arg Leu Thr
           1135                1140                1145
   tcc atc tcc tgc ctt gtg taa gaacctgagt tccttgtaat taaatatcaa      3508
   Ser Ile Ser Cys Leu Val
       1150                1155
   ctgaattaaa aaaaaaaaaa aaaaa                                           3533

<210> 4
<211> 1154
<212> PRT
<213> Homo sapiens

<400> 4
Met Glu Ala Gly Ala Gly Ala Gly Ala Gly Ala Ala Gly Trp Ser Cys
  1               5                  10                  15
Pro Gly Pro Gly Pro Thr Val Thr Thr Leu Gly Ser Tyr Glu Ala Ser
             20                  25                  30
Glu Gly Cys Glu Arg Lys Lys Gly Gln Arg Trp Gly Ser Leu Glu Arg
         35                  40                  45
Arg Gly Met Gln Ala Met Glu Gly Glu Val Leu Leu Pro Ala Leu Tyr
     50                  55                  60
Glu Glu Glu Glu Glu Glu Glu Glu Glu Glu Glu Glu Val Glu Glu Glu
 65                  70                  75                  80
Glu Glu Gln Val Gln Lys Gly Gly Ser Val Gly Ser Leu Ser Val Asn
                 85                  90                  95
Lys His Arg Gly Leu Ser Leu Thr Glu Thr Glu Leu Glu Glu Leu Arg
            100                 105                 110
Ala Gln Val Leu Gln Leu Val Ala Glu Leu Glu Glu Thr Arg Glu Leu
        115                 120                 125

Ala Gly Gln His Glu Asp Asp Ser Leu Glu Leu Gln Gly Leu Leu Glu
    130                 135                 140
Asp Glu Arg Leu Ala Ser Ala Gln Gln Ala Glu Val Phe Thr Lys Gln
145                 150                 155                 160
Ile Gln Gln Leu Gln Gly Glu Leu Arg Ser Leu Arg Glu Glu Ile Ser
                165                 170                 175
Leu Leu Glu His Glu Lys Glu Ser Glu Leu Lys Glu Ile Glu Gln Glu
            180                 185                 190
Leu His Leu Ala Gln Ala Glu Ile Gln Ser Leu Arg Gln Ala Ala Glu
        195                 200                 205
Asp Ser Ala Thr Glu His Glu Ser Asp Ile Ala Ser Leu Gln Glu Asp
    210                 215                 220
Leu Cys Arg Met Gln Asn Glu Leu Glu Asp Met Glu Arg Ile Arg Gly
225                 230                 235                 240
Asp Tyr Glu Met Glu Ile Ala Ser Leu Arg Ala Glu Met Glu Met Lys
                245                 250                 255
Ser Ser Glu Pro Ser Gly Ser Leu Gly Leu Ser Asp Tyr Ser Gly Leu
            260                 265                 270
Gln Glu Glu Leu Gln Glu Leu Arg Glu Arg Tyr His Phe Leu Asn Glu
        275                 280                 285
Glu Tyr Arg Ala Leu Gln Glu Ser Asn Ser Ser Leu Thr Gly Gln Leu
    290                 295                 300
Ala Asp Leu Glu Ser Glu Arg Thr Gln Arg Ala Thr Glu Arg Trp Leu
305                 310                 315                 320
Gln Ser Gln Thr Leu Ser Met Thr Ser Ala Glu Ser Gln Thr Ser Glu
                325                 330                 335
Met Asp Phe Leu Glu Pro Asp Pro Glu Met Gln Leu Leu Arg Gln Gln
            340                 345                 350
Leu Arg Asp Ala Glu Glu Gln Met His Gly Met Lys Asn Lys Cys Gln
        355                 360                 365
Glu Leu Cys Cys Glu Leu Glu Glu Leu Gln His His Arg Gln Val Ser
    370                 375                 380
Glu Glu Glu Gln Arg Arg Leu Gln Arg Glu Leu Lys Cys Ala Gln Asn
385                 390                 395                 400
Glu Val Leu Arg Phe Gln Thr Ser His Ser Val Thr Gln Glu Leu Leu
                405                 410                 415
Cys Arg Leu Gln Lys Leu His Leu Gln His Gln Asn Val Thr Cys Glu
            420                 425                 430
Lys Glu Lys Leu Leu Glu Arg Gln Gln Gln Leu Gln Glu Glu Leu Gln
        435                 440                 445
Cys His Glu Ala Glu Leu Gln His Leu Arg Asp Thr Val Ala Ser Phe
    450                 455                 460
Lys Glu Ser Asn Glu Lys Asp Thr Glu Thr His Ala Gln Leu Gln Glu
465                 470                 475                 480
Met Lys Gln Leu Tyr Gln Ala Ser Lys Asp Glu Leu Glu Arg Gln Lys
                485                 490                 495
His Met Tyr Asp Gln Leu Glu Gln Asp Leu Leu Leu Cys Gln Leu Glu

            500                 505                 510
Leu Lys Glu Leu Lys Ala Ser His Pro Ile Pro Glu Asp Lys Gly Lys
        515                 520                 525
Cys Ala Asn Lys Cys Asp Thr Leu Leu Ser Arg Leu Thr Glu Leu Gln
    530                 535                 540
Glu Lys Tyr Lys Ala Ser Gln Lys Glu Met Gly Gln Leu Gln Met Glu
545                 550                 555                 560
Gln Cys Glu Leu Leu Glu Asp Gln Arg Arg Met Gln Glu Glu Gln Gly
                565                 570                 575
Gln Leu Gln Glu Glu Leu His Arg Leu Thr Leu Pro Leu Pro Lys Ser
            580                 585                 590
Gly Leu Leu Leu Lys Ser Gln Glu Leu Leu Thr Lys Leu Glu Asp Leu
        595                 600                 605
Cys Glu Leu Gln Leu Leu Tyr Gln Gly Met Gln Glu Glu Gln Lys Lys
    610                 615                 620
Leu Ile Gln Asn Gln Asp Cys Val Leu Lys Glu Gln Leu Glu Ile His
625                 630                 635                 640
Glu Glu Leu Arg Arg Phe Lys Glu Ser His Phe Gln Glu Val Leu Glu
                645                 650                 655
Asn Pro Asp Asp Ser Lys Leu Ala Lys Ser Ser Lys Cys Asn Arg Asn
            660                 665                 670
Lys Gln Ser Lys Leu Leu Met Glu Gln Met Gln Ala Leu Gln Val Met
        675                 680                 685
Tyr Asp Ala Gly Gln Ala Lys Gln Glu Leu Leu Gln Gln Glu Gln Gly
    690                 695                 700
Arg Leu Leu Glu Glu Arg Lys Arg Leu Gln Ala Asp Leu Gln Leu Cys
705                 710                 715                 720
Leu Glu Glu Met Gln Leu Leu Gln Val Gln Ser Pro Ser Ile Lys Met
                725                 730                 735
Ser Leu Glu Ser Tyr Gly Lys Ser Tyr Gly Ser Met Val Pro Ser Asn
            740                 745                 750
Glu Asn Cys Arg Lys Thr Tyr Asp Thr Thr Val Asp Asp Asn Glu Ser
        755                 760                 765
Tyr Tyr Lys Ser Tyr Thr Ser Thr Gln Thr Ser Ser Lys Ser Phe Leu
    770                 775                 780
Lys Ser Tyr Asp Ser Ser Thr Ser Ala Ser Glu Ala Tyr Gly Lys Ser
785                 790                 795                 800
Tyr Cys Thr Thr Ser Asn Ser Ser Ile Thr Tyr Lys Lys Ser Tyr Gly
                805                 810                 815
Ser Thr Ser Ser Ser Asp Thr Cys Gln Lys Ser Phe Val Ser Ser Cys
            820                 825                 830
Thr Asp Glu Glu Pro Ala Glu Pro Glu Asp Met Glu Arg Phe Glu Glu
        835                 840                 845
Met Val Val Lys Val Leu Ile Lys Leu Gln Ala Val Gln Ala Met Tyr
    850                 855                 860
Gln Ile Ser Gln Glu Glu His Ser Gln Leu Gln Glu Gln Met Glu Lys
865                 870                 875                 880

Leu Leu Ala Lys Gln Lys Asp Leu Lys Glu Glu Leu Asp Ala Cys Glu
                885                 890                 895
Arg Glu Phe Lys Glu Cys Met Glu Cys Leu Glu Lys Pro Met Ala Pro
            900                 905                 910
Gln Asn Asp Lys Asn Glu Ile Lys Glu Leu Gln Thr Lys Leu Arg Glu
        915                 920                 925
Leu Gln Leu Gln Tyr Gln Ala Ser Met Asp Glu Gln Gly Gln Leu Leu
    930                 935                 940
Val Val Gln Glu Gln Leu Glu Gly Gln Leu Gln Cys Cys Gln Glu Glu
945                 950                 955                 960
Leu Arg Gln Leu Arg Glu Lys Arg Pro Ser Val Val Lys Glu Ala Arg
                965                 970                 975
Gly Lys Asn Ala Asn Lys Asn Met Asn Lys Asn Ala Asn Gly Val Lys
            980                 985                 990
Met Lys Lys Val Thr Lys Pro Cys Ser Asp Thr Ser Glu Ser Asp Leu
        995                 1000                1005
Glu Thr Arg Lys Lys Ile Arg Arg Lys Met Lys Arg Thr Lys Arg Lys
    1010                1015                1020
Arg Arg Arg Lys Lys Thr Val Lys Arg Arg Lys Met Thr Pro Thr Leu
1025                1030                1035                1040
Pro Leu Lys Val Pro Lys Lys Ile Thr Pro Ser Asp Phe Pro Arg Ala
                1045                1050                1055
Lys Arg Thr Cys Leu Gly Cys Gly Ser Leu Trp Tyr Ser Trp Leu Leu
            1060                1065                1070
Gln Leu Trp Leu Cys Met Cys Tyr Pro Thr Cys Asp Ser Arg Ser Gln
        1075                1080                1085
Ser Ser Ala Ser Trp Ser Asp Gly Arg Pro Trp Pro Ala Arg Gly Gln
    1090                1095                1100
Ile Pro Ser Gly His His Pro Gln Leu Trp Ala Gly His Thr Val Pro
1105                1110                1115                1120
Glu Pro Ser Pro Tyr Val Pro Cys Val Pro Ile Ser Ser Ala Ser Val
                1125                1130                1135
Thr Gln Ala Glu Lys Ala Cys Gly Glu Arg Leu Thr Ser Ile Ser Cys
            1140                1145                1150
Leu Val

<210> 5
<211> 3714
<212> DNA
<213> Homo sapiens

<220>
<221> CDS
<222> (14)..(3478)

<400> 5
ggcacgagcg gtc atg gag gcg ggc gcc gga gcc ggc gcg gga gcc gcg     49
               Met Glu Ala Gly Ala Gly Ala Gly Ala Gly Ala Ala
                1               5                  10

ggc tgg agc tgc ccg ggc cca gga ccc aca gtg acc act cta ggc tcc     97
Gly Trp Ser Cys Pro Gly Pro Gly Pro Thr Val Thr Thr Leu Gly Ser
        15                  20                  25

tat gag gct tcc gag ggc tgt gag agg aag aag ggc caa cgc tgg ggg     145
Tyr Glu Ala Ser Glu Gly Cys Glu Arg Lys Lys Gly Gln Arg Trp Gly
    30                  35                  40

tcc ctg gaa cga cgg ggg atg caa gct atg gag ggg gag gtg tta ctc     193
Ser Leu Glu Arg Arg Gly Met Gln Ala Met Glu Gly Glu Val Leu Leu
45                  50                  55                  60

cca gct ctc tat gag gag gaa gag gaa gag gaa gag gag gaa gaa gag     241
Pro Ala Leu Tyr Glu Glu Glu Glu Glu Glu Glu Glu Glu Glu Glu Glu
                65                  70                  75

gtg gaa gaa gaa gaa gaa caa gtg cag aaa ggt ggc agt gtt ggc tct     289
Val Glu Glu Glu Glu Glu Gln Val Gln Lys Gly Gly Ser Val Gly Ser
            80                  85                  90

ctg tca gtc aac aag cac cgg gga ctg agc ctc acg gag aca gag ctg     337
Leu Ser Val Asn Lys His Arg Gly Leu Ser Leu Thr Glu Thr Glu Leu
        95                  100                 105

gag gag ctg cgg gct cag gtg ctg cag ctg gtg gca gaa ctg gag gag     385
Glu Glu Leu Arg Ala Gln Val Leu Gln Leu Val Ala Glu Leu Glu Glu
    110                 115                 120

acc cgg gaa ctg gca ggg cag cat gag gat gac tcc ttg gag cta cag     433
Thr Arg Glu Leu Ala Gly Gln His Glu Asp Asp Ser Leu Glu Leu Gln
125                 130                 135                 140

ggg ctc ctg gag gat gaa cgg cta gcc agc gcc cag cag gca gag gtg     481
Gly Leu Leu Glu Asp Glu Arg Leu Ala Ser Ala Gln Gln Ala Glu Val
                145                 150                 155

ttc acc aag cag atc cag cag ctc caa ggt gag ctg cgt tct cta cgg     529
Phe Thr Lys Gln Ile Gln Gln Leu Gln Gly Glu Leu Arg Ser Leu Arg
            160                 165                 170

gag gag att tcc ctg tta gag cat gag aaa gaa agc gaa ctt aag gaa     577
Glu Glu Ile Ser Leu Leu Glu His Glu Lys Glu Ser Glu Leu Lys Glu

        175                 180                 185

ata gaa cag gaa ttg cat ttg gcc cag gct gag atc cag agt ctg cgg 625
Ile Glu Gln Glu Leu His Leu Ala Gln Ala Glu Ile Gln Ser Leu Arg
    190                 195                 200

caa gca gca gag gat tcc gca act gaa cat gag agt gac ata gca tcc 673
Gln Ala Ala Glu Asp Ser Ala Thr Glu His Glu Ser Asp Ile Ala Ser
205                 210                 215                 220

ctg cag gag gat ctc tgc cgg atg cag aat gaa ctt gaa gac atg gaa 721
Leu Gln Glu Asp Leu Cys Arg Met Gln Asn Glu Leu Glu Asp Met Glu
                225                 230                 235

cgc att cgg gga gat tat gag atg gag atc gcc tcc ctc cgt gca gaa 769
Arg Ile Arg Gly Asp Tyr Glu Met Glu Ile Ala Ser Leu Arg Ala Glu
            240                 245                 250

atg gaa atg aag agc tct gaa cca tcc ggt agt tta ggt ctc tca gat 817
Met Glu Met Lys Ser Ser Glu Pro Ser Gly Ser Leu Gly Leu Ser Asp
        255                 260                 265

tac tct ggg tta caa gaa gaa ctg cag gag ctg cgg gaa cgc tac cat 865
Tyr Ser Gly Leu Gln Glu Glu Leu Gln Glu Leu Arg Glu Arg Tyr His
    270                 275                 280

ttc ctg aat gag gaa tac cgg gcc ctg cag gag agc aac agc agc ctc 913
Phe Leu Asn Glu Glu Tyr Arg Ala Leu Gln Glu Ser Asn Ser Ser Leu
285                 290                 295                 300

acg ggg cag ctt gca gat ctg gag agt gag agg aca cag aga gca aca 961
Thr Gly Gln Leu Ala Asp Leu Glu Ser Glu Arg Thr Gln Arg Ala Thr
                305                 310                 315

gag aga tgg ctg cag tcc caa aca ctg agt atg acg tca gca gag tct 1009
Glu Arg Trp Leu Gln Ser Gln Thr Leu Ser Met Thr Ser Ala Glu Ser
            320                 325                 330

cag act tca gaa atg gat ttc tta gag cct gat cct gaa atg cag ttg 1057
Gln Thr Ser Glu Met Asp Phe Leu Glu Pro Asp Pro Glu Met Gln Leu
        335                 340                 345

tta cgg cag cag cta cgg gat gct gaa gag cag atg cat ggc atg aag 1105
Leu Arg Gln Gln Leu Arg Asp Ala Glu Glu Gln Met His Gly Met Lys
    350                 355                 360

aac aag tgt cag gaa ttg tgt tgt gag ttg gaa gag cta cag cat cat 1153

Asn Lys Cys Gln Glu Leu Cys Cys Glu Leu Glu Glu Leu Gln His His
365                 370                 375                 380

cgc cag gtc agt gag gag gag cag agg cgg ctg cag agg gag ctc aag     1201
Arg Gln Val Ser Glu Glu Glu Gln Arg Arg Leu Gln Arg Glu Leu Lys
                385                 390                 395

tgt gct cag aat gag gtg ctt cgg ttt cag acc tcc cac agt gtc acc     1249
Cys Ala Gln Asn Glu Val Leu Arg Phe Gln Thr Ser His Ser Val Thr
            400                 405                 410

cag gag tta ctg tgc cgg ctg cag aag ctg cac ctc cag cac cag aac     1297
Gln Glu Leu Leu Cys Arg Leu Gln Lys Leu His Leu Gln His Gln Asn
        415                 420                 425

gtc aca tgt gag aag gaa aag ctg ctg gaa cgg cag cag cag ctg cag     1345
Val Thr Cys Glu Lys Glu Lys Leu Leu Glu Arg Gln Gln Gln Leu Gln
    430                 435                 440

gag gag ctg cag tgc cat gag gca gag ctg cag cac ctc agg gat acg     1393
Glu Glu Leu Gln Cys His Glu Ala Glu Leu Gln His Leu Arg Asp Thr
445                 450                 455                 460

gtg gcc tcc ttc aaa gag agc aat gag aag gac aca gag acg cac gct     1441
Val Ala Ser Phe Lys Glu Ser Asn Glu Lys Asp Thr Glu Thr His Ala
                465                 470                 475

cag ctt cag gag atg aag cag ctg tac cag gcc agc aag gac gag ctg     1489
Gln Leu Gln Glu Met Lys Gln Leu Tyr Gln Ala Ser Lys Asp Glu Leu
            480                 485                 490

gag cgg cag aag cac atg tat gac cag ctg gag cag gac ctc ctg ctc     1537
Glu Arg Gln Lys His Met Tyr Asp Gln Leu Glu Gln Asp Leu Leu Leu
        495                 500                 505

tgc cag ctg gag ctg aaa gag ctc aag gcc tcc cac ccc att ccg gag     1585
Cys Gln Leu Glu Leu Lys Glu Leu Lys Ala Ser His Pro Ile Pro Glu
    510                 515                 520

gac aaa gga aag tgt gct aat aag tgt gac aca ctg ctg tcc aga ctg     1633
Asp Lys Gly Lys Cys Ala Asn Lys Cys Asp Thr Leu Leu Ser Arg Leu
525                 530                 535                 540

aca gaa ttg cag gaa aag tac aag gcc agc cag aag gag atg ggg cag     1681
Thr Glu Leu Gln Glu Lys Tyr Lys Ala Ser Gln Lys Glu Met Gly Gln
                545                 550                 555

ctg cag atg gag cag tgt gag ctc ctg gag gat cag agg agg atg cag 1729
Leu Gln Met Glu Gln Cys Glu Leu Leu Glu Asp Gln Arg Arg Met Gln
            560                 565                 570

gag gag cag ggc cag ctg cag gaa gag ctg cac agg ctc aca ctg cca 1777
Glu Glu Gln Gly Gln Leu Gln Glu Glu Leu His Arg Leu Thr Leu Pro
        575                 580                 585

ctg cca aag agt ggc ctc tta ctc aag agt cag gag cta ctc acc aag 1825
Leu Pro Lys Ser Gly Leu Leu Leu Lys Ser Gln Glu Leu Leu Thr Lys
    590                 595                 600

tta gaa gac ctg tgt gag ctg cag ctg ctc tac caa ggc atg cag gag 1873
Leu Glu Asp Leu Cys Glu Leu Gln Leu Leu Tyr Gln Gly Met Gln Glu
605                 610                 615                 620

gaa cag aag aag ctg ata cag aac caa gac tgt gta tta aaa gaa caa 1921
Glu Gln Lys Lys Leu Ile Gln Asn Gln Asp Cys Val Leu Lys Glu Gln
                625                 630                 635

tta gag atc cac gaa gag ctg cga cgt ttc aaa gag tct cat ttc cag 1969
Leu Glu Ile His Glu Glu Leu Arg Arg Phe Lys Glu Ser His Phe Gln
            640                 645                 650

gaa gtg ttg gag aat ccc gat gat tcc aaa ttg gct aag tcc tcc aaa 2017
Glu Val Leu Glu Asn Pro Asp Asp Ser Lys Leu Ala Lys Ser Ser Lys
        655                 660                 665

tgt aat cga aac aag caa tcc aag ctg ctc atg gag cag atg cag gcc 2065
Cys Asn Arg Asn Lys Gln Ser Lys Leu Leu Met Glu Gln Met Gln Ala
    670                 675                 680

ctg cag gtg atg tat gac gcc ggt cag gcg aag cag gag ctc ttg cag 2113
Leu Gln Val Met Tyr Asp Ala Gly Gln Ala Lys Gln Glu Leu Leu Gln
685                 690                 695                 700

caa gag caa ggg agg ctc cta gag gag cgg aag agg ctg cag gca gac 2161
Gln Glu Gln Gly Arg Leu Leu Glu Glu Arg Lys Arg Leu Gln Ala Asp
                705                 710                 715

ttg cag ctc tgc ctg gaa gaa atg cag ctg ctt caa gtc cag tcc cct 2209
Leu Gln Leu Cys Leu Glu Glu Met Gln Leu Leu Gln Val Gln Ser Pro
            720                 725                 730

tct ata aaa atg agc ctt gag tcc tac ggg aag agc tat ggt agc atg 2257
Ser Ile Lys Met Ser Leu Glu Ser Tyr Gly Lys Ser Tyr Gly Ser Met
        735                 740                 745

gtc ccc agc aat gag aac tgt cgc aag act tat gat acc act gtg gat 2305
Val Pro Ser Asn Glu Asn Cys Arg Lys Thr Tyr Asp Thr Thr Val Asp
    750                 755                 760

gac aat gag agc tat tac aag agt tac acc agc acc cag acc agc agc 2353
Asp Asn Glu Ser Tyr Tyr Lys Ser Tyr Thr Ser Thr Gln Thr Ser Ser
765                 770                 775                 780

aag agc ttt ctc aag agc tat gac agc agc acc agt gcc agt gag gcc 2401
Lys Ser Phe Leu Lys Ser Tyr Asp Ser Ser Thr Ser Ala Ser Glu Ala
                785                 790                 795

tat ggg aag agt tac tgc act acc agc aac agc agc att acc tat aag 2449
Tyr Gly Lys Ser Tyr Cys Thr Thr Ser Asn Ser Ser Ile Thr Tyr Lys
            800                 805                 810

aag agt tac ggc agc acc agt agc tct gac acc tgc cag aag agt ttt 2497
Lys Ser Tyr Gly Ser Thr Ser Ser Ser Asp Thr Cys Gln Lys Ser Phe
        815                 820                 825

gtc agc agc tgc act gac gag gaa cct gct gag cct gaa gac atg gag 2545
Val Ser Ser Cys Thr Asp Glu Glu Pro Ala Glu Pro Glu Asp Met Glu
    830                 835                 840

cgc ttt gag gaa atg gtt gtg aaa gtg ctg atc aag ctg cag gcg gtg 2593
Arg Phe Glu Glu Met Val Val Lys Val Leu Ile Lys Leu Gln Ala Val
845                 850                 855                 860

cag gcc atg tac cag ata agc cag gag gaa cac agc cag ctg caa gag 2641
Gln Ala Met Tyr Gln Ile Ser Gln Glu Glu His Ser Gln Leu Gln Glu
                865                 870                 875

cag atg gaa aag tta ctg gcc aag cag aaa gac ctg aag gaa gag ctg 2689
Gln Met Glu Lys Leu Leu Ala Lys Gln Lys Asp Leu Lys Glu Glu Leu
            880                 885                 890

gat gcc tgt gaa agg gag ttc aag gag tgc atg gaa tgc ctt gaa aag 2737
Asp Ala Cys Glu Arg Glu Phe Lys Glu Cys Met Glu Cys Leu Glu Lys
        895                 900                 905

ccc atg gcc ccc cag aac gac aag aat gag atc aaa gaa ctg cag acc 2785
Pro Met Ala Pro Gln Asn Asp Lys Asn Glu Ile Lys Glu Leu Gln Thr
    910                 915                 920

aag ctg cgg gag ctg cag ctg caa tac cag gct agc atg gat gag cag 2833
Lys Leu Arg Glu Leu Gln Leu Gln Tyr Gln Ala Ser Met Asp Glu Gln

925                 930                 935                 940

ggg cag ctt ctg gta gtg cag gag cag ctg gag ggg cag ctg cag tgc    2881
Gly Gln Leu Leu Val Val Gln Glu Gln Leu Glu Gly Gln Leu Gln Cys
                945                 950                 955

tgc cag gag gag ctc cgc cag ctc agg gag aag agg cct tct gtt gtc    2929
Cys Gln Glu Glu Leu Arg Gln Leu Arg Glu Lys Arg Pro Ser Val Val
            960                 965                 970

aaa gaa gcc cgg ggg aag aat gct aat aag aac atg aac aag aat gcc    2977
Lys Glu Ala Arg Gly Lys Asn Ala Asn Lys Asn Met Asn Lys Asn Ala
        975                 980                 985

aat ggg gtt aaa atg aaa aag gtg acc aag cca tgc tcg gat act tct    3025
Asn Gly Val Lys Met Lys Lys Val Thr Lys Pro Cys Ser Asp Thr Ser
    990                 995                 1000

gag agc gac ctt gag acc aga aag agt ctg gag gta gtg ctg tac tac    3073
Glu Ser Asp Leu Glu Thr Arg Lys Ser Leu Glu Val Val Leu Tyr Tyr
1005                1010                1015                1020

aag gcc agc cag agg aaa tta gat gga cta gca aaa gag gag gaa aag    3121
Lys Ala Ser Gln Arg Lys Leu Asp Gly Leu Ala Lys Glu Glu Glu Lys
                1025                1030                1035

aaa gag gag atg gag gag gaa aaa aag gaa gtg aaa gag gaa gca aag    3169
Lys Glu Glu Met Glu Glu Glu Lys Lys Glu Val Lys Glu Glu Ala Lys
            1040                1045                1050

gag cag tgt ggg gat gag cta gtt gct gag cca gca gat cct gag gaa    3217
Glu Gln Cys Gly Asp Glu Leu Val Ala Glu Pro Ala Asp Pro Glu Glu
        1055                1060                1065

gct aaa tcc aca gaa gat cag gag gaa aat gaa gag gac aaa gag gaa    3265
Ala Lys Ser Thr Glu Asp Gln Glu Glu Asn Glu Glu Asp Lys Glu Glu
    1070                1075                1080

gag gag aag gaa gaa gac agt gaa gag gag gaa gat gac gcc gac tct    3313
Glu Glu Lys Glu Glu Asp Ser Glu Glu Glu Glu Asp Asp Ala Asp Ser
1085                1090                1095                1100

tcc ctt gaa agt ccc gaa gaa aat aac ccc ctc aga ctt tcc gag agc    3361
Ser Leu Glu Ser Pro Glu Glu Asn Asn Pro Leu Arg Leu Ser Glu Ser
                1105                1110                1115

aaa aag aac atg ttt ggg ttg tgg aag cct atg gta ttc ttg gct att    3409

Lys Lys Asn Met Phe Gly Leu Trp Lys Pro Met Val Phe Leu Ala Ile
        1120                1125                1130

gca gct gtg gct ctg tat gtg tta ccc aac atg cga cag cag gag tca    3457
Ala Ala Val Ala Leu Tyr Val Leu Pro Asn Met Arg Gln Gln Glu Ser
    1135                1140                1145

gag ttc tgc ctc atg gag tga tggcagacct tggccagcgc gagggcagat      3508
Glu Phe Cys Leu Met Glu
1150                1155

ccccagtggc caccaccctc agctttgggc aggacacact gtgccagaac cctccccata 3568

tgttccatgt gtccccatct cctcagcctc agtcacccag gctgaaaagg cttgtgggga 3628

gcggctgact tccatctcct gccttgtgta agaacctgag ttccttgtaa ttaaatatca 3688

actgaattaa aaaaaaaaaa aaaaaa                                      3714

<210> 6
<211> 1154
<212> PRT
<213> Homo sapiens

<400> 6
Met Glu Ala Gly Ala Gly Ala Gly Ala Gly Ala Ala Gly Trp Ser Cys
1               5                   10                  15
Pro Gly Pro Gly Pro Thr Val Thr Thr Leu Gly Ser Tyr Glu Ala Ser
            20                  25                  30
Glu Gly Cys Glu Arg Lys Lys Gly Gln Arg Trp Gly Ser Leu Glu Arg
        35                  40                  45
Arg Gly Met Gln Ala Met Glu Gly Glu Val Leu Leu Pro Ala Leu Tyr
    50                  55                  60
Glu Glu Glu Glu Glu Glu Glu Glu Glu Glu Glu Glu Val Glu Glu Glu
65                  70                  75                  80
Glu Glu Gln Val Gln Lys Gly Gly Ser Val Gly Ser Leu Ser Val Asn
                85                  90                  95
Lys His Arg Gly Leu Ser Leu Thr Glu Thr Glu Leu Glu Glu Leu Arg
            100                 105                 110
Ala Gln Val Leu Gln Leu Val Ala Glu Leu Glu Glu Thr Arg Glu Leu
        115                 120                 125
Ala Gly Gln His Glu Asp Asp Ser Leu Glu Leu Gln Gly Leu Leu Glu
    130                 135                 140
Asp Glu Arg Leu Ala Ser Ala Gln Gln Ala Glu Val Phe Thr Lys Gln
145                 150                 155                 160
Ile Gln Gln Leu Gln Gly Glu Leu Arg Ser Leu Arg Glu Glu Ile Ser

                165                 170                 175
Leu Leu Glu His Glu Lys Glu Ser Glu Leu Lys Glu Ile Glu Gln Glu
            180                 185                 190
Leu His Leu Ala Gln Ala Glu Ile Gln Ser Leu Arg Gln Ala Ala Glu
        195                 200                 205
Asp Ser Ala Thr Glu His Glu Ser Asp Ile Ala Ser Leu Gln Glu Asp
    210                 215                 220
Leu Cys Arg Met Gln Asn Glu Leu Glu Asp Met Glu Arg Ile Arg Gly
225                 230                 235                 240
Asp Tyr Glu Met Glu Ile Ala Ser Leu Arg Ala Glu Met Glu Met Lys
                245                 250                 255
Ser Ser Glu Pro Ser Gly Ser Leu Gly Leu Ser Asp Tyr Ser Gly Leu
            260                 265                 270
Gln Glu Glu Leu Gln Glu Leu Arg Glu Arg Tyr His Phe Leu Asn Glu
        275                 280                 285
Glu Tyr Arg Ala Leu Gln Glu Ser Asn Ser Ser Leu Thr Gly Gln Leu
    290                 295                 300
Ala Asp Leu Glu Ser Glu Arg Thr Gln Arg Ala Thr Glu Arg Trp Leu
305                 310                 315                 320
Gln Ser Gln Thr Leu Ser Met Thr Ser Ala Glu Ser Gln Thr Ser Glu
                325                 330                 335
Met Asp Phe Leu Glu Pro Asp Pro Glu Met Gln Leu Leu Arg Gln Gln
            340                 345                 350
Leu Arg Asp Ala Glu Glu Gln Met His Gly Met Lys Asn Lys Cys Gln
        355                 360                 365
Glu Leu Cys Cys Glu Leu Glu Glu Leu Gln His His Arg Gln Val Ser
    370                 375                 380
Glu Glu Glu Gln Arg Arg Leu Gln Arg Glu Leu Lys Cys Ala Gln Asn
385                 390                 395                 400
Glu Val Leu Arg Phe Gln Thr Ser His Ser Val Thr Gln Glu Leu Leu
                405                 410                 415
Cys Arg Leu Gln Lys Leu His Leu Gln His Gln Asn Val Thr Cys Glu
            420                 425                 430
Lys Glu Lys Leu Leu Glu Arg Gln Gln Gln Leu Gln Glu Glu Leu Gln
        435                 440                 445
Cys His Glu Ala Glu Leu Gln His Leu Arg Asp Thr Val Ala Ser Phe
    450                 455                 460
Lys Glu Ser Asn Glu Lys Asp Thr Glu Thr His Ala Gln Leu Gln Glu
465                 470                 475                 480
Met Lys Gln Leu Tyr Gln Ala Ser Lys Asp Glu Leu Glu Arg Gln Lys
                485                 490                 495
His Met Tyr Asp Gln Leu Glu Gln Asp Leu Leu Leu Cys Gln Leu Glu
            500                 505                 510
Leu Lys Glu Leu Lys Ala Ser His Pro Ile Pro Glu Asp Lys Gly Lys
        515                 520                 525
Cys Ala Asn Lys Cys Asp Thr Leu Leu Ser Arg Leu Thr Glu Leu Gln
    530                 535                 540

Glu Lys Tyr Lys Ala Ser Gln Lys Glu Met Gly Gln Leu Gln Met Glu
545                 550                 555                 560
Gln Cys Glu Leu Leu Glu Asp Gln Arg Arg Met Gln Glu Glu Gln Gly
                565                 570                 575
Gln Leu Gln Glu Glu Leu His Arg Leu Thr Leu Pro Leu Pro Lys Ser
            580                 585                 590
Gly Leu Leu Leu Lys Ser Gln Glu Leu Leu Thr Lys Leu Glu Asp Leu
        595                 600                 605
Cys Glu Leu Gln Leu Leu Tyr Gln Gly Met Gln Glu Glu Gln Lys Lys
    610                 615                 620
Leu Ile Gln Asn Gln Asp Cys Val Leu Lys Glu Gln Leu Glu Ile His
625                 630                 635                 640
Glu Glu Leu Arg Arg Phe Lys Glu Ser His Phe Gln Glu Val Leu Glu
                645                 650                 655
Asn Pro Asp Asp Ser Lys Leu Ala Lys Ser Ser Lys Cys Asn Arg Asn
            660                 665                 670
Lys Gln Ser Lys Leu Leu Met Glu Gln Met Gln Ala Leu Gln Val Met
        675                 680                 685
Tyr Asp Ala Gly Gln Ala Lys Gln Glu Leu Leu Gln Gln Glu Gln Gly
    690                 695                 700
Arg Leu Leu Glu Glu Arg Lys Arg Leu Gln Ala Asp Leu Gln Leu Cys
705                 710                 715                 720
Leu Glu Glu Met Gln Leu Leu Gln Val Gln Ser Pro Ser Ile Lys Met
                725                 730                 735
Ser Leu Glu Ser Tyr Gly Lys Ser Tyr Gly Ser Met Val Pro Ser Asn
            740                 745                 750
Glu Asn Cys Arg Lys Thr Tyr Asp Thr Thr Val Asp Asp Asn Glu Ser
        755                 760                 765
Tyr Tyr Lys Ser Tyr Thr Ser Thr Gln Thr Ser Ser Lys Ser Phe Leu
    770                 775                 780
Lys Ser Tyr Asp Ser Ser Thr Ser Ala Ser Glu Ala Tyr Gly Lys Ser
785                 790                 795                 800
Tyr Cys Thr Thr Ser Asn Ser Ser Ile Thr Tyr Lys Lys Ser Tyr Gly
                805                 810                 815
Ser Thr Ser Ser Ser Asp Thr Cys Gln Lys Ser Phe Val Ser Ser Cys
            820                 825                 830
Thr Asp Glu Glu Pro Ala Glu Pro Glu Asp Met Glu Arg Phe Glu Glu
        835                 840                 845
Met Val Val Lys Val Leu Ile Lys Leu Gln Ala Val Gln Ala Met Tyr
    850                 855                 860
Gln Ile Ser Gln Glu Glu His Ser Gln Leu Gln Glu Gln Met Glu Lys
865                 870                 875                 880
Leu Leu Ala Lys Gln Lys Asp Leu Lys Glu Glu Leu Asp Ala Cys Glu
                885                 890                 895
Arg Glu Phe Lys Glu Cys Met Glu Cys Leu Glu Lys Pro Met Ala Pro
            900                 905                 910
Gln Asn Asp Lys Asn Glu Ile Lys Glu Leu Gln Thr Lys Leu Arg Glu

915                 920                 925
Leu Gln Leu Gln Tyr Gln Ala Ser Met Asp Glu Gln Gly Gln Leu Leu
    930                 935                 940
Val Val Gln Glu Gln Leu Glu Gly Gln Leu Gln Cys Cys Gln Glu Glu
945                 950                 955                 960
Leu Arg Gln Leu Arg Glu Lys Arg Pro Ser Val Val Lys Glu Ala Arg
                965                 970                 975
Gly Lys Asn Ala Asn Lys Asn Met Asn Lys Asn Ala Asn Gly Val Lys
            980                 985                 990
Met Lys Lys Val Thr Lys Pro Cys Ser Asp Thr Ser Glu Ser Asp Leu
        995                 1000                1005
Glu Thr Arg Lys Ser Leu Glu Val Val Leu Tyr Tyr Lys Ala Ser Gln
    1010                1015                1020
Arg Lys Leu Asp Gly Leu Ala Lys Glu Glu Glu Lys Lys Glu Glu Met
1025                1030                1035                1040
Glu Glu Glu Lys Lys Glu Val Lys Glu Glu Ala Lys Glu Gln Cys Gly
                1045                1050                1055
Asp Glu Leu Val Ala Glu Pro Ala Asp Pro Glu Glu Ala Lys Ser Thr
            1060                1065                1070
Glu Asp Gln Glu Glu Asn Glu Glu Asp Lys Glu Glu Glu Glu Lys Glu
        1075                1080                1085
Glu Asp Ser Glu Glu Glu Glu Asp Asp Ala Asp Ser Ser Leu Glu Ser
    1090                1095                1100
Pro Glu Glu Asn Asn Pro Leu Arg Leu Ser Glu Ser Lys Lys Asn Met
1105                1110                1115                1120
Phe Gly Leu Trp Lys Pro Met Val Phe Leu Ala Ile Ala Ala Val Ala
                1125                1130                1135
Leu Tyr Val Leu Pro Asn Met Arg Gln Gln Glu Ser Glu Phe Cys Leu
            1140                1145                1150
Met Glu

<210> 7
<211> 3692
<212> DNA
<213> Homo sapiens

<220>
<221> CDS
<222> (14)..(3637)

<400> 7
ggcacgagcg gtc atg gag gcg ggc gcc gga gcc ggc gcg gga gcc gcg      49
               Met Glu Ala Gly Ala Gly Ala Gly Ala Gly Ala Ala
                 1               5                  10

ggc tgg agc tgc ccg ggc cca gga ccc aca gtg acc act cta ggc tcc 97
Gly Trp Ser Cys Pro Gly Pro Gly Pro Thr Val Thr Thr Leu Gly Ser
        15                  20                  25

tat gag gct tcc gag ggc tgt gag agg aag aag ggc caa cgc tgg ggg 145
Tyr Glu Ala Ser Glu Gly Cys Glu Arg Lys Lys Gly Gln Arg Trp Gly
    30                  35                  40

tcc ctg gaa cga cgg ggg atg caa gct atg gag ggg gag gtg tta ctc 193
Ser Leu Glu Arg Arg Gly Met Gln Ala Met Glu Gly Glu Val Leu Leu
45                  50                  55                  60

cca gct ctc tat gag gag gaa gag gaa gag gaa gag gag gaa gaa gag 241
Pro Ala Leu Tyr Glu Glu Glu Glu Glu Glu Glu Glu Glu Glu Glu Glu
                65                  70                  75

gtg gaa gaa gaa gaa gaa caa gtg cag aaa ggt ggc agt gtt ggc tct 289
Val Glu Glu Glu Glu Glu Gln Val Gln Lys Gly Gly Ser Val Gly Ser
            80                  85                  90

ctg tca gtc aac aag cac cgg gga ctg agc ctc acg gag aca gag ctg 337
Leu Ser Val Asn Lys His Arg Gly Leu Ser Leu Thr Glu Thr Glu Leu
        95                  100                 105

gag gag ctg cgg gct cag gtg ctg cag ctg gtg gca gaa ctg gag gag 385
Glu Glu Leu Arg Ala Gln Val Leu Gln Leu Val Ala Glu Leu Glu Glu
    110                 115                 120

acc cgg gaa ctg gca ggg cag cat gag gat gac tcc ttg gag cta cag 433
Thr Arg Glu Leu Ala Gly Gln His Glu Asp Asp Ser Leu Glu Leu Gln
125                 130                 135                 140

ggg ctc ctg gag gat gaa cgg cta gcc agc gcc cag cag gca gag gtg 481
Gly Leu Leu Glu Asp Glu Arg Leu Ala Ser Ala Gln Gln Ala Glu Val
                145                 150                 155

ttc acc aag cag atc cag cag ctc caa ggt gag ctg cgt tct cta cgg 529
Phe Thr Lys Gln Ile Gln Gln Leu Gln Gly Glu Leu Arg Ser Leu Arg
            160                 165                 170

gag gag att tcc ctg tta gag cat gag aaa gaa agc gaa ctt aag gaa 577
Glu Glu Ile Ser Leu Leu Glu His Glu Lys Glu Ser Glu Leu Lys Glu
        175                 180                 185

ata gaa cag gaa ttg cat ttg gcc cag gct gag atc cag agt ctg cgg 625
Ile Glu Gln Glu Leu His Leu Ala Gln Ala Glu Ile Gln Ser Leu Arg
    190                 195                 200

caa gca gca gag gat tcc gca act gaa cat gag agt gac ata gca tcc 673
Gln Ala Ala Glu Asp Ser Ala Thr Glu His Glu Ser Asp Ile Ala Ser
205                 210                 215                 220

ctg cag gag gat ctc tgc cgg atg cag aat gaa ctt gaa gac atg gaa 721
Leu Gln Glu Asp Leu Cys Arg Met Gln Asn Glu Leu Glu Asp Met Glu
                225                 230                 235

cgc att cgg gga gat tat gag atg gag atc gcc tcc ctc cgt gca gaa 769
Arg Ile Arg Gly Asp Tyr Glu Met Glu Ile Ala Ser Leu Arg Ala Glu
            240                 245                 250

atg gaa atg aag agc tct gaa cca tcc ggt agt tta ggt ctc tca gat 817
Met Glu Met Lys Ser Ser Glu Pro Ser Gly Ser Leu Gly Leu Ser Asp
        255                 260                 265

tac tct ggg tta caa gaa gaa ctg cag gag ctg cgg gaa cgc tac cat 865
Tyr Ser Gly Leu Gln Glu Glu Leu Gln Glu Leu Arg Glu Arg Tyr His
    270                 275                 280

ttc ctg aat gag gaa tac cgg gcc ctg cag gag agc aac agc agc ctc 913
Phe Leu Asn Glu Glu Tyr Arg Ala Leu Gln Glu Ser Asn Ser Ser Leu
285                 290                 295                 300

acg ggg cag ctt gca gat ctg gag agt gag agg aca cag aga gca aca 961
Thr Gly Gln Leu Ala Asp Leu Glu Ser Glu Arg Thr Gln Arg Ala Thr
                305                 310                 315

gag aga tgg ctg cag tcc caa aca ctg agt atg acg tca gca gag tct 1009
Glu Arg Trp Leu Gln Ser Gln Thr Leu Ser Met Thr Ser Ala Glu Ser
            320                 325                 330

cag act tca gaa atg gat ttc tta gag cct gat cct gaa atg cag ttg 1057
Gln Thr Ser Glu Met Asp Phe Leu Glu Pro Asp Pro Glu Met Gln Leu
        335                 340                 345

tta cgg cag cag cta cgg gat gct gaa gag cag atg cat ggc atg aag 1105
Leu Arg Gln Gln Leu Arg Asp Ala Glu Glu Gln Met His Gly Met Lys
    350                 355                 360

aac aag tgt cag gaa ttg tgt tgt gag ttg gaa gag cta cag cat cat 1153
Asn Lys Cys Gln Glu Leu Cys Cys Glu Leu Glu Glu Leu Gln His His
365                 370                 375                 380

cgc cag gtc agt gag gag gag cag agg cgg ctg cag agg gag ctc aag 1201
Arg Gln Val Ser Glu Glu Glu Gln Arg Arg Leu Gln Arg Glu Leu Lys

                385                 390                 395

tgt gct cag aat gag gtg ctt cgg ttt cag acc tcc cac agt gtc acc 1249
Cys Ala Gln Asn Glu Val Leu Arg Phe Gln Thr Ser His Ser Val Thr
            400                 405                 410

cag aat gag gag ctg aag tcc aga ctc tgt acc ctg cag aaa aaa tat 1297
Gln Asn Glu Glu Leu Lys Ser Arg Leu Cys Thr Leu Gln Lys Lys Tyr
        415                 420                 425

gat act agc cag gat gag cag aac gag ctc ttg aag atg cag ctg caa 1345
Asp Thr Ser Gln Asp Glu Gln Asn Glu Leu Leu Lys Met Gln Leu Gln
    430                 435                 440

ctt cag act gag ctc cgg cag ctc aaa gtc atg aaa tcc aca ctt gta 1393
Leu Gln Thr Glu Leu Arg Gln Leu Lys Val Met Lys Ser Thr Leu Val
445                 450                 455                 460

gaa aac cag agt gag aag gag tta ctg tgc cgg ctg cag aag ctg cac 1441
Glu Asn Gln Ser Glu Lys Glu Leu Leu Cys Arg Leu Gln Lys Leu His
                465                 470                 475

ctc cag cac cag aac gtc aca tgt gag aag gaa aag ctg ctg gaa cgg 1489
Leu Gln His Gln Asn Val Thr Cys Glu Lys Glu Lys Leu Leu Glu Arg
            480                 485                 490

cag cag cag ctg cag gag gag ctg cag tgc cat gag gca gag ctg cag 1537
Gln Gln Gln Leu Gln Glu Glu Leu Gln Cys His Glu Ala Glu Leu Gln
        495                 500                 505

cac ctc agg gat acg gtg gcc tcc ttc aaa gag agc aat gag aag gac 1585
His Leu Arg Asp Thr Val Ala Ser Phe Lys Glu Ser Asn Glu Lys Asp
    510                 515                 520

aca gag acg cac gct cag ctt cag gag atg aag cag ctg tac cag gcc 1633
Thr Glu Thr His Ala Gln Leu Gln Glu Met Lys Gln Leu Tyr Gln Ala
525                 530                 535                 540

agc aag gac gag ctg gag cgg cag aag cac atg tat gac cag ctg gag 1681
Ser Lys Asp Glu Leu Glu Arg Gln Lys His Met Tyr Asp Gln Leu Glu
                545                 550                 555

cag gac ctc ctg ctc tgc cag ctg gag ctg aaa gag ctc aag gcc tcc 1729
Gln Asp Leu Leu Leu Cys Gln Leu Glu Leu Lys Glu Leu Lys Ala Ser
            560                 565                 570

cac ccc att ccg gag gac aaa gga aag tgt gct aat aag tgt gac aca 1777

His Pro Ile Pro Glu Asp Lys Gly Lys Cys Ala Asn Lys Cys Asp Thr
        575                 580                 585

ctg ctg tcc aga ctg aca gaa ttg cag gaa aag tac aag gcc agc cag      1825
Leu Leu Ser Arg Leu Thr Glu Leu Gln Glu Lys Tyr Lys Ala Ser Gln
    590                 595                 600

aag gag atg ggg cag ctg cag atg gag cag tgt gag ctc ctg gag gat      1873
Lys Glu Met Gly Gln Leu Gln Met Glu Gln Cys Glu Leu Leu Glu Asp
605                 610                 615                 620

cag agg agg atg cag gag gag cag ggc cag ctg cag gaa gag ctg cac      1921
Gln Arg Arg Met Gln Glu Glu Gln Gly Gln Leu Gln Glu Glu Leu His
                625                 630                 635

agg ctc aca ctg cca ctg cca aag agt ggc ctc tta ctc aag agt cag      1969
Arg Leu Thr Leu Pro Leu Pro Lys Ser Gly Leu Leu Leu Lys Ser Gln
            640                 645                 650

gag cta ctc acc aag tta gaa gac ctg tgt gag ctg cag ctg ctc tac      2017
Glu Leu Leu Thr Lys Leu Glu Asp Leu Cys Glu Leu Gln Leu Leu Tyr
        655                 660                 665

caa ggc atg cag gag gaa cag aag aag ctg ata cag aac caa gac tgt      2065
Gln Gly Met Gln Glu Glu Gln Lys Lys Leu Ile Gln Asn Gln Asp Cys
    670                 675                 680

gta tta aaa gaa caa tta gag atc cac gaa gag ctg cga cgt ttc aaa      2113
Val Leu Lys Glu Gln Leu Glu Ile His Glu Glu Leu Arg Arg Phe Lys
685                 690                 695                 700

gag tct cat ttc cag gaa gtg ttg gag aat ccc gat gat tcc aaa ttg      2161
Glu Ser His Phe Gln Glu Val Leu Glu Asn Pro Asp Asp Ser Lys Leu
                705                 710                 715

gct aag tcc tcc aaa tgt aat cga aac aag caa tcc aag ctg ctc atg      2209
Ala Lys Ser Ser Lys Cys Asn Arg Asn Lys Gln Ser Lys Leu Leu Met
            720                 725                 730

gag cag atg cag gcc ctg cag gtg atg tat gac gcc ggt cag gcg aag      2257
Glu Gln Met Gln Ala Leu Gln Val Met Tyr Asp Ala Gly Gln Ala Lys
        735                 740                 745

cag gag ctc ttg cag caa gag caa ggg agg ctc cta gag gag cgg aag      2305
Gln Glu Leu Leu Gln Gln Glu Gln Gly Arg Leu Leu Glu Glu Arg Lys
    750                 755                 760

agg ctg cag gca gac ttg cag ctc tgc ctg gaa gaa atg cag ctg ctt 2353
Arg Leu Gln Ala Asp Leu Gln Leu Cys Leu Glu Glu Met Gln Leu Leu
765                 770                 775                 780

caa gtc cag tcc cct tct ata aaa atg agc ctt gag tcc tac ggg aag 2401
Gln Val Gln Ser Pro Ser Ile Lys Met Ser Leu Glu Ser Tyr Gly Lys
                785                 790                 795

agc tat ggt agc atg gtc ccc agc aat gag aac tgt cgc aag act tat 2449
Ser Tyr Gly Ser Met Val Pro Ser Asn Glu Asn Cys Arg Lys Thr Tyr
            800                 805                 810

gat acc act gtg gat gac aat gag agc tat tac aag agt tac acc agc 2497
Asp Thr Thr Val Asp Asp Asn Glu Ser Tyr Tyr Lys Ser Tyr Thr Ser
        815                 820                 825

acc cag acc agc agc aag agc ttt ctc aag agc tat gac agc agc acc 2545
Thr Gln Thr Ser Ser Lys Ser Phe Leu Lys Ser Tyr Asp Ser Ser Thr
    830                 835                 840

agt gcc agt gag gcc tat ggg aag agt tac tgc act acc agc aac agc 2593
Ser Ala Ser Glu Ala Tyr Gly Lys Ser Tyr Cys Thr Thr Ser Asn Ser
845                 850                 855                 860

agc att acc tat aag aag agt tac ggc agc acc agt agc tct gac acc 2641
Ser Ile Thr Tyr Lys Lys Ser Tyr Gly Ser Thr Ser Ser Ser Asp Thr
                865                 870                 875

tgc cag aag agt ttt gtc agc agc tgc act gac gag gaa cct gct gag 2689
Cys Gln Lys Ser Phe Val Ser Ser Cys Thr Asp Glu Glu Pro Ala Glu
            880                 885                 890

cct gaa gac atg gag cgc ttt gag gaa atg gtt gtg aaa gtg ctg atc 2737
Pro Glu Asp Met Glu Arg Phe Glu Glu Met Val Val Lys Val Leu Ile
        895                 900                 905

aag ctg cag gcg gtg cag gcc atg tac cag ata agc cag gag gaa cac 2785
Lys Leu Gln Ala Val Gln Ala Met Tyr Gln Ile Ser Gln Glu Glu His
    910                 915                 920

agc cag ctg caa gag cag atg gaa aag tta ctg gcc aag cag aaa gac 2833
Ser Gln Leu Gln Glu Gln Met Glu Lys Leu Leu Ala Lys Gln Lys Asp
925                 930                 935                 940

ctg aag gaa gag ctg gat gcc tgt gaa agg gag ttc aag gag tgc atg 2881
Leu Lys Glu Glu Leu Asp Ala Cys Glu Arg Glu Phe Lys Glu Cys Met
                945                 950                 955

gaa tgc ctt gaa aag ccc atg gcc ccc cag aac gac aag aat gag atc 2929
Glu Cys Leu Glu Lys Pro Met Ala Pro Gln Asn Asp Lys Asn Glu Ile
            960                 965                 970

aaa gaa ctg cag acc aag ctg cgg gag ctg cag ctg caa tac cag gct 2977
Lys Glu Leu Gln Thr Lys Leu Arg Glu Leu Gln Leu Gln Tyr Gln Ala
        975                 980                 985

agc atg gat gag cag ggg cag ctt ctg gta gtg cag gag cag ctg gag 3025
Ser Met Asp Glu Gln Gly Gln Leu Leu Val Val Gln Glu Gln Leu Glu
    990                 995                 1000

ggg cag ctg cag tgc tgc cag gag gag ctc cgc cag ctc agg gag aag 3073
Gly Gln Leu Gln Cys Cys Gln Glu Glu Leu Arg Gln Leu Arg Glu Lys
1005                1010                1015                1020

agg cct tct gtt gtc aaa gaa gcc cgg ggg aag aat gct aat aag aac 3121
Arg Pro Ser Val Val Lys Glu Ala Arg Gly Lys Asn Ala Asn Lys Asn
                1025                1030                1035

atg aac aag aat gcc aat ggg gtt aaa atg aaa aag gtg acc aag cca 3169
Met Asn Lys Asn Ala Asn Gly Val Lys Met Lys Lys Val Thr Lys Pro
            1040                1045                1050

tgc tcg gat act tct gag agc gac ctt gag acc aga aag aag atc agg 3217
Cys Ser Asp Thr Ser Glu Ser Asp Leu Glu Thr Arg Lys Lys Ile Arg
        1055                1060                1065

agg aaa atg aag agg aca aag agg aag agg aga agg aag aag aca gtg 3265
Arg Lys Met Lys Arg Thr Lys Arg Lys Arg Arg Arg Lys Lys Thr Val
    1070                1075                1080

aag agg agg aag atg acg ccg act ctt ccc ttg aaa gtc ccg aag aaa 3313
Lys Arg Arg Lys Met Thr Pro Thr Leu Pro Leu Lys Val Pro Lys Lys
1085                1090                1095                1100

ata acc ccc tca gac ttt ccg aga gca aaa aga aca tgt ttg ggt tgt 3361
Ile Thr Pro Ser Asp Phe Pro Arg Ala Lys Arg Thr Cys Leu Gly Cys
                1105                1110                1115

gga agc cta tgg tat tct tgg cta ttg cag ctg tgg ctc tgt atg tgt 3409
Gly Ser Leu Trp Tyr Ser Trp Leu Leu Gln Leu Trp Leu Cys Met Cys
            1120                1125                1130

tac cca aca tgc gac agc agg agt cag agt tct gcc tca tgg agt gat 3457
Tyr Pro Thr Cys Asp Ser Arg Ser Gln Ser Ser Ala Ser Trp Ser Asp

1135                1140                1145

ggc aga cct tgg cca gcg cga ggg cag atc ccc agt ggc cac cac cct    3505
Gly Arg Pro Trp Pro Ala Arg Gly Gln Ile Pro Ser Gly His His Pro
    1150                1155                1160

cag ctt tgg gca gga cac act gtg cca gaa ccc tcc cca tat gtt cca    3553
Gln Leu Trp Ala Gly His Thr Val Pro Glu Pro Ser Pro Tyr Val Pro
1165                1170                1175                1180

tgt gtc ccc atc tcc tca gcc tca gtc acc cag gct gaa aag gct tgt    3601
Cys Val Pro Ile Ser Ser Ala Ser Val Thr Gln Ala Glu Lys Ala Cys
                1185                1190                1195

ggg gag cgg ctg act tcc atc tcc tgc ctt gtg taa gaacctgagt         3647
Gly Glu Arg Leu Thr Ser Ile Ser Cys Leu Val
            1200                1205

tccttgtaat taaatatcaa ctgaattaaa aaaaaaaaaa aaaaa                   3692

<210> 8
<211> 1207
<212> PRT
<213> Homo sapiens

<400> 8
Met Glu Ala Gly Ala Gly Ala Gly Ala Gly Ala Ala Gly Trp Ser Cys
1               5                   10                  15
Pro Gly Pro Gly Pro Thr Val Thr Thr Leu Gly Ser Tyr Glu Ala Ser
            20                  25                  30
Glu Gly Cys Glu Arg Lys Lys Gly Gln Arg Trp Gly Ser Leu Glu Arg
        35                  40                  45
Arg Gly Met Gln Ala Met Glu Gly Glu Val Leu Leu Pro Ala Leu Tyr
    50                  55                  60
Glu Glu Glu Glu Glu Glu Glu Glu Glu Glu Glu Glu Val Glu Glu Glu
65                  70                  75                  80
Glu Glu Gln Val Gln Lys Gly Gly Ser Val Gly Ser Leu Ser Val Asn
                85                  90                  95
Lys His Arg Gly Leu Ser Leu Thr Glu Thr Glu Leu Glu Glu Leu Arg
            100                 105                 110
Ala Gln Val Leu Gln Leu Val Ala Glu Leu Glu Glu Thr Arg Glu Leu
        115                 120                 125
Ala Gly Gln His Glu Asp Asp Ser Leu Glu Leu Gln Gly Leu Leu Glu
    130                 135                 140
Asp Glu Arg Leu Ala Ser Ala Gln Gln Ala Glu Val Phe Thr Lys Gln
145                 150                 155                 160

Ile Gln Gln Leu Gln Gly Glu Leu Arg Ser Leu Arg Glu Glu Ile Ser
                165                 170                 175
Leu Leu Glu His Glu Lys Glu Ser Glu Leu Lys Glu Ile Glu Gln Glu
            180                 185                 190
Leu His Leu Ala Gln Ala Glu Ile Gln Ser Leu Arg Gln Ala Ala Glu
        195                 200                 205
Asp Ser Ala Thr Glu His Glu Ser Asp Ile Ala Ser Leu Gln Glu Asp
    210                 215                 220
Leu Cys Arg Met Gln Asn Glu Leu Glu Asp Met Glu Arg Ile Arg Gly
225                 230                 235                 240
Asp Tyr Glu Met Glu Ile Ala Ser Leu Arg Ala Glu Met Glu Met Lys
                245                 250                 255
Ser Ser Glu Pro Ser Gly Ser Leu Gly Leu Ser Asp Tyr Ser Gly Leu
            260                 265                 270
Gln Glu Glu Leu Gln Glu Leu Arg Glu Arg Tyr His Phe Leu Asn Glu
        275                 280                 285
Glu Tyr Arg Ala Leu Gln Glu Ser Asn Ser Ser Leu Thr Gly Gln Leu
    290                 295                 300
Ala Asp Leu Glu Ser Glu Arg Thr Gln Arg Ala Thr Glu Arg Trp Leu
305                 310                 315                 320
Gln Ser Gln Thr Leu Ser Met Thr Ser Ala Glu Ser Gln Thr Ser Glu
                325                 330                 335
Met Asp Phe Leu Glu Pro Asp Pro Glu Met Gln Leu Leu Arg Gln Gln
            340                 345                 350
Leu Arg Asp Ala Glu Glu Gln Met His Gly Met Lys Asn Lys Cys Gln
        355                 360                 365
Glu Leu Cys Cys Glu Leu Glu Glu Leu Gln His His Arg Gln Val Ser
    370                 375                 380
Glu Glu Glu Gln Arg Arg Leu Gln Arg Glu Leu Lys Cys Ala Gln Asn
385                 390                 395                 400
Glu Val Leu Arg Phe Gln Thr Ser His Ser Val Thr Gln Asn Glu Glu
                405                 410                 415
Leu Lys Ser Arg Leu Cys Thr Leu Gln Lys Lys Tyr Asp Thr Ser Gln
            420                 425                 430
Asp Glu Gln Asn Glu Leu Leu Lys Met Gln Leu Gln Leu Gln Thr Glu
        435                 440                 445
Leu Arg Gln Leu Lys Val Met Lys Ser Thr Leu Val Glu Asn Gln Ser
    450                 455                 460
Glu Lys Glu Leu Leu Cys Arg Leu Gln Lys Leu His Leu Gln His Gln
465                 470                 475                 480
Asn Val Thr Cys Glu Lys Glu Lys Leu Leu Glu Arg Gln Gln Gln Leu
                485                 490                 495
Gln Glu Glu Leu Gln Cys His Glu Ala Glu Leu Gln His Leu Arg Asp
            500                 505                 510
Thr Val Ala Ser Phe Lys Glu Ser Asn Glu Lys Asp Thr Glu Thr His
        515                 520                 525
Ala Gln Leu Gln Glu Met Lys Gln Leu Tyr Gln Ala Ser Lys Asp Glu

530                 535                 540
Leu Glu Arg Gln Lys His Met Tyr Asp Gln Leu Glu Gln Asp Leu Leu
545                 550                 555                 560
Leu Cys Gln Leu Glu Leu Lys Glu Leu Lys Ala Ser His Pro Ile Pro
                565                 570                 575
Glu Asp Lys Gly Lys Cys Ala Asn Lys Cys Asp Thr Leu Leu Ser Arg
            580                 585                 590
Leu Thr Glu Leu Gln Glu Lys Tyr Lys Ala Ser Gln Lys Glu Met Gly
        595                 600                 605
Gln Leu Gln Met Glu Gln Cys Glu Leu Leu Glu Asp Gln Arg Arg Met
    610                 615                 620
Gln Glu Glu Gln Gly Gln Leu Gln Glu Glu Leu His Arg Leu Thr Leu
625                 630                 635                 640
Pro Leu Pro Lys Ser Gly Leu Leu Leu Lys Ser Gln Glu Leu Leu Thr
                645                 650                 655
Lys Leu Glu Asp Leu Cys Glu Leu Gln Leu Leu Tyr Gln Gly Met Gln
            660                 665                 670
Glu Glu Gln Lys Lys Leu Ile Gln Asn Gln Asp Cys Val Leu Lys Glu
        675                 680                 685
Gln Leu Glu Ile His Glu Glu Leu Arg Arg Phe Lys Glu Ser His Phe
    690                 695                 700
Gln Glu Val Leu Glu Asn Pro Asp Asp Ser Lys Leu Ala Lys Ser Ser
705                 710                 715                 720
Lys Cys Asn Arg Asn Lys Gln Ser Lys Leu Leu Met Glu Gln Met Gln
                725                 730                 735
Ala Leu Gln Val Met Tyr Asp Ala Gly Gln Ala Lys Gln Glu Leu Leu
            740                 745                 750
Gln Gln Glu Gln Gly Arg Leu Leu Glu Glu Arg Lys Arg Leu Gln Ala
        755                 760                 765
Asp Leu Gln Leu Cys Leu Glu Glu Met Gln Leu Leu Gln Val Gln Ser
    770                 775                 780
Pro Ser Ile Lys Met Ser Leu Glu Ser Tyr Gly Lys Ser Tyr Gly Ser
785                 790                 795                 800
Met Val Pro Ser Asn Glu Asn Cys Arg Lys Thr Tyr Asp Thr Thr Val
                805                 810                 815
Asp Asp Asn Glu Ser Tyr Tyr Lys Ser Tyr Thr Ser Thr Gln Thr Ser
            820                 825                 830
Ser Lys Ser Phe Leu Lys Ser Tyr Asp Ser Ser Thr Ser Ala Ser Glu
        835                 840                 845
Ala Tyr Gly Lys Ser Tyr Cys Thr Thr Ser Asn Ser Ser Ile Thr Tyr
    850                 855                 860
Lys Lys Ser Tyr Gly Ser Thr Ser Ser Ser Asp Thr Cys Gln Lys Ser
865                 870                 875                 880
Phe Val Ser Ser Cys Thr Asp Glu Glu Pro Ala Glu Pro Glu Asp Met
                885                 890                 895
Glu Arg Phe Glu Glu Met Val Val Lys Val Leu Ile Lys Leu Gln Ala
            900                 905                 910

Val Gln Ala Met Tyr Gln Ile Ser Gln Glu Glu His Ser Gln Leu Gln
        915                 920                 925
Glu Gln Met Glu Lys Leu Leu Ala Lys Gln Lys Asp Leu Lys Glu Glu
    930                 935                 940
Leu Asp Ala Cys Glu Arg Glu Phe Lys Glu Cys Met Glu Cys Leu Glu
945                 950                 955                 960
Lys Pro Met Ala Pro Gln Asn Asp Lys Asn Glu Ile Lys Glu Leu Gln
                965                 970                 975
Thr Lys Leu Arg Glu Leu Gln Leu Gln Tyr Gln Ala Ser Met Asp Glu
            980                 985                 990
Gln Gly Gln Leu Leu Val Val Gln Glu Gln Leu Glu Gly Gln Leu Gln
        995                 1000                1005
Cys Cys Gln Glu Glu Leu Arg Gln Leu Arg Glu Lys Arg Pro Ser Val
    1010                1015                1020
Val Lys Glu Ala Arg Gly Lys Asn Ala Asn Lys Asn Met Asn Lys Asn
1025                1030                1035                1040
Ala Asn Gly Val Lys Met Lys Lys Val Thr Lys Pro Cys Ser Asp Thr
                1045                1050                1055
Ser Glu Ser Asp Leu Glu Thr Arg Lys Lys Ile Arg Arg Lys Met Lys
            1060                1065                1070
Arg Thr Lys Arg Lys Arg Arg Arg Lys Lys Thr Val Lys Arg Arg Lys
        1075                1080                1085
Met Thr Pro Thr Leu Pro Leu Lys Val Pro Lys Lys Ile Thr Pro Ser
    1090                1095                1100
Asp Phe Pro Arg Ala Lys Arg Thr Cys Leu Gly Cys Gly Ser Leu Trp
1105                1110                1115                1120
Tyr Ser Trp Leu Leu Gln Leu Trp Leu Cys Met Cys Tyr Pro Thr Cys
                1125                1130                1135
Asp Ser Arg Ser Gln Ser Ser Ala Ser Trp Ser Asp Gly Arg Pro Trp
            1140                1145                1150
Pro Ala Arg Gly Gln Ile Pro Ser Gly His His Pro Gln Leu Trp Ala
        1155                1160                1165
Gly His Thr Val Pro Glu Pro Ser Pro Tyr Val Pro Cys Val Pro Ile
    1170                1175                1180
Ser Ser Ala Ser Val Thr Gln Ala Glu Lys Ala Cys Gly Glu Arg Leu
1185                1190                1195                1200
Thr Ser Ile Ser Cys Leu Val
                1205

<210> 9
<211> 3873
<212> DNA
<213> Homo sapiens

<220>

<221> CDS
<222> (14)..(3637)

<400> 9
ggcacgagcg gtc atg gag gcg ggc gcc gga gcc ggc gcg gga gcc gcg     49
               Met Glu Ala Gly Ala Gly Ala Gly Ala Gly Ala Ala
               1               5                   10

ggc tgg agc tgc ccg ggc cca gga ccc aca gtg acc act cta ggc tcc     97
Gly Trp Ser Cys Pro Gly Pro Gly Pro Thr Val Thr Thr Leu Gly Ser
        15                  20                  25

tat gag gct tcc gag ggc tgt gag agg aag aag ggc caa cgc tgg ggg     145
Tyr Glu Ala Ser Glu Gly Cys Glu Arg Lys Lys Gly Gln Arg Trp Gly
    30                  35                  40

tcc ctg gaa cga cgg ggg atg caa gct atg gag ggg gag gtg tta ctc     193
Ser Leu Glu Arg Arg Gly Met Gln Ala Met Glu Gly Glu Val Leu Leu
45                  50                  55                  60

cca gct ctc tat gag gag gaa gag gaa gag gaa gag gag gaa gaa gag     241
Pro Ala Leu Tyr Glu Glu Glu Glu Glu Glu Glu Glu Glu Glu Glu Glu
                65                  70                  75

gtg gaa gaa gaa gaa gaa caa gtg cag aaa ggt ggc agt gtt ggc tct     289
Val Glu Glu Glu Glu Glu Gln Val Gln Lys Gly Gly Ser Val Gly Ser
            80                  85                  90

ctg tca gtc aac aag cac cgg gga ctg agc ctc acg gag aca gag ctg     337
Leu Ser Val Asn Lys His Arg Gly Leu Ser Leu Thr Glu Thr Glu Leu
        95                  100                 105

gag gag ctg cgg gct cag gtg ctg cag ctg gtg gca gaa ctg gag gag     385
Glu Glu Leu Arg Ala Gln Val Leu Gln Leu Val Ala Glu Leu Glu Glu
    110                 115                 120

acc cgg gaa ctg gca ggg cag cat gag gat gac tcc ttg gag cta cag     433
Thr Arg Glu Leu Ala Gly Gln His Glu Asp Asp Ser Leu Glu Leu Gln
125                 130                 135                 140

ggg ctc ctg gag gat gaa cgg cta gcc agc gcc cag cag gca gag gtg     481
Gly Leu Leu Glu Asp Glu Arg Leu Ala Ser Ala Gln Gln Ala Glu Val
                145                 150                 155

ttc acc aag cag atc cag cag ctc caa ggt gag ctg cgt tct cta cgg     529
Phe Thr Lys Gln Ile Gln Gln Leu Gln Gly Glu Leu Arg Ser Leu Arg
            160                 165                 170

gag gag att tcc ctg tta gag cat gag aaa gaa agc gaa ctt aag gaa 577
Glu Glu Ile Ser Leu Leu Glu His Glu Lys Glu Ser Glu Leu Lys Glu
        175                 180                 185

ata gaa cag gaa ttg cat ttg gcc cag gct gag atc cag agt ctg cgg 625
Ile Glu Gln Glu Leu His Leu Ala Gln Ala Glu Ile Gln Ser Leu Arg
    190                 195                 200

caa gca gca gag gat tcc gca act gaa cat gag agt gac ata gca tcc 673
Gln Ala Ala Glu Asp Ser Ala Thr Glu His Glu Ser Asp Ile Ala Ser
205                 210                 215                 220

ctg cag gag gat ctc tgc cgg atg cag aat gaa ctt gaa gac atg gaa 721
Leu Gln Glu Asp Leu Cys Arg Met Gln Asn Glu Leu Glu Asp Met Glu
                225                 230                 235

cgc att cgg gga gat tat gag atg gag atc gcc tcc ctc cgt gca gaa 769
Arg Ile Arg Gly Asp Tyr Glu Met Glu Ile Ala Ser Leu Arg Ala Glu
            240                 245                 250

atg gaa atg aag agc tct gaa cca tcc ggt agt tta ggt ctc tca gat 817
Met Glu Met Lys Ser Ser Glu Pro Ser Gly Ser Leu Gly Leu Ser Asp
        255                 260                 265

tac tct ggg tta caa gaa gaa ctg cag gag ctg cgg gaa cgc tac cat 865
Tyr Ser Gly Leu Gln Glu Glu Leu Gln Glu Leu Arg Glu Arg Tyr His
    270                 275                 280

ttc ctg aat gag gaa tac cgg gcc ctg cag gag agc aac agc agc ctc 913
Phe Leu Asn Glu Glu Tyr Arg Ala Leu Gln Glu Ser Asn Ser Ser Leu
285                 290                 295                 300

acg ggg cag ctt gca gat ctg gag agt gag agg aca cag aga gca aca 961
Thr Gly Gln Leu Ala Asp Leu Glu Ser Glu Arg Thr Gln Arg Ala Thr
                305                 310                 315

gag aga tgg ctg cag tcc caa aca ctg agt atg acg tca gca gag tct 1009
Glu Arg Trp Leu Gln Ser Gln Thr Leu Ser Met Thr Ser Ala Glu Ser
            320                 325                 330

cag act tca gaa atg gat ttc tta gag cct gat cct gaa atg cag ttg 1057
Gln Thr Ser Glu Met Asp Phe Leu Glu Pro Asp Pro Glu Met Gln Leu
        335                 340                 345

tta cgg cag cag cta cgg gat gct gaa gag cag atg cat ggc atg aag 1105
Leu Arg Gln Gln Leu Arg Asp Ala Glu Glu Gln Met His Gly Met Lys

350                 355                 360

aac aag tgt cag gaa ttg tgt tgt gag ttg gaa gag cta cag cat cat     1153
Asn Lys Cys Gln Glu Leu Cys Cys Glu Leu Glu Glu Leu Gln His His
365                 370                 375                 380

cgc cag gtc agt gag gag gag cag agg cgg ctg cag agg gag ctc aag     1201
Arg Gln Val Ser Glu Glu Glu Gln Arg Arg Leu Gln Arg Glu Leu Lys
                385                 390                 395

tgt gct cag aat gag gtg ctt cgg ttt cag acc tcc cac agt gtc acc     1249
Cys Ala Gln Asn Glu Val Leu Arg Phe Gln Thr Ser His Ser Val Thr
            400                 405                 410

cag aat gag gag ctg aag tcc aga ctc tgt acc ctg cag aaa aaa tat     1297
Gln Asn Glu Glu Leu Lys Ser Arg Leu Cys Thr Leu Gln Lys Lys Tyr
        415                 420                 425

gat act agc cag gat gag cag aac gag ctc ttg aag atg cag ctg caa     1345
Asp Thr Ser Gln Asp Glu Gln Asn Glu Leu Leu Lys Met Gln Leu Gln
    430                 435                 440

ctt cag act gag ctc cgg cag ctc aaa gtc atg aaa tcc aca ctt gta     1393
Leu Gln Thr Glu Leu Arg Gln Leu Lys Val Met Lys Ser Thr Leu Val
445                 450                 455                 460

gaa aac cag agt gag aag gag tta ctg tgc cgg ctg cag aag ctg cac     1441
Glu Asn Gln Ser Glu Lys Glu Leu Leu Cys Arg Leu Gln Lys Leu His
                465                 470                 475

ctc cag cac cag aac gtc aca tgt gag aag gaa aag ctg ctg gaa cgg     1489
Leu Gln His Gln Asn Val Thr Cys Glu Lys Glu Lys Leu Leu Glu Arg
            480                 485                 490

cag cag cag ctg cag gag gag ctg cag tgc cat gag gca gag ctg cag     1537
Gln Gln Gln Leu Gln Glu Glu Leu Gln Cys His Glu Ala Glu Leu Gln
        495                 500                 505

cac ctc agg gat acg gtg gcc tcc ttc aaa gag agc aat gag aag gac     1585
His Leu Arg Asp Thr Val Ala Ser Phe Lys Glu Ser Asn Glu Lys Asp
    510                 515                 520

aca gag acg cac gct cag ctt cag gag atg aag cag ctg tac cag gcc     1633
Thr Glu Thr His Ala Gln Leu Gln Glu Met Lys Gln Leu Tyr Gln Ala
525                 530                 535                 540

agc aag gac gag ctg gag cgg cag aag cac atg tat gac cag ctg gag     1681

Ser Lys Asp Glu Leu Glu Arg Gln Lys His Met Tyr Asp Gln Leu Glu
                545                 550                 555

cag gac ctc ctg ctc tgc cag ctg gag ctg aaa gag ctc aag gcc tcc     1729
Gln Asp Leu Leu Leu Cys Gln Leu Glu Leu Lys Glu Leu Lys Ala Ser
            560                 565                 570

cac ccc att ccg gag gac aaa gga aag tgt gct aat aag tgt gac aca     1777
His Pro Ile Pro Glu Asp Lys Gly Lys Cys Ala Asn Lys Cys Asp Thr
        575                 580                 585

ctg ctg tcc aga ctg aca gaa ttg cag gaa aag tac aag gcc agc cag     1825
Leu Leu Ser Arg Leu Thr Glu Leu Gln Glu Lys Tyr Lys Ala Ser Gln
    590                 595                 600

aag gag atg ggg cag ctg cag atg gag cag tgt gag ctc ctg gag gat     1873
Lys Glu Met Gly Gln Leu Gln Met Glu Gln Cys Glu Leu Leu Glu Asp
605                 610                 615                 620

cag agg agg atg cag gag gag cag ggc cag ctg cag gaa gag ctg cac     1921
Gln Arg Arg Met Gln Glu Glu Gln Gly Gln Leu Gln Glu Glu Leu His
                625                 630                 635

agg ctc aca ctg cca ctg cca aag agt ggc ctc tta ctc aag agt cag     1969
Arg Leu Thr Leu Pro Leu Pro Lys Ser Gly Leu Leu Leu Lys Ser Gln
            640                 645                 650

gag cta ctc acc aag tta gaa gac ctg tgt gag ctg cag ctg ctc tac     2017
Glu Leu Leu Thr Lys Leu Glu Asp Leu Cys Glu Leu Gln Leu Leu Tyr
        655                 660                 665

caa ggc atg cag gag gaa cag aag aag ctg ata cag aac caa gac tgt     2065
Gln Gly Met Gln Glu Glu Gln Lys Lys Leu Ile Gln Asn Gln Asp Cys
    670                 675                 680

gta tta aaa gaa caa tta gag atc cac gaa gag ctg cga cgt ttc aaa     2113
Val Leu Lys Glu Gln Leu Glu Ile His Glu Glu Leu Arg Arg Phe Lys
685                 690                 695                 700

gag tct cat ttc cag gaa gtg ttg gag aat ccc gat gat tcc aaa ttg     2161
Glu Ser His Phe Gln Glu Val Leu Glu Asn Pro Asp Asp Ser Lys Leu
                705                 710                 715

gct aag tcc tcc aaa tgt aat cga aac aag caa tcc aag ctg ctc atg     2209
Ala Lys Ser Ser Lys Cys Asn Arg Asn Lys Gln Ser Lys Leu Leu Met
            720                 725                 730

gag cag atg cag gcc ctg cag gtg atg tat gac gcc ggt cag gcg aag     2257
Glu Gln Met Gln Ala Leu Gln Val Met Tyr Asp Ala Gly Gln Ala Lys
        735                 740                 745

cag gag ctc ttg cag caa gag caa ggg agg ctc cta gag gag cgg aag     2305
Gln Glu Leu Leu Gln Gln Glu Gln Gly Arg Leu Leu Glu Glu Arg Lys
    750                 755                 760

agg ctg cag gca gac ttg cag ctc tgc ctg gaa gaa atg cag ctg ctt     2353
Arg Leu Gln Ala Asp Leu Gln Leu Cys Leu Glu Glu Met Gln Leu Leu
765                 770                 775                 780

caa gtc cag tcc cct tct ata aaa atg agc ctt gag tcc tac ggg aag     2401
Gln Val Gln Ser Pro Ser Ile Lys Met Ser Leu Glu Ser Tyr Gly Lys
                785                 790                 795

agc tat ggt agc atg gtc ccc agc aat gag aac tgt cgc aag act tat     2449
Ser Tyr Gly Ser Met Val Pro Ser Asn Glu Asn Cys Arg Lys Thr Tyr
            800                 805                 810

gat acc act gtg gat gac aat gag agc tat tac aag agt tac acc agc     2497
Asp Thr Thr Val Asp Asp Asn Glu Ser Tyr Tyr Lys Ser Tyr Thr Ser
        815                 820                 825

acc cag acc agc agc aag agc ttt ctc aag agc tat gac agc agc acc     2545
Thr Gln Thr Ser Ser Lys Ser Phe Leu Lys Ser Tyr Asp Ser Ser Thr
    830                 835                 840

agt gcc agt gag gcc tat ggg aag agt tac tgc act acc agc aac agc     2593
Ser Ala Ser Glu Ala Tyr Gly Lys Ser Tyr Cys Thr Thr Ser Asn Ser
845                 850                 855                 860

agc att acc tat aag aag agt tac ggc agc acc agt agc tct gac acc     2641
Ser Ile Thr Tyr Lys Lys Ser Tyr Gly Ser Thr Ser Ser Ser Asp Thr
                865                 870                 875

tgc cag aag agt ttt gtc agc agc tgc act gac gag gaa cct gct gag     2689
Cys Gln Lys Ser Phe Val Ser Ser Cys Thr Asp Glu Glu Pro Ala Glu
            880                 885                 890

cct gaa gac atg gag cgc ttt gag gaa atg gtt gtg aaa gtg ctg atc     2737
Pro Glu Asp Met Glu Arg Phe Glu Glu Met Val Val Lys Val Leu Ile
        895                 900                 905

aag ctg cag gcg gtg cag gcc atg tac cag ata agc cag gag gaa cac     2785
Lys Leu Gln Ala Val Gln Ala Met Tyr Gln Ile Ser Gln Glu Glu His
    910                 915                 920

agc cag ctg caa gag cag atg gaa aag tta ctg gcc aag cag aaa gac 2833
Ser Gln Leu Gln Glu Gln Met Glu Lys Leu Leu Ala Lys Gln Lys Asp
925                 930                 935                 940

ctg aag gaa gag ctg gat gcc tgt gaa agg gag ttc aag gag tgc atg 2881
Leu Lys Glu Glu Leu Asp Ala Cys Glu Arg Glu Phe Lys Glu Cys Met
                945                 950                 955

gaa tgc ctt gaa aag ccc atg gcc ccc cag aac gac aag aat gag atc 2929
Glu Cys Leu Glu Lys Pro Met Ala Pro Gln Asn Asp Lys Asn Glu Ile
            960                 965                 970

aaa gaa ctg cag acc aag ctg cgg gag ctg cag ctg caa tac cag gct 2977
Lys Glu Leu Gln Thr Lys Leu Arg Glu Leu Gln Leu Gln Tyr Gln Ala
        975                 980                 985

agc atg gat gag cag ggg cag ctt ctg gta gtg cag gag cag ctg gag 3025
Ser Met Asp Glu Gln Gly Gln Leu Leu Val Val Gln Glu Gln Leu Glu
    990                 995                 1000

ggg cag ctg cag tgc tgc cag gag gag ctc cgc cag ctc agg gag aag 3073
Gly Gln Leu Gln Cys Cys Gln Glu Glu Leu Arg Gln Leu Arg Glu Lys
1005                1010                1015                1020

agg cct tct gtt gtc aaa gaa gcc cgg ggg aag aat gct aat aag aac 3121
Arg Pro Ser Val Val Lys Glu Ala Arg Gly Lys Asn Ala Asn Lys Asn
                1025                1030                1035

atg aac aag aat gcc aat ggg gtt aaa atg aaa aag gtg acc aag cca 3169
Met Asn Lys Asn Ala Asn Gly Val Lys Met Lys Lys Val Thr Lys Pro
            1040                1045                1050

tgc tcg gat act tct gag agc gac ctt gag acc aga aag agt ctg gag 3217
Cys Ser Asp Thr Ser Glu Ser Asp Leu Glu Thr Arg Lys Ser Leu Glu
        1055                1060                1065

gta gtg ctg tac tac aag gcc agc cag agg aaa tta gat gga cta gca 3265
Val Val Leu Tyr Tyr Lys Ala Ser Gln Arg Lys Leu Asp Gly Leu Ala
    1070                1075                1080

aaa gag gag gaa aag aaa gag gag atg gag gag gaa aaa aag gaa gtg 3313
Lys Glu Glu Glu Lys Lys Glu Glu Met Glu Glu Glu Lys Lys Glu Val
1085                1090                1095                1100

aaa gag gaa gca aag gag cag tgt ggg gat gag cta gtt gct gag cca 3361
Lys Glu Glu Ala Lys Glu Gln Cys Gly Asp Glu Leu Val Ala Glu Pro

1105                1110                1115

gca gat cct gag gaa gct aaa tcc aca gaa gat cag gag gaa aat gaa    3409
Ala Asp Pro Glu Glu Ala Lys Ser Thr Glu Asp Gln Glu Glu Asn Glu
        1120                1125                1130

gag gac aaa gag gaa gag gag aag gaa gaa gac agt gaa gag gag gaa    3457
Glu Asp Lys Glu Glu Glu Glu Lys Glu Glu Asp Ser Glu Glu Glu Glu
    1135                1140                1145

gat gac gcc gac tct tcc ctt gaa agt ccc gaa gaa aat aac ccc ctc    3505
Asp Asp Ala Asp Ser Ser Leu Glu Ser Pro Glu Glu Asn Asn Pro Leu
1150                1155                1160

aga ctt tcc gag agc aaa aag aac atg ttt ggg ttg tgg aag cct atg    3553
Arg Leu Ser Glu Ser Lys Lys Asn Met Phe Gly Leu Trp Lys Pro Met
1165                1170                1175                1180

gta ttc ttg gct att gca gct gtg gct ctg tat gtg tta ccc aac atg    3601
Val Phe Leu Ala Ile Ala Ala Val Ala Leu Tyr Val Leu Pro Asn Met
                1185                1190                1195

cga cag cag gag tca gag ttc tgc ctc atg gag tga tggcagacct         3647
Arg Gln Gln Glu Ser Glu Phe Cys Leu Met Glu
            1200                1205

tggccagcgc gagggcagat ccccagtggc caccaccctc agctttgggc aggacacact 3707

gtgccagaac cctccccata tgttccatgt gtccccatct cctcagcctc agtcacccag 3767

gctgaaaagg cttgtgggga gcggctgact tccatctcct gccttgtgta agaacctgag 3827

ttccttgtaa ttaaatatca actgaattaa aaaaaaaaaa aaaaaa                3873

<210> 10
<211> 1207
<212> PRT
<213> Homo sapiens

<400> 10
Met Glu Ala Gly Ala Gly Ala Gly Ala Gly Ala Ala Gly Trp Ser Cys
1               5                   10                  15
Pro Gly Pro Gly Pro Thr Val Thr Thr Leu Gly Ser Tyr Glu Ala Ser
            20                  25                  30
Glu Gly Cys Glu Arg Lys Lys Gly Gln Arg Trp Gly Ser Leu Glu Arg
        35                  40                  45

Arg Gly Met Gln Ala Met Glu Gly Glu Val Leu Leu Pro Ala Leu Tyr
    50                  55                  60
Glu Glu Glu Glu Glu Glu Glu Glu Glu Glu Glu Glu Val Glu Glu Glu
65                  70                  75                  80
Glu Glu Gln Val Gln Lys Gly Gly Ser Val Gly Ser Leu Ser Val Asn
                85                  90                  95
Lys His Arg Gly Leu Ser Leu Thr Glu Thr Glu Leu Glu Glu Leu Arg
            100                 105                 110
Ala Gln Val Leu Gln Leu Val Ala Glu Leu Glu Glu Thr Arg Glu Leu
        115                 120                 125
Ala Gly Gln His Glu Asp Asp Ser Leu Glu Leu Gln Gly Leu Leu Glu
    130                 135                 140
Asp Glu Arg Leu Ala Ser Ala Gln Gln Ala Glu Val Phe Thr Lys Gln
145                 150                 155                 160
Ile Gln Gln Leu Gln Gly Glu Leu Arg Ser Leu Arg Glu Glu Ile Ser
                165                 170                 175
Leu Leu Glu His Glu Lys Glu Ser Glu Leu Lys Glu Ile Glu Gln Glu
            180                 185                 190
Leu His Leu Ala Gln Ala Glu Ile Gln Ser Leu Arg Gln Ala Ala Glu
        195                 200                 205
Asp Ser Ala Thr Glu His Glu Ser Asp Ile Ala Ser Leu Gln Glu Asp
    210                 215                 220
Leu Cys Arg Met Gln Asn Glu Leu Glu Asp Met Glu Arg Ile Arg Gly
225                 230                 235                 240
Asp Tyr Glu Met Glu Ile Ala Ser Leu Arg Ala Glu Met Glu Met Lys
                245                 250                 255
Ser Ser Glu Pro Ser Gly Ser Leu Gly Leu Ser Asp Tyr Ser Gly Leu
            260                 265                 270
Gln Glu Glu Leu Gln Glu Leu Arg Glu Arg Tyr His Phe Leu Asn Glu
        275                 280                 285
Glu Tyr Arg Ala Leu Gln Glu Ser Asn Ser Ser Leu Thr Gly Gln Leu
    290                 295                 300
Ala Asp Leu Glu Ser Glu Arg Thr Gln Arg Ala Thr Glu Arg Trp Leu
305                 310                 315                 320
Gln Ser Gln Thr Leu Ser Met Thr Ser Ala Glu Ser Gln Thr Ser Glu
                325                 330                 335
Met Asp Phe Leu Glu Pro Asp Pro Glu Met Gln Leu Leu Arg Gln Gln
            340                 345                 350
Leu Arg Asp Ala Glu Glu Gln Met His Gly Met Lys Asn Lys Cys Gln
        355                 360                 365
Glu Leu Cys Cys Glu Leu Glu Glu Leu Gln His His Arg Gln Val Ser
    370                 375                 380
Glu Glu Glu Gln Arg Arg Leu Gln Arg Glu Leu Lys Cys Ala Gln Asn
385                 390                 395                 400
Glu Val Leu Arg Phe Gln Thr Ser His Ser Val Thr Gln Asn Glu Glu
                405                 410                 415
Leu Lys Ser Arg Leu Cys Thr Leu Gln Lys Lys Tyr Asp Thr Ser Gln

            420                 425                 430
Asp Glu Gln Asn Glu Leu Leu Lys Met Gln Leu Gln Leu Gln Thr Glu
        435                 440                 445
Leu Arg Gln Leu Lys Val Met Lys Ser Thr Leu Val Glu Asn Gln Ser
    450                 455                 460
Glu Lys Glu Leu Leu Cys Arg Leu Gln Lys Leu His Leu Gln His Gln
465                 470                 475                 480
Asn Val Thr Cys Glu Lys Glu Lys Leu Leu Glu Arg Gln Gln Gln Leu
                485                 490                 495
Gln Glu Glu Leu Gln Cys His Glu Ala Glu Leu Gln His Leu Arg Asp
            500                 505                 510
Thr Val Ala Ser Phe Lys Glu Ser Asn Glu Lys Asp Thr Glu Thr His
        515                 520                 525
Ala Gln Leu Gln Glu Met Lys Gln Leu Tyr Gln Ala Ser Lys Asp Glu
    530                 535                 540
Leu Glu Arg Gln Lys His Met Tyr Asp Gln Leu Glu Gln Asp Leu Leu
545                 550                 555                 560
Leu Cys Gln Leu Glu Leu Lys Glu Leu Lys Ala Ser His Pro Ile Pro
                565                 570                 575
Glu Asp Lys Gly Lys Cys Ala Asn Lys Cys Asp Thr Leu Leu Ser Arg
            580                 585                 590
Leu Thr Glu Leu Gln Glu Lys Tyr Lys Ala Ser Gln Lys Glu Met Gly
        595                 600                 605
Gln Leu Gln Met Glu Gln Cys Glu Leu Leu Glu Asp Gln Arg Arg Met
    610                 615                 620
Gln Glu Glu Gln Gly Gln Leu Gln Glu Glu Leu His Arg Leu Thr Leu
625                 630                 635                 640
Pro Leu Pro Lys Ser Gly Leu Leu Leu Lys Ser Gln Glu Leu Leu Thr
                645                 650                 655
Lys Leu Glu Asp Leu Cys Glu Leu Gln Leu Leu Tyr Gln Gly Met Gln
            660                 665                 670
Glu Glu Gln Lys Lys Leu Ile Gln Asn Gln Asp Cys Val Leu Lys Glu
        675                 680                 685
Gln Leu Glu Ile His Glu Glu Leu Arg Arg Phe Lys Glu Ser His Phe
    690                 695                 700
Gln Glu Val Leu Glu Asn Pro Asp Asp Ser Lys Leu Ala Lys Ser Ser
705                 710                 715                 720
Lys Cys Asn Arg Asn Lys Gln Ser Lys Leu Leu Met Glu Gln Met Gln
                725                 730                 735
Ala Leu Gln Val Met Tyr Asp Ala Gly Gln Ala Lys Gln Glu Leu Leu
            740                 745                 750
Gln Gln Glu Gln Gly Arg Leu Leu Glu Glu Arg Lys Arg Leu Gln Ala
        755                 760                 765
Asp Leu Gln Leu Cys Leu Glu Glu Met Gln Leu Leu Gln Val Gln Ser
    770                 775                 780
Pro Ser Ile Lys Met Ser Leu Glu Ser Tyr Gly Lys Ser Tyr Gly Ser
785                 790                 795                 800

Met Val Pro Ser Asn Glu Asn Cys Arg Lys Thr Tyr Asp Thr Thr Val
                805                 810                 815
Asp Asp Asn Glu Ser Tyr Tyr Lys Ser Tyr Thr Ser Thr Gln Thr Ser
            820                 825                 830
Ser Lys Ser Phe Leu Lys Ser Tyr Asp Ser Ser Thr Ser Ala Ser Glu
        835                 840                 845
Ala Tyr Gly Lys Ser Tyr Cys Thr Thr Ser Asn Ser Ser Ile Thr Tyr
    850                 855                 860
Lys Lys Ser Tyr Gly Ser Thr Ser Ser Ser Asp Thr Cys Gln Lys Ser
865                 870                 875                 880
Phe Val Ser Ser Cys Thr Asp Glu Glu Pro Ala Glu Pro Glu Asp Met
                885                 890                 895
Glu Arg Phe Glu Glu Met Val Val Lys Val Leu Ile Lys Leu Gln Ala
            900                 905                 910
Val Gln Ala Met Tyr Gln Ile Ser Gln Glu Glu His Ser Gln Leu Gln
        915                 920                 925
Glu Gln Met Glu Lys Leu Leu Ala Lys Gln Lys Asp Leu Lys Glu Glu
    930                 935                 940
Leu Asp Ala Cys Glu Arg Glu Phe Lys Glu Cys Met Glu Cys Leu Glu
945                 950                 955                 960
Lys Pro Met Ala Pro Gln Asn Asp Lys Asn Glu Ile Lys Glu Leu Gln
                965                 970                 975
Thr Lys Leu Arg Glu Leu Gln Leu Gln Tyr Gln Ala Ser Met Asp Glu
            980                 985                 990
Gln Gly Gln Leu Leu Val Val Gln Glu Gln Leu Glu Gly Gln Leu Gln
        995                 1000                1005
Cys Cys Gln Glu Glu Leu Arg Gln Leu Arg Glu Lys Arg Pro Ser Val
    1010                1015                1020
Val Lys Glu Ala Arg Gly Lys Asn Ala Asn Lys Asn Met Asn Lys Asn
1025                1030                1035                1040
Ala Asn Gly Val Lys Met Lys Lys Val Thr Lys Pro Cys Ser Asp Thr
                1045                1050                1055
Ser Glu Ser Asp Leu Glu Thr Arg Lys Ser Leu Glu Val Val Leu Tyr
            1060                1065                1070
Tyr Lys Ala Ser Gln Arg Lys Leu Asp Gly Leu Ala Lys Glu Glu Glu
        1075                1080                1085
Lys Lys Glu Glu Met Glu Glu Glu Lys Lys Glu Val Lys Glu Glu Ala
    1090                1095                1100
Lys Glu Gln Cys Gly Asp Glu Leu Val Ala Glu Pro Ala Asp Pro Glu
1105                1110                1115                1120
Glu Ala Lys Ser Thr Glu Asp Gln Glu Glu Asn Glu Glu Asp Lys Glu
                1125                1130                1135
Glu Glu Glu Lys Glu Glu Asp Ser Glu Glu Glu Glu Asp Asp Ala Asp
            1140                1145                1150
Ser Ser Leu Glu Ser Pro Glu Glu Asn Asn Pro Leu Arg Leu Ser Glu
        1155                1160                1165
Ser Lys Lys Asn Met Phe Gly Leu Trp Lys Pro Met Val Phe Leu Ala

1170                 1175                1180
Ile Ala Ala Val Ala Leu Tyr Val Leu Pro Asn Met Arg Gln Gln Glu
1185                1190                1195                1200
Ser Glu Phe Cys Leu Met Glu
                1205

<210> 11
<211> 3847
<212> DNA
<213> Homo sapiens

<220>
<221> CDS
<222> (211)..(2883)

<400> 11
gcggaggtgg agtacaacgg gggcttccac ctggccatcg acgtggacct ggtcttcggc 60

aagtccgcct acttgtttgt caagctgtcc cgcgtggtgg ggaggctgcg cttggtcttt 120

acgcgcgtgc ccttcaccca ctggttcttc tccttcgtgg aagacccgct gatcgacttc 180

gaggtgcgct cgcagtttga agggcggccc atg ccc cag ctc acc tcc atc atc 234
                                 Met Pro Gln Leu Thr Ser Ile Ile
                                 1               5

gtc aac cag ctc aag aag atc atc aag cgc aag cac acc cta ccg aat 282
Val Asn Gln Leu Lys Lys Ile Ile Lys Arg Lys His Thr Leu Pro Asn
    10                  15                  20

tac aag atc agg ttt aag ccg ttt ttt cca tac cag acc ttg caa gga 330
Tyr Lys Ile Arg Phe Lys Pro Phe Phe Pro Tyr Gln Thr Leu Gln Gly
25                  30                  35                  40

ttt gaa gaa gat gaa gag cat atc cat ata caa caa tgg gca ctt act 378
Phe Glu Glu Asp Glu Glu His Ile His Ile Gln Gln Trp Ala Leu Thr
                45                  50                  55

gaa ggc cgt ctt aaa gtt acg ttg tta gaa tgt agc agg tta ctc att 426
Glu Gly Arg Leu Lys Val Thr Leu Leu Glu Cys Ser Arg Leu Leu Ile
            60                  65                  70

ttt gga tcc tat gac aga gag gca aat gtt cat tgc aca ctt gag tta 474
Phe Gly Ser Tyr Asp Arg Glu Ala Asn Val His Cys Thr Leu Glu Leu
        75                  80                  85

agc agt agt gtt tgg gaa gaa aaa cag agg agt tct att aag acg gtt 522
Ser Ser Ser Val Trp Glu Glu Lys Gln Arg Ser Ser Ile Lys Thr Val
    90                  95                  100

gaa tta ata aaa gga aat tta caa agt gtt gga ctt aca ctt cgt ctt 570
Glu Leu Ile Lys Gly Asn Leu Gln Ser Val Gly Leu Thr Leu Arg Leu
105                 110                 115                 120

gtc cag tca act gat ggg tat gct ggg cac gtc atc att gaa act gtg 618
Val Gln Ser Thr Asp Gly Tyr Ala Gly His Val Ile Ile Glu Thr Val
                125                 130                 135

gct cca aac tcg cct gct gca att gca gat ctt cag cgg gga gat cga 666
Ala Pro Asn Ser Pro Ala Ala Ile Ala Asp Leu Gln Arg Gly Asp Arg
            140                 145                 150

ctt atc gcc att gga ggt gtg aaa atc aca tca aca ctg caa gtg ttg 714
Leu Ile Ala Ile Gly Gly Val Lys Ile Thr Ser Thr Leu Gln Val Leu
        155                 160                 165

aag ctt atc aag cag gct ggt gac cga gtc ctg gtg tac tat gaa agg 762
Lys Leu Ile Lys Gln Ala Gly Asp Arg Val Leu Val Tyr Tyr Glu Arg
    170                 175                 180

cct gtt ggc cag agt aat caa ggt gca gtg ctg caa gat aac ttt ggc 810
Pro Val Gly Gln Ser Asn Gln Gly Ala Val Leu Gln Asp Asn Phe Gly
185                 190                 195                 200

cag ttg gaa gaa aac ttt ttg tca agc tca tgc caa tcg ggt tat gaa 858
Gln Leu Glu Glu Asn Phe Leu Ser Ser Ser Cys Gln Ser Gly Tyr Glu
                205                 210                 215

gag gaa gct gcc ggg ttg aca gta gat act gaa agt aga gag ctg gat 906
Glu Glu Ala Ala Gly Leu Thr Val Asp Thr Glu Ser Arg Glu Leu Asp
            220                 225                 230

tct gaa ttt gaa gac ttg gca agt gat gtc aga gca caa aat gag ttc 954
Ser Glu Phe Glu Asp Leu Ala Ser Asp Val Arg Ala Gln Asn Glu Phe
        235                 240                 245

aaa gat gag gca caa tca tta agt cat agt ccc aaa cgt gtt cca aca 1002
Lys Asp Glu Ala Gln Ser Leu Ser His Ser Pro Lys Arg Val Pro Thr
    250                 255                 260

aca ctt tct att aaa ccc ctt gga gct ata tca cca gtt tta aac cgt 1050
Thr Leu Ser Ile Lys Pro Leu Gly Ala Ile Ser Pro Val Leu Asn Arg

265                 270                 275                 280

aaa tta gct gta gga agt cac cca cta cca ccg aaa att cag tcc aaa    1098
Lys Leu Ala Val Gly Ser His Pro Leu Pro Pro Lys Ile Gln Ser Lys
                285                 290                 295

gat gga aat aaa cct cca ccc cta aaa act tct gag ata aca gac cca    1146
Asp Gly Asn Lys Pro Pro Pro Leu Lys Thr Ser Glu Ile Thr Asp Pro
            300                 305                 310

gca caa gtg tca aaa cca acc caa gga tct gct ttc aaa cca cct gtg    1194
Ala Gln Val Ser Lys Pro Thr Gln Gly Ser Ala Phe Lys Pro Pro Val
        315                 320                 325

cca cca cga cca caa gcg aaa gtt cct ttg cct tcc gcc gat gct cca    1242
Pro Pro Arg Pro Gln Ala Lys Val Pro Leu Pro Ser Ala Asp Ala Pro
    330                 335                 340

aat cag gca gaa cca gat gtt ctc gtt gaa aag cca gag aag gtg gtg    1290
Asn Gln Ala Glu Pro Asp Val Leu Val Glu Lys Pro Glu Lys Val Val
345                 350                 355                 360

cca cct cct ctt gta gat aaa tct gct gaa aag caa gca aaa aat gtg    1338
Pro Pro Pro Leu Val Asp Lys Ser Ala Glu Lys Gln Ala Lys Asn Val
                365                 370                 375

gat gcc ata gac gat gca gct gca cct aag caa ttt tta gca aag caa    1386
Asp Ala Ile Asp Asp Ala Ala Ala Pro Lys Gln Phe Leu Ala Lys Gln
            380                 385                 390

gaa gtg gcc aaa gat gtc act tca gaa act ttc tgc cct act aag gac    1434
Glu Val Ala Lys Asp Val Thr Ser Glu Thr Phe Cys Pro Thr Lys Asp
        395                 400                 405

agt tcg gac gac cgt caa aca tgg gaa tca tca gaa att ctt tat cgt    1482
Ser Ser Asp Asp Arg Gln Thr Trp Glu Ser Ser Glu Ile Leu Tyr Arg
    410                 415                 420

aat aag cta gga aaa tgg aca aga acc aga gca tcc tgt ttg ttt gac    1530
Asn Lys Leu Gly Lys Trp Thr Arg Thr Arg Ala Ser Cys Leu Phe Asp
425                 430                 435                 440

ata gaa gcc tgt cac agg tac tta aac att gca ttg tgg tgc agg gat    1578
Ile Glu Ala Cys His Arg Tyr Leu Asn Ile Ala Leu Trp Cys Arg Asp
                445                 450                 455

cct ttc aag ttg gga ggt ctc atc tgt ttg ggg cat gtt agt tta aaa    1626

Pro Phe Lys Leu Gly Gly Leu Ile Cys Leu Gly His Val Ser Leu Lys
            460                 465                 470

ctt gaa gat gtg gct tta gga tgc cta gct aca tca aac acg gaa tac 1674
Leu Glu Asp Val Ala Leu Gly Cys Leu Ala Thr Ser Asn Thr Glu Tyr
        475                 480                 485

ctt tcc aaa ttg aga ctg gaa gcc ccc tca cct aag gct ata gtc act 1722
Leu Ser Lys Leu Arg Leu Glu Ala Pro Ser Pro Lys Ala Ile Val Thr
    490                 495                 500

aga acc gca cta cgc aat ctg agt atg caa aag gga ttc aat gac aaa 1770
Arg Thr Ala Leu Arg Asn Leu Ser Met Gln Lys Gly Phe Asn Asp Lys
505                 510                 515                 520

ttt tgc tat ggt gac att act att cac ttc aaa tat ttg aaa gaa gga 1818
Phe Cys Tyr Gly Asp Ile Thr Ile His Phe Lys Tyr Leu Lys Glu Gly
                525                 530                 535

gaa tca gac cac cat gta gtt act aac gta gaa aaa gaa aaa gaa ccc 1866
Glu Ser Asp His His Val Val Thr Asn Val Glu Lys Glu Lys Glu Pro
            540                 545                 550

cat ttg gtt gaa gaa gtt tct gtt ctc cct aaa gag gag caa ttt gtt 1914
His Leu Val Glu Glu Val Ser Val Leu Pro Lys Glu Glu Gln Phe Val
        555                 560                 565

gga cag atg ggt tta aca gaa aac aaa cac agt ttt cag gat act cag 1962
Gly Gln Met Gly Leu Thr Glu Asn Lys His Ser Phe Gln Asp Thr Gln
    570                 575                 580

ttc cag aac cca aca tgg tgt gac tac tgt aag aaa aaa gtt tgg act 2010
Phe Gln Asn Pro Thr Trp Cys Asp Tyr Cys Lys Lys Lys Val Trp Thr
585                 590                 595                 600

aaa gca gct tcc cag tgt atg ttt tgt gct tat gtt tgc cat aaa aaa 2058
Lys Ala Ala Ser Gln Cys Met Phe Cys Ala Tyr Val Cys His Lys Lys
                605                 610                 615

tgt caa gaa aag tgt cta gct gag act tct gtt tgt gga gca act gat 2106
Cys Gln Glu Lys Cys Leu Ala Glu Thr Ser Val Cys Gly Ala Thr Asp
            620                 625                 630

agg cga ata gac agg aca ctg aaa aac ctt agg ctg gaa gga cag gaa 2154
Arg Arg Ile Asp Arg Thr Leu Lys Asn Leu Arg Leu Glu Gly Gln Glu
        635                 640                 645

acc ctc tta ggc ctg cct cct cgt gtt gat gct gaa gct agc aag tca 2202
Thr Leu Leu Gly Leu Pro Pro Arg Val Asp Ala Glu Ala Ser Lys Ser
    650                 655                 660

gtc aat aaa aca aca ggt ttg aca agg cat att atc aat act agt tct 2250
Val Asn Lys Thr Thr Gly Leu Thr Arg His Ile Ile Asn Thr Ser Ser
665                 670                 675                 680

cgt tta tta aat ttg cgt caa gtc tct aaa act cgc ctt tct gaa cca 2298
Arg Leu Leu Asn Leu Arg Gln Val Ser Lys Thr Arg Leu Ser Glu Pro
                685                 690                 695

gga acc gat ctc gta gaa cct tca cca aaa cac aca ccc aac acg tca 2346
Gly Thr Asp Leu Val Glu Pro Ser Pro Lys His Thr Pro Asn Thr Ser
            700                 705                 710

gac aac gaa ggc agt gac acg gag gtc tgt ggt cca aac agt cct tct 2394
Asp Asn Glu Gly Ser Asp Thr Glu Val Cys Gly Pro Asn Ser Pro Ser
        715                 720                 725

aaa cgg gga aac agc aca gga ata aag tta gtg aga aaa gag ggt ggt 2442
Lys Arg Gly Asn Ser Thr Gly Ile Lys Leu Val Arg Lys Glu Gly Gly
    730                 735                 740

ctg gat gac agt gtt ttc att gca gtt aaa gaa att ggt cgt gat ctg 2490
Leu Asp Asp Ser Val Phe Ile Ala Val Lys Glu Ile Gly Arg Asp Leu
745                 750                 755                 760

tac agg ggc ttg cct aca gag gaa agg atc cag aaa cta gag ttc atg 2538
Tyr Arg Gly Leu Pro Thr Glu Glu Arg Ile Gln Lys Leu Glu Phe Met
                765                 770                 775

ttg gat aag cta cag aat gaa att gat cag gag ttg gaa cac aat aat 2586
Leu Asp Lys Leu Gln Asn Glu Ile Asp Gln Glu Leu Glu His Asn Asn
            780                 785                 790

tcc ctt gtt aga gaa gaa aaa gag aca act gat aca agg aaa aaa tca 2634
Ser Leu Val Arg Glu Glu Lys Glu Thr Thr Asp Thr Arg Lys Lys Ser
        795                 800                 805

ctt ctt tct gct gcc tta gct aaa tca ggt gaa agg cta caa gct cta 2682
Leu Leu Ser Ala Ala Leu Ala Lys Ser Gly Glu Arg Leu Gln Ala Leu
    810                 815                 820

aca ctt ctt atg att cac tac aga gca ggc att gaa gat ata gaa act 2730
Thr Leu Leu Met Ile His Tyr Arg Ala Gly Ile Glu Asp Ile Glu Thr
825                 830                 835                 840

tta gaa agt ctg tct tta gac cag cac tcc aaa aaa ata agc aag tac 2778
Leu Glu Ser Leu Ser Leu Asp Gln His Ser Lys Lys Ile Ser Lys Tyr
                845                 850                 855

aca gat gat aca gaa gaa gac ctt gat aat gaa ata agc caa cta ata 2826
Thr Asp Asp Thr Glu Glu Asp Leu Asp Asn Glu Ile Ser Gln Leu Ile
            860                 865                 870

gac tct cag cca ttc agc agc ata tca gat gac tta ttt ggc cca tcc 2874
Asp Ser Gln Pro Phe Ser Ser Ile Ser Asp Asp Leu Phe Gly Pro Ser
        875                 880                 885

gag tct gtg tagcagacag gtctatttaa actttcaaat gaacagggta 2923
Glu Ser Val
    890

aagttgcatc taaagtacca cagatacaac catgtttaaa tcctcgtatg cactctggcc 2983

tgcttctcca gttacttgct tgtgtaagaa caaaaatgag aaaggttgtt ttccagtaaa 3043

aacatgacca gcttactaat tggttgtttt ggattgcatt tatagctatg cttttttggg 3103

tttatactgg gaatttattt ttactaaatt atttaacttt tctaattatg taattatgta 3163

agctagcttt tcatgtttat gtatgtatgg tgtccccttg tgttattttt cttcctcttg 3223

gtttttgaat tagtgttaaa tagaatactg tctagattct taaaatattt tcatttccat 3283

catggttata acaaatttgc tgcatgccca aactgacaac agcantcact gagggaacag 3343

gttttgaatc tttcttttgt gttatgaagt ttatcgtctc tacttgcttg agatttttgt 3403

tattttgggg gtttgggggt gctttttgtt ttgtttttgc caaatgtaac atgaaagcag 3463

atgctgcagc tttagtctgt tatgctgatt tagtaaaaaa aaatttttta catatattgc 3523

ttgctttcga tgcttctgtg aaattttttt ctaaagcttt tgtgcagctg tatggtaaaa 3583

atatggtgat taatttgaag agcttacatt gaaagacaat gtaataggaa ataaatgtag 3643

attgcagttg gtcaagaatt ttgtagagag gataacaaga cttaattact gaaaaacagt 3703

aacatagcat tttgaaatat aatcttttaa aatattgatg ctttcctttt aaatggaaat 3763

ttaaatttta taattaaaag tttaaacatt tatgataatt ttcctcatca gttctcccat 3823

aggaaataaa gcatgtgaaa gggt                                          3847

<210> 12
<211> 891
<212> PRT
<213> Homo sapiens

<400> 12
Met Pro Gln Leu Thr Ser Ile Ile Val Asn Gln Leu Lys Lys Ile Ile
1               5                   10                  15

Lys Arg Lys His Thr Leu Pro Asn Tyr Lys Ile Arg Phe Lys Pro Phe
            20                  25                  30

Phe Pro Tyr Gln Thr Leu Gln Gly Phe Glu Glu Asp Glu Glu His Ile
        35                  40                  45

His Ile Gln Gln Trp Ala Leu Thr Glu Gly Arg Leu Lys Val Thr Leu
    50                  55                  60

Leu Glu Cys Ser Arg Leu Leu Ile Phe Gly Ser Tyr Asp Arg Glu Ala
65                  70                  75                  80

Asn Val His Cys Thr Leu Glu Leu Ser Ser Ser Val Trp Glu Glu Lys
                85                  90                  95

Gln Arg Ser Ser Ile Lys Thr Val Glu Leu Ile Lys Gly Asn Leu Gln
            100                 105                 110

Ser Val Gly Leu Thr Leu Arg Leu Val Gln Ser Thr Asp Gly Tyr Ala
        115                 120                 125

Gly His Val Ile Ile Glu Thr Val Ala Pro Asn Ser Pro Ala Ala Ile
    130                 135                 140

Ala Asp Leu Gln Arg Gly Asp Arg Leu Ile Ala Ile Gly Gly Val Lys
145                 150                 155                 160

Ile Thr Ser Thr Leu Gln Val Leu Lys Leu Ile Lys Gln Ala Gly Asp
                165                 170                 175

Arg Val Leu Val Tyr Tyr Glu Arg Pro Val Gly Gln Ser Asn Gln Gly
            180                 185                 190

Ala Val Leu Gln Asp Asn Phe Gly Gln Leu Glu Glu Asn Phe Leu Ser
        195                 200                 205

Ser Ser Cys Gln Ser Gly Tyr Glu Glu Glu Ala Ala Gly Leu Thr Val
    210                 215                 220

Asp Thr Glu Ser Arg Glu Leu Asp Ser Glu Phe Glu Asp Leu Ala Ser
225                 230                 235                 240

Asp Val Arg Ala Gln Asn Glu Phe Lys Asp Glu Ala Gln Ser Leu Ser
                245                 250                 255

His Ser Pro Lys Arg Val Pro Thr Thr Leu Ser Ile Lys Pro Leu Gly
            260                 265                 270

Ala Ile Ser Pro Val Leu Asn Arg Lys Leu Ala Val Gly Ser His Pro
        275                 280                 285

Leu Pro Pro Lys Ile Gln Ser Lys Asp Gly Asn Lys Pro Pro Pro Leu
    290                 295                 300

Lys Thr Ser Glu Ile Thr Asp Pro Ala Gln Val Ser Lys Pro Thr Gln
305                 310                 315                 320

Gly Ser Ala Phe Lys Pro Pro Val Pro Pro Arg Pro Gln Ala Lys Val
                325                 330                 335

Pro Leu Pro Ser Ala Asp Ala Pro Asn Gln Ala Glu Pro Asp Val Leu
            340                 345                 350

Val Glu Lys Pro Glu Lys Val Val Pro Pro Pro Leu Val Asp Lys Ser
        355                 360                 365

Ala Glu Lys Gln Ala Lys Asn Val Asp Ala Ile Asp Asp Ala Ala Ala
    370                 375                 380

Pro Lys Gln Phe Leu Ala Lys Gln Glu Val Ala Lys Asp Val Thr Ser
385                 390                 395                 400

Glu Thr Phe Cys Pro Thr Lys Asp Ser Ser Asp Asp Arg Gln Thr Trp
                405                 410                 415

Glu Ser Ser Glu Ile Leu Tyr Arg Asn Lys Leu Gly Lys Trp Thr Arg
            420                 425                 430

Thr Arg Ala Ser Cys Leu Phe Asp Ile Glu Ala Cys His Arg Tyr Leu
        435                 440                 445

Asn Ile Ala Leu Trp Cys Arg Asp Pro Phe Lys Leu Gly Gly Leu Ile

450                 455                 460

Cys Leu Gly His Val Ser Leu Lys Leu Glu Asp Val Ala Leu Gly Cys
465                 470                 475                 480

Leu Ala Thr Ser Asn Thr Glu Tyr Leu Ser Lys Leu Arg Leu Glu Ala
                485                 490                 495

Pro Ser Pro Lys Ala Ile Val Thr Arg Thr Ala Leu Arg Asn Leu Ser
            500                 505                 510

Met Gln Lys Gly Phe Asn Asp Lys Phe Cys Tyr Gly Asp Ile Thr Ile
        515                 520                 525

His Phe Lys Tyr Leu Lys Glu Gly Glu Ser Asp His His Val Val Thr
    530                 535                 540

Asn Val Glu Lys Glu Lys Glu Pro His Leu Val Glu Glu Val Ser Val
545                 550                 555                 560

Leu Pro Lys Glu Glu Gln Phe Val Gly Gln Met Gly Leu Thr Glu Asn
                565                 570                 575

Lys His Ser Phe Gln Asp Thr Gln Phe Gln Asn Pro Thr Trp Cys Asp
            580                 585                 590

Tyr Cys Lys Lys Lys Val Trp Thr Lys Ala Ala Ser Gln Cys Met Phe
        595                 600                 605

Cys Ala Tyr Val Cys His Lys Lys Cys Gln Glu Lys Cys Leu Ala Glu
    610                 615                 620

Thr Ser Val Cys Gly Ala Thr Asp Arg Arg Ile Asp Arg Thr Leu Lys
625                 630                 635                 640

Asn Leu Arg Leu Glu Gly Gln Glu Thr Leu Leu Gly Leu Pro Pro Arg
                645                 650                 655

Val Asp Ala Glu Ala Ser Lys Ser Val Asn Lys Thr Thr Gly Leu Thr
            660                 665                 670

Arg His Ile Ile Asn Thr Ser Ser Arg Leu Leu Asn Leu Arg Gln Val
        675                 680                 685

Ser Lys Thr Arg Leu Ser Glu Pro Gly Thr Asp Leu Val Glu Pro Ser
    690                 695                 700

Pro Lys His Thr Pro Asn Thr Ser Asp Asn Glu Gly Ser Asp Thr Glu
705                 710                 715                 720

Val Cys Gly Pro Asn Ser Pro Ser Lys Arg Gly Asn Ser Thr Gly Ile
                725                 730                 735

Lys Leu Val Arg Lys Glu Gly Gly Leu Asp Asp Ser Val Phe Ile Ala
            740                 745                 750

Val Lys Glu Ile Gly Arg Asp Leu Tyr Arg Gly Leu Pro Thr Glu Glu
        755                 760                 765

Arg Ile Gln Lys Leu Glu Phe Met Leu Asp Lys Leu Gln Asn Glu Ile
    770                 775                 780

Asp Gln Glu Leu Glu His Asn Asn Ser Leu Val Arg Glu Glu Lys Glu
785                 790                 795                 800

Thr Thr Asp Thr Arg Lys Lys Ser Leu Leu Ser Ala Ala Leu Ala Lys
                805                 810                 815

Ser Gly Glu Arg Leu Gln Ala Leu Thr Leu Leu Met Ile His Tyr Arg
            820                 825                 830

Ala Gly Ile Glu Asp Ile Glu Thr Leu Glu Ser Leu Ser Leu Asp Gln
        835                 840                 845

His Ser Lys Lys Ile Ser Lys Tyr Thr Asp Asp Thr Glu Glu Asp Leu
    850                 855                 860

Asp Asn Glu Ile Ser Gln Leu Ile Asp Ser Gln Pro Phe Ser Ser Ile
865                 870                 875                 880

Ser Asp Asp Leu Phe Gly Pro Ser Glu Ser Val
                885                 890

<210> 13
<211> 20
<212> DNA
<213> Artificial Sequence

<220>
<223> Description of Artificial Sequence:T3

<400> 13

aattaaccct cactaaaggg 20

<210> 14
<211> 22
<212> DNA
<213> Artificial Sequence

<220>
<223> Description of Artificial Sequence:T7

<400> 14
gtaatacgac tcactatagg gc 22

<210> 15
<211> 22
<212> DNA
<213> Artificial Sequence

<220>
<223> Description of Artificial Sequence:KU-GB-2 Sense
Primer

<400> 15
agagcgacct tgagaccaga aa 22

<210> 16
<211> 22
<212> DNA
<213> Artificial Sequence

<220>
<223> Description of Artificial Sequence:KU-GB-2
Antisense Primer

<400> 16
acacaaggca ggagatggaa gt 22

<210> 17
<211> 20
<212> DNA
<213> Artificial Sequence

<220>

<223> Description of Artificial Sequence:KU-GB-5 Sense
      Primer

<400> 17
aaaactcgcc tttctgaacc                                                    20


<210> 18
<211> 20
<212> DNA
<213> Artificial Sequence

<220>
<223> Description of Artificial Sequence:KU-GB-5
      Antisense Primer

<400> 18
agcaagtaac tggagaagca                                                    20


<210> 19
<211> 25
<212> DNA
<213> Artificial Sequence

<220>
<223> Description of Artificial Sequence:Beta-actin
      Sense Primer

<400> 19
gtcgacaacg gctccggcat gtgca                                              25


<210> 20
<211> 25
<212> DNA
<213> Artificial Sequence

<220>
<223> Description of Artificial Sequence:Beta-actin
      Antisense Primer

<400> 20
ggatcttcat gaggtagtca gtcag                                              25

## Description

### Technical Field

The present invention relates to: a glioma antigen or its gene expressing and showing immunoreaction in a human malignant brain tumor, i.e. glioma; a method for preparing said glioma antigen or its gene; a diagnostic drug for detecting a glioma; a detecting and diagnostic probe; an antibody against a glioma antigen and its gene; a screening method for a promoter or a suppressor for the immunity-inducing activity using a glioma antigen; etc.

### Background Art

There has long been expectations on immunotherapies and many attempts have been taken. However, sufficient anti-tumor effects have not been materialized. Cancer immunotherapies have historically been handled with a central focus on the non-specific immunotherapy, however, it has become evident in recent years that a T cell plays an important role at tumor rejection in vivo in human melanoma. Moreover, many efforts have been drawn to the isolation of a T cell recognition tumor antigen which would possibly induce cytotoxic T lymphocyte (CTL) and determining of a MHC Class I -restricted epitope, since when MAGE-1 antigen (Science 254, 1643-7, 1991) was reported by a Belgian group in 1991. The present inventors isolated a CD8+T cell recognition by the CDNA expression cloning method by using a tumor reactive T cell of melanoma (Proc. Natl. Acad. Sci. USA 91, 3515-3519, 1994, Proc. Natl. Acad. Sci. USA 91, 6458-6462, 1994, J. Exp. Med. 180, 347-352, 1994, J. Immunol. 154, 3961-3968, 1995, Immunologic Res. 16, 313-340, 1997), and they reported that an anti-tumor effect was recognized for part of melanoma by this specific immunothoerapy using said antigen (Microbiology Immunology 42, 803-813, 1998, Kawakami, Y., P. F. Robbins, RF. Wang. et al. Identification of Melanoma antigens by T lymphocytes and their use in the immunotherapy of cancer. In Principle and Practice of Oncology. Update. V. DeVita, S. Hellman, S.A.Rosenberg eds. J.B.Lippincott Co. Philadelphia, pl-20, 1996,. Nature Med. 4, 321, 1998). It is further found that the antigen recognized by T cell is a peptide bound to a MHC molecule, which is not necessarily derived from a cell surface protein, and intranuclear or cytoplasm protein can be recognized as an antigen (Immunity 10, 281-7, 1999).

In 1995, groups of Pfreundschuh in Germany and Old et al. in U.S. reported the SEREX method (serolog.ical identification of recombinant cDNA expression cloning; Proc. Natl. Acad. Sci. USA 92, 11810-11813, 1995) for detecting cancer antigen protein which is recognized by an IgG antibody in sera of cancer patients. The method is applicable for cancer wherein the establishment of cell line is difficult, and does not necessarily require a tumor and a CTL. Many tumor antigens have been isolated by applying this method, and it has been reported that this method is also effective for detecting antigens recognized by celluler immunity since antigens such as MAGE-1 and tyrosinase which induce CTL were found to be included in the antigens isolated by this method. It is further reported that the cancer antigens recognized by an IgG antibody of a patient was isolated in melanoma, renal cancer, esophageal cancer, colon cancer, lung cancer, etc., by applying the aforementioned method (Int. J. Cancer 72, 965-971, 1997, Cancer Res. 58, 1034-1041, 1998, Int. J. Cancer 29, 652-658, 1998, Int. J. Oncol. 14, 703-708, 1999, CancerRes. 56, 4766-4772, 1996, Hum. Mol. Genet 6, 33-39, 1997). These antigens include important antigens such as a CT (cancer-testis) antigen (SSX2/HOM-MEL-40, NY-ESO-1, SCP-1, CT7 etc.), a differentiated antigen (galectin-4/NY-CO-27), a mutant antigen (p53 etc.). Among these antigens, HLA-A2-restricted epitope is particularly determined with NY-ESO-1. Further, the antigens isolated by the SEREX method include molecules such as e1F-4γ or HER2/neu which over-express in cancer, and which possibly relate to malignant alteration. There is a possibility that these molecules over-expressing in cancer can be applied not only to a therapy but also to a marker for diagnosis or for possibly projecting recurrence and prognosis.

On the other hand, central nervous system has been known as a venue for immunological tolerance. However, it has recently been appeared that an activated lymphocyte reacts with an intracerebral antigen after transiting through a blood brain barrier and invading into brain (J. Immunol. 158, 2318-26, 1997, J. Neurosci. 18, 5804-16, 1998). The present inventors further indicated the possibility that a specific immunity-inducing against a tumor antigen in a brain could be a new effective therapy method (Neuro-Oncology 1, S105, 1999). Image analyses by MRI et al. and histopathological analyses by using a specimen obtained through an operation are presently focused on for the diagnostic methods for detecting glioma, malignant brain tumor. However, as the genes related to the generation of glioma are becoming evident due to the progress of molecular cell biology in recent years, the possibility of genetic diagnosis wherein said genes are analyzed is also indicated (Glia. 15, 308-27, 1995).

At present it is still the case for cancer, which is currently the primary cause of death, that most of advanced cancers cannot be remedied in spite of the improvement in their mechanisms of generation, diagnostic methods, and therapy methods. In particular, malignant brain tumors are extremely difficult to be remedied, and there has been no effective therapy to date in spite of the improvement in brain surgery operations and radiotherapies in recent years. It is required to develop a new and early diagnostic method and a therapy method in order to improve such situation. The object of the present invention is to provide a human glioma antigen which can be applied to diagnosis and therapy of glioma, a gene encoding such antigen, and a method for preparing the same.

The present inventors have made a keen study to attain the objects mentioned above, and the present inventors constructed cDNA by using mRNA obtained from a glioma cell line, and this CDNA was introduced into λ phage vector to infect Escherichia coli, and by using thus constructed cDNA library (6.0x10⁵ λ phage cDNA clones) derived from a glioma cell line, the present inventors screened the serum of each glioma patient, and found that a glioma antigen which reacts only to the sera of glioma patients and does not react to the sera of healthy persons, and which selectively expresses in cancer cells including a glioma cell line is present, and that an immune system recognizes the protein wherein glioma expresses. Here, the present invention is completed.

### Disclosure of the Invention

The present invention relates to: a method for preparing a glioma antigen and/or a glioma antigen gene, comprising the following processes, (1) a process of constructing λ phage CDNA library comprising the steps of: total RNA is extracted and purified from a glioma cell line; cDNA is synthesized with the purified mRNA, and this cDNA is introduced into λphage vector to infect E. coli, (2) a process of generating a reactive serum by eliminating reactants after applying the extract of E. coli to the diluted serum of a glioma patient, (3) a process of detecting positive clones which are reacted by an antibody in the serum by using a labeled anti-IgG antibody after applying the aforementioned λ phage cDNA library to the reactive serum, (4) a process of confirming antibody-reactivity after repeating several screenings for the detected positive clones, (5) a process of a serum screening using the antigens isolated from the positive clones together with at least the sera of glioma patients and healthy persons (claim 1); a diagnostic drug for detecting glioma comprising an antibody which specifically binds to whole or part of one or more types of glioma antigens obtained by the method for preparing glioma antigens and/or glioma antigen genes described in claim 1, and/or to whole or part of said glioma antigens (claim 2); a detecting and diagnostic probe for glioma comprising whole or part of antisense strand of DNA or RNA encoding one or more types of glioma antigens obtained by the method for preparing glioma antigens and/or glioma antigen genes described in claim 1 (claim 3); an anti-tumor agent with an antibody specifically binding to whole or part of one or more types of glioma antigens obtained by the method for preparing glioma antigens and/or a glioma antigen genes described in claim 1, and/or to whole or part of said glioma antigens as an effective ingredient (claim 4).

The present invention further relates to DNA encoding the following protein (a) or (b), (a) a protein comprising the amino acid sequence shown by SEQ ID NO: 2 (b) a protein which comprises an amino acid sequence wherein one or a few amino acids are deleted, substituted or added in the amino acid sequence shown by SEQ ID NO:2, and which has the immunity-inducing activity (claim 5), DNA comprising the base sequence shown by SEQ ID No: 1 or its complementary sequence and part or whole of these sequences (claim 6); DNA which hybridizes under a stringent condition with DNA of claim 6, and which encodes a protein having the immunity-inducing activity (claim 7); DNA encoding the following protein (a) or (b), (a) a protein comprising the amino acid sequence shown by SEQ ID NO:4, 6, 8, or 10, (b) a protein which comprises an amino acid sequence wherein one or a few amino acids are deleted, substituted or added in the amino acid sequence shown by SEQ ID NO:4, 6, 8, or 10, and which has the immunity-inducing activity (claim 8), DNA comprising the base sequence shown by SEQ ID No: 3. 5, 7, or 9, or its complementary sequence and part or whole of these sequences (claim 9); DNA which hybridizes under a stringent condition with DNA of claim 9, and which encodes a protein having the immunity-inducing activity (claim 10); DNA encoding the following protein (a) or (b), (a) a protein comprising the amino acid sequence shown by SEQ ID NO:12, (b) a protein which comprises an amino acid sequence wherein one or a few amino acids are deleted, substituted or added in the amino acid sequence shown by SEQ ID NO:12, and which has the immunity-inducing activity (claim 11), DNA comprising the base sequence shown by SEQ ID No:11, or its complementary sequence and part or whole of these sequences (claim 12); DNA which hybridizes under a stringent condition with DNA of claim 12, and which encodes a protein having the immunity-inducing activity (claim 13).

The present invention still further relates to: a protein comprising the amino acid sequence shown by SEQ ID NO: 2 (claim 14); a protein which comprises an amino acid sequence wherein one or a few amino acids are deleted, substituted or added in the amino acid sequence shown by SEQ ID NO: 2, and which has the immunity-inducing activity (claim 15); a protein comprising the amino acid sequence shown by SEQ ID NO:4, 6, 8, or 10 (claim 16); and a protein which comprises an amino acid sequence wherein one or a few amino acids are deleted, substituted or added in the amino acid sequence shown by SEQ ID NO: 4, 6, 8, or 10, and which has the immunity-inducing activity (claim 17); a protein comprising the amino acid sequence shown by SEQ ID NO:12 (claim 18); a protein which comprises an amino acid sequence wherein one or a few amino acids are deleted, substituted or added in the amino acid sequence shown by SEQ ID NO:12, and which has the immunity-inducing activity (claim 19); a peptide comprising a partial protein of any of claims 14 to 19, and which has the immunity-inducing activity (claim 20).

The present invention also relates to: a fusion protein or a fusion peptide wherein the protein of any of claims 14 to 19 or the peptide of claim 20, and a marker protein and/or a peptide tag, are bound (claim 21); an antibody against the protein of any of claims 14 to 19 or the peptide of claim 20 (claim 22); a host cell comprising an expression system capable of expressing the protein of any of claims 14 to 19 or the peptide of claim 20 (claim 23); a non-human animal whose gene function to encode the protein of any of claims 14 to 19 or the peptide of claim 20 is deficient on its chromosome or which over-expresses the protein of any of claims 14 to 19 or the peptide of claim 20 (claim 24); a screening method for a promoter or a suppressor for the immunity-inducing activity wherein the immunity-inducing activity in T cell is measured and assessed by using the protein of any of claims 14 to 19, the peptide of claim 20, a test substance, and T cell (claim 25).

### Brief Description of Drawings

Fig. 1 is a drawing showing the result of expression analysis of glioma antigen KU-GB-1 by Northern blot method.
Fig. 2 is a drawing showing the result of expression analysis of glioma antigen KU-GB-2 by RT-PCR.
Fig. 3 is a drawing showing the result of expression analysis of glioma antigen KU-GB-5 by RT-PCR.

### Best Mode of Carrying Out the Invention

A method for preparing a glioma antigen and/or a glioma antigen gene of the present invention is not particularly restricted as long as it includes the following processes, (1) a process of constructing λ phage cDNA library comprising the steps of: total RNA is extracted and purified from a glioma cell line; cDNA is synthesized with the purified mRNA, and this cDNA is introduced into λ phage vector to infect E. coli, (2) a process of generating a reactive serum by eliminating reactants after applying the extract of E. coli to the diluted serum of a glioma patient, (3) a process of detecting positive clones which are reacted by an antibody in the serum by using a labeled anti-IgG antibody after applying the aforementioned λ phage cDNA library to the reactive serum, (4) a process of confirming antibody-reactivity after repeating several screenings for the detected positive clones, (5) a process of a serum screening using the antigens isolated from the positive clones together with at least the sera of glioma patients and healthy persons. However, it is preferable to amplify cDNA insert of the positive clone obtained by the process (4) by PCR, and to determine its sequence, and then to conduct homology search with the use of the existing gene data base. Besides, it is preferable to put serum screening in the process (5) not only on the serum of glioma patients and healthy persons, but also on the serum of other brain disease and other cancer patients.

By way of the aforementioned preparation methods, two groups of antigens i.e. a group of glioma specific antigens (glioma specific antigen genes) reacting only to the sera of glioma patients, and a group of glioma non-specific antigens (glioma non-specific antigen genes) which react to the sera of both glioma and other cancer patients can be obtained. The group of glioma specific antigens (glioma specific antigen genes) is exemplified by novel KU-GB-2, KU-GB-5, L13 together with Cytochrome-C, DEK oncogene, Nuclear Antigen SP100, Sarcolemmal associated protein, KIAA1014 protein, MutL (E. coli) homolog1 (hMLH1) (GenBank Accession No. NM_000249) etc. On the other hand, the group of glioma non-specific antigens (glioma non-specific antigen genes) is exemplified by KU-GB-1, Mitotic centromere associated protein, human S5A (GenBank Accession No.NM_002810, U24704, U51007), or human pUb-R5 (GenBank Accession No.AB033605) as an isoform of human S5A, Minichromosome maintenance deficient-3, etc. Besides, by expressing these glioma antigens either in vivo or in vitro in dendritic cells that are strong antigen-presenting cells, immunity can be induced by injecting these antigen-expressing dendritic cells.

As for a diagnostic drug for detecting glioma of the present invention, a reagent comprising whole or part of one or more types of glioma antigens obtained by the aforementioned preparation method can be used. Part of glioma antigens can be exemplified by an antigen recognition site and the like of glioma antigens. Or when used in combination of more than one antigen, it is preferable to select from the aforementioned glioma specific antigen group or glioma non-specific group. Besides, as for a diagnostic drug for detecting glioma of the present invention, reagents comprising antibodies such as monoclonal antibodies specifically binding to whole or part of the aforementioned glioma antigens and the like can be used. When said antibodies are used in combination of more than one type, it is preferable to select from the antibodies against the aforementioned glioma specific antigen group or the glioma non-specific antigen group. Examples of diagnostic methods for glioma by using a diagnostic drug for detecting glioma of the present invention include: a method of investigating whether antibody reaction can be acknowledged by applying labeled glioma antigens to an IgG antibody of the serum obtained from a specimen; a method of image diagnosis of glioma of which metes and bounds are indefinite by administering into blood labeled antibodies such as fluorescence or Fe to glioma antigens.

There is no particular limitation to a detecting and diagnostic probe for detecting glioma of the present invention, as long as such reagent includes whole or part of antisense strand of DNA or RNA encoding one or more types of glioma antigens obtained by the aforementioned method for preparing glioma antigen genes. However, DNA or RNA comprising more than 20 bp is preferable, and it is also preferable to select from antisense strands of DNA or RNA encoding the aforementioned glioma specific antigen group or glioma non-specific antigen group when used in combination of more than one type of probes. A detecting and diagnostic method of glioma using a detecting and diagnostic probe for glioma of the present invention is exemplified by a method of detecting mRNA of the present glioma antigens obtained from a specimen by using a labeling antisense strands. The specific examples of test samples used for detection and diagnosis are genomic DNA, RNA or cDNA that are obtained from the cells of subjects, for example, biopsy such as blood, urine, saliva, tissues, etc., but the test samples will not be limited to these examples.

As for an anti-tumor agent of the present invention, a formulation comprising whole or part of one or more types of glioma antigens obtained from the aforementioned method for preparing glioma antigens as an effective ingredient can be used. Part of glioma antigen can be exemplified by an antigen recognition site of glioma antigen. And it is preferable to select from the aforementioned glioma specific antigen group or glioma non-specific antigen group when used in combination of more than one type of antigens. A formulation comprising antibodies such as monoclonal antibodies which specifically bind to whole or part of said glioma antigen as an effective ingredient can also be used for anti-tumor agent of the present invention. It is also preferable to select from the antibodies to the aforementioned glioma specific antigen group or glioma non-specific antigen group when more than one type of antibodies are used in combination. When the anti-tumor agents of the present invention are injected orally, intravenously, intradermally, sucutaneously, etc., an anti-tumor effect can be expected due to the increase in the T cell-inducing activity in vivo. Further, T cell can be induced into activated T cell by being stimulated in vitro by using anti-tumor agent of the present invention. For example, tumor reactive activated T cell will be induced when peripheral blood lymphocyte or tumor-infiltrating lymphocyte are stimulated by an anti-tumor agent of the present invention with IL-2, and said activated T cell can be effectively used for adoptive immunotherapy. Genetic therapy wherein genes are selectively introduced into glioma or missile therapy wherein anti-tumor agent is selectively made to act on glioma can be possible by using antibody such as monoclonal antibody of the present invention.

Proteins as objects of the present invention can be exemplified by novel antigen proteins or their isoforms among the glioma antigens obtained by the aforementioned preparation method of glioma antigens. Examples include: glioma KU-GB-1 comprising the amino acid sequence shown by SEQ ID NO:2; four isoforms of glioma KU-GB-2 i.e. glioma KU-GB-2a comprising the amino acid sequence shown by SEQ ID NO:4; glioma KU-GB-2b comprising the amino acid sequence shown by SEQ ID NO: 6 ; glioma KU-GB-2c comprising the amino acid sequence shown by SEQ ID NO:8; glioma KU-GB-2d comprising the amino acid sequence shown by SEQ ID NO:10; glioma KU-GB-5 comprising the amino acid sequence shown by SEQ ID NO:12; and a protein which comprises an the amino acid sequence wherein one or a few amino acids are deleted, substituted or added in the amino acid sequence shown by SEQ ID NO:2, 4, 6, 8, 10, or 12 and which has the immunity-inducing activity. The word immunity-inducing activity used herein stands for the activity inducing immunoreaction such as antibody production, cellular immunity, immunological tolerance, and among said immunity-inducing activity, the ones with the T cell inducing activity which increases the frequency of cytotoxic T cell (CTL) precursor cell in peripheral blood is particularly preferable. A peptide of the present invention can be exemplified by a peptide comprising part of said protein and, which has an immunity-inducing activity. Said peptide is not specifically restricted, but can be eligibly exemplified by a peptide comprising recognition site of antibodies, and a recognition site of CD4 + T cell and/or CD8 + T cell. In addition to the aforementioned proteins and peptides as objects of the present invention, recombinant proteins or peptides which specifically bind to antibodies of said proteins and peptides are also included in the present invention, which may collectively be referred to as "the present glioma antigen". Besides, the origin of the present glioma antigen is not limited to human, and the present glioma antigen is useful for a diagnostic drug for detecting tumor or a screening method for a promoter or a suppressor for the immunity-inducing activity as described hereinafter.

DNA as an object of the present invention is exemplified by DNA encoding novel antigen proteins among the glioma antigens obtained from the aforementioned method for preparing glioma antigens. The specific examples include: DNA encoding the protein KU-GB-1 comprising the amino acid sequence shown by SEQ ID NO: 2; DNA encoding protein KU-GB-2a comprising the amino acid sequence shown by SEQ ID NO:4; DNA encoding protein KU-GB-2b comprising the amino acid sequence shown by SEQ ID NO:6, DNA encoding protein KU-GB-2c comprising the amino acid sequence shown by SEQ ID NO: 8; DNA encoding protein KU-GB-2d comprising the amino acid sequence shown by SEQ ID NO:10; DNA encoding protein KU-GB-5 comprising the amino acid sequence shown by SEQ ID NO.12; and DNA encoding a protein which comprises an amino acid sequence wherein one or a few amino acids are deleted, substituted or added in the amino acid sequence shown by SEQ ID NO:2, 4, 6, 8, 10, or 12, and which has the immunity-inducing activity, and DNA comprising the base sequence shown by SEQ ID No:1, 3, 5, 7, 9, or 11, or its complementary sequence and part or whole of these sequences.

Further, DNA encoding a protein of the interest, which has the same effect as glioma antigens such as KU-GB-1, KU-GB-2a, KU-GB-2b, KU-GB-2C. KU-GB-2d, KU-GB-5 etc., having the immunity-inducing activity can be obtained by the hybridization with a DNA library derived from various glioma cells under a stringent condition by using the base sequence shown by SEQ ID NO:1, 3, 5, 7, 9, or 11 or its complimentary sequence and part or whole of these sequences as a probe, and then by the isolation of DNA which hybridize with the probe. DNA obtained hereby is also within the scope of the present invention. A hybridization condition for obtaining the DNA of the present invention is exemplified by hybridization at 42°C, and washing at 42°C in a buffer solution containing 1 × SSC, 0.1% SDS, and more preferable exemplification is hybridization at 65° C and washing at 65° C in a washing buffer solution containing 0.1 × SSC, 0.1% SDS. There are a number of factors other than the temperature condition mentioned above that affect the hybridization stringency and those skilled in the art can actualize the same stringency as that of the hybridization referred to in the above by appropriately combining various factors.

Besides, a gene or DNA encoding the present glioma antigen mentioned above, and the present glioma antigen and the like can be used for generating a fusion peptide or a fusion protein wherein the present glioma antigen and a marker protein and/or a peptide tag are bound, an antibody against the present glioma antigen, a host cell comprising expression system capable of expressing the present glioma antigen, a non-human animal whose gene function to encode the present glioma antigens is deficient on its chromosome, or a non-human animal over-expressing the present glioma antigen as specifically described hereinafter.

Any fusion protein and fusion peptide may be used as a fusion protein and a fusion peptide for the present invention as long as the present glioma antigens bind to marker proteins and/or peptide tags. As for a marker protein, the specific examples include conventionally known ones such as alkaline phosphatase, the Fc region of an antibody, HRP. GFP, etc. Conventionally known peptide tags including His tag, FLAG tag, S tag, etc. are the specific examples of the peptide tags for the use in the present invention. However, there is no limitation to these examples. These fusion proteins can be generated according to the ordinary protocols and are useful for the following: purification of the glioma antigen KU-GB-1, KU-GB-2a, KU-GB-2b, KU-GB-2c, KU-GB-2d, KU-GB-5 or the like using affinity of Ni-NTA and a His tag; detection of a protein having the T cell-inducing activity; quantification of an antibody against the glioma antigen KU-GB-1, KU-GB-2a, KU-GB-2b,. KU-GB-2c, KU-GB-2d, KU-GB-5 or the like; as a diagnostic marker for cancer such as glioma and the like; as a laboratory reagent in this field of art.

An antibody against the present glioma antigen of the present invention can be particularly exemplified by an immune-specific antibody such as a monoclonal antibody, a polyclonal antibody, a chimeric antibody, a single-stranded antibody, a humanized antibody, etc.These antibodies can be generated according to the ordinary protocols by using the present glioma antigen. However, an monoclonal antibody is more preferable than the other sorts of antibodies mentioned above because of its specificity, and particularly, monoclonal antibodies specifically recognizing epitopes such as KU-GB-1, KU-GB-2a, KU-GB-2b, KU-GB-2c, KU-GB-2d, KU-GB-5 etc., or the complex of epitopes and HLA are more preferable. Such monoclonal antibodies or the like are useful not only for diagnosis of cancers such as glioma and missile therapy, but also for elucidating the development mechanism of malignant tumors such as glioma and the like.

Antibodies of the present invention can be created by administering to an animal (preferably non-human) the present glioma antigen, their fragments containing epitopes, or cells expressing the present glioma antigens, particularly an complex of epitopes and HLA, on the membrane surface, according to the conventional protocols. The monoclonal antibodies can be prepared, for instance, by any optional method such as a hybridoma method that brings antibodies produced by cultured materials of continuous cell line (Nature 256, 495-497, 1975), a trioma method, a human B-cell hybridoma method (Immunology Today 4, 72, 1983), and an EBV-hybridoma method (MONOCLONAL ANTIBODIES AND CANCER THERAPY, pp.77-96, Alan R. Liss, Inc., 1985), etc.

The method for preparing a single chain antibody (US PAT. No. 4,946,778) can be adopted to prepare single-stranded antibodies to the present glioma antigens of the present invention mentioned above. Besides, transgenic mice, other mammals, etc. can be used for expressing humanized antibodies, clones expressing the present glioma antigens can be isolated/identified using the antibodies mentioned above, and their polypeptides can be purified by affinity chromatography. Antibodies to the present glioma antigens or to their peptides containing antigenic epitopes can possibly be used for diagnosis and therapy for glioma etc. Furthermore, recombinant proteins or peptides to which these antibodies specifically bind are also covered by the present glioma antigens of the present invention as described earlier.

A host cell comprising an expression system capable of expressing the present glioma antigens can be constructed by introducing genes encoding the present glioma antigens into the host cell, by the methods described in many standard laboratory manuals such as manuals of Davis et al. (BASIC METHODS IN MOLECULAR BIOLOGY, 1986) and of Sambrook et al. (MOLECULAR CLONING: A LABORATORY MANUAL, 2nd Ed., Cold Spring Harbor Laboratory Press, Cold Spring Harbor, N.Y., 1989), and the specific examples include calcium-phosphate transfection, DEAE-dextran-mediated transfection, transvection, microinjection, cationic lipid-mediated transfection, electroporation, transduction, scrape loading, ballistic introduction, infection, etc.

The examples of host cells mentioned above include bacterial prokaryotic cells such as E.coli, Streptomyces, Bacillus Subtilis, Streptococcus, Staphylococcus, etc., eukaryotic cells such as yeast, aspergillus, etc., insect cells such as Drosophila S2, Spodoptera Sf9, etc., animal cells such as L cell CHO cell, COS cell, HeLa cell, C127 cell, BALB/c3T3 cell (including mutants deficient in dihydrofolate reductase, tymidine kinase, etc.), BHK21 cell, HEK293 cell, and plant cells or the like. Further, a host cell having a HLA-expressing ability, and which comprises an expression system capable of expressing the present glioma antigen can be generated by introducing a gene encoding the present glioma antigen into a host cell having a HLA-expressing ability or a host cell not originally having said ability to those which HLAcDNA was transfected according to the aforementioned methods.

There is no limitation to an expression system as long as the expression system is capable of expressing the present glioma antigens described above in a host cell and the examples include chromosome-, episome- and virus-derived expression systems, for instance, vectors derived from bacterial plasmid, vectors derived yeast plasmid, vectors derived papovavirus such as SV40, vaccinia virus, adenovirus, adeno-associated virus, chicken pox virus, pseudorabies virus, retrovirus, and vectors derived bacteriophage or transposon and vectors derived the combination of these two, e.g. vectors derived from genetic factors of plasmid and bacteriophage, such as cosmid and phagemid. The expression systems may comprise a control sequence for regulating the expression in addition to a sequence for raising the expression.

Host cells comprising the above-mentioned expression systems and the cell membranes of the cells, or the present glioma antigens obtained by culturing the cells can be used in the screening methods of the present invention as described below. For example, the method of F. Pietri-Rouxel et al. (Eur. J. Biochem., 247, 1174-1179, 1997) or the like can be used to obtain cell membranes. Further, to collect and purify the present glioma antigens from the cell culture, the known methods can be adopted including ammonium sulfate- or ethanol-precipitation, acid extraction, anion- or cation-exchange chromatography, phosphocellulose chromatography, hydrophobic interaction chromatography, affinity chromatography, hydroxyapatite chromatography and lectin chromatography, where the high performance liquid chromatography is preferably used. As a column especially used for affinity chromatography, columns to which antibodies to the present glioma antigens are bound, for instance, are used, and when ordinary peptide tags are added to the present glioma antigens mentioned above, columns to which substances having affinity with the peptide tags are bound are used in order to obtain the present glioma antigens.

A non-human animal whose gene function to encode the present glioma antigens of the present invention is deficient on its chromosome means a non-human animal part or whole of whose gene on its chromosome encoding the present glioma antigens is inactivated by gene mutations such as disruption, deletion, replacement, etc., so that whose function to express the present glioma antigens is lost. Further, a non-human animal which over-expresses the present glioma antigens is specifically exemplified by a non-human animal which produces larger amount of the present glioma antigens than a wild-type non-human animal does. Although rodents or the like such as mice, rats, etc. are particularly exemplified for non-human animals of the present invention, the examples will not be limited to these animals only.

Homozygous non-human animals that are born according to Mendel's Law include the deficient type or the over-expressing type for the present glioma antigens as well as their wild type littermates. By using the deficient type animals or the over-expressing type animals of these homozygous non-human animals together with their wild-type littermates at the same time, accurate comparative experiments can be carried out on the individual level. In performing screening of the present invention described below, it is, therefore, preferable to use the wild type non-human animals, i.e. animals of the same species as, or even better the littermates of, non-human animals whose gene function to encode the present glioma antigens is deficient or over-expressing on their chromosomes in parallel with the deficient or over-expressing type animals. The method of producing a non-human animal whose gene function to encode the present glioma antigens is deficient or over-expressing on its chromosome is now explained in the following with the present glioma antigen knockout mouse and the present glioma antigen transgenic mouse.

A mouse, for instance, whose gene function to encode the present glioma antigen is deficient on its chromosome i.e. the present glioma antigen knockout mouse is generated by the following steps. A gene encoding the present glioma antigen is screened by using a gene fragment obtained by a method such as PCR or the like from the mouse gene library. A screened gene which encodes the present glioma antigens is subcloned with a viral vector or the like and is identified by DNA sequencing. Then whole or part of a gene of this clone which encodes the present glioma antigen is substituted with a pMC1 neo gene cassette or the like. A gene such as a diphtheria toxin A fragment (DT-A) gene, a herpes simplex virus tymidine kinase (HSV-tk) gene, etc. is introduced onto the 3'-end, and thus a targeting vector is constructed.

The targeting vectors thus constructed are linearlized and introduced into ES cells by electroporation or the like to cause homologous recombination. Among the homologous recombinants, ES cells in which homologous recombination have occurred are selected by the use of antibiotics such as G418, ganciclovir (GANC), etc. It is preferable to confirm whether the selected ES cells are the recombinants of the interest by Southern blotting or the like. A clone of the ES cells confirmed is microinjected into a mouse blastocyst and which blastocyst is placed back to the recipient mouse to generate a chimeric mouse. A heterozygous mouse can be obtained by intercrossing the chimeric mouse and a wild type mouse. By further intercrossing the heterozygous mice, the present glioma antigen knockout mice of the present invention can be generated. Whether the present glioma antigens knockout mouse is generated is examined by Northern blotting upon isolating RNA from the mouse obtained by the above-described method and by Western blotting or the like with which the expression of the mouse can be directly examined.

The present glioma antigen transgenic mouse is created by the following steps. A promoter such as chicken β-actin, mouse neurofilament, SV40, etc. and poly (A) such as rabbit β-globin, SV40, etc. or introns are fused with cDNA encoding the present glioma antigen to generate a transgene. This transgene is microinjected into the pronucleus of a mouse fertilized egg. After the obtained egg cell is cultured, it is transplanted to the oviduct of the recipient mouse which was fed thereafter. Neonetal mice that have the aforementioned cDNA were selected from among all the mice born and thus the transgenic mice are created. Neonatal mice having the cDNA can be selected by extracting crude DNA from the mice tails or the like and then by a dot hybridization method using a gene encoding the introduced present glioma antigen as a probe and by PCR method or the like using a specific primer.

As for a screening method for a promoter or a suppressor for the immunity-inducing activity of the present invention, it is not specifically restricted as long as the method comprises the measurement and assessment of the immunity-inducing activity of T cell by using the present glioma antigen, a test substance, and T cell. Examples include methods wherein immunity-inducing activity in said T cell is measured and assessed by using the following: a test substance, the present . glioma antigen, and T cell; a test substance, a cell membrane or a cell which expresses the present glioma antigens, and T cell; T cell and a host cell expressing the present glioma antigens and to which vectors expressing HLA are transfected together with vectors expressing a test polypeptide; T cell and a host cell expressing the present glioma antigens which has a HLA-expressing ability and which is transfected with vectors expressing a test polypeptide, and a method wherein immunity-inducing activity in a T cell is measured and assessed on non-human animals by administering a test substance to said non-human animals such as the aforementioned knock-out mice and transgenic mice. Specific examples of the aforementioned cell membrane or a cell include: the primarily cultured cells or the like obtained from non-human animals wherein a gene encoding the present glioma antigen is over-expressing, or wild type of non-human animals; a host cell comprising an expression system capable of expressing the aforementioned present glioma antigens; cell membrane of these cells. The method of contacting a cell membrane or cell and a test substance is exemplified by a method wherein a cell membrane or a cell which expresses the present glioma antigen is cultured in vitro under the presence of a test substance, and subsequently contacted with T cell. A method wherein immunity-inducing activity in a T cell is measured and assessed can be specifically exemplified by a method of measuring which has an amount of IFN γ released from T cell onto culture media as an indicator. Further, a promoter for the immunity-inducing activity obtained by the aforementioned screening method can be applied to the therapy for patients in need of the promotion of immunity-inducing activity. Meanwhile, a suppressor for the immunity-inducing activity can be applied to the therapy for patients in need of the suppression of the immunity-inducing activity.

The present invention will be further specifically explained in the following with reference to the examples, but the technical scope of the invention will not be limited to these examples.

### [Cell lines and tissues]

Human glioma cell line (GI-1, T98G, U87MG, U251), human malignant melanoma cell line (SKmel23, 1362mel, 888mel), lung cancer cell line (LU99, RERF-LC-MA, EBC1), esophageal cancer cell line (TE10), breast cancer cell line (MDA231), pancreatic cancer cell line (PK1), renal cell carcinoma cell line (RCC6), bladder carcinoma cell line (BC47), prostate cancer cell line (PC3), leukemia cell line (HL60, Molt4) are cultured in RPMI comprising 10% of a fetal bovine serum to which penicillin (100IU/ml) and streptomycin (100µg/ml) is added respectively. Pancreatic cancer cell line (KU7) was cultured in RPMI1640 comprising 10% of a fetal bovine serum to which penicillin/streptomycin (1%), L-glutamine (1%), HEPES (1OmM), EGF (6µ/l), insulin (150U/1), hydrocortisone (0.5mg/l), transferrin (1Omg/ are added. Part of lesion extirpated during an operation was applied to a human glioma tissue (GB13 GB16, GB17, GB4). Total RNA of normal tissues (brain, heart, . lung, stomach, colon, liver, spleen, small intestine, renal, testis, placenta, muscle, embryonic brain) were purchased from Clontech.

### [Generating of cDNA library]

Total RNA was isolated from the aforementioned glioma cell line U87-MG and T98G by the cecium chloride ultracentrifuge method. After mixing 1 mg of each cell line, mRNA was purified by way of poly (A) selection using oligotex (dT) 30 (TAKARA). cDNA was synthesized by reverse transcription by using ZAP-cDNA Synthesis Kit (Stratagene) from 5µg of purified mRNA. λ phage cDNA library was constructed by incorporating said cDNA into λ ZAP expressing phagemid vector.

### [Serum Screening of cDNA library]

The cDNA library constructed as above is sprayed onto 150mm NZY agarose plate by 1 × 10⁴ clone, and cultured at 42° C for 4 hours, and then at 37° C for 4 hours. Thereafter, recombinant protein of which expression was induced by 1Om of IPTG on E. coli (XL1-B11ue) was transcribed onto nitro cellulose filter (Hybond-c, Amersham, Buckinghamshire, England). After adsorbed bacteria or phage are removed by washing the filter with TBS comprising 0.05% of TWIN20 (10mM Tris-HC1, 150mM Nacl; pH7.5), non-specific reaction was suppressed by TBS comprising 5% of skimmed milk. This filter was applied to a patient's serum which had been diluted for 100 times or 400 times at 37° C for 4 hours. As for the sera of patients, the ones collected from 12 glioma patients as shown in Table 1, and the ones collected from 8 glioma patients as shown in Table 2 and combined with the sera of 4 glioma patients [ (the sera of the patients G20, G21, G22, G27 were combined), (the sera of the patients G18, G23, G24, G28 were combined)] were applied. These sera were preserved at -80°C, and diluted five times with TBS solution comprising 5% of skimmed milk immediately prior to be used. These sera were further combined with lysate of bacteriolysed E. coli at 1:2 and reacted at 4° C for 8 hours in order to precedently remove the antibody which reacts to bacteria. Its supernatant was ultimately diluted for 100 times (as for the cases of sera of patients shown in Table 1 was used) or 400 times (as for the cases of sera of 4 patients shown in Table 2 was used.).

**Table 1**

| Patients' serum | age | sex | diagnosis | positive clones | different inserts |
|---|---|---|---|---|---|
| G | 48 | M | Glioblastoma | 5 | 4 |
| H | 77 | F | Anaplastic | 4 | 4 |
| | | | oligoastrocytoma | | |
| I | 53 | F | Glioblastoma | 3 | 2 |
| K | 26 | F | Glioblastoma | 18 | 8 |
| | | | Anaplastic | 15 | |
| L | 61 | M | oligoastrocytoma | | 5 |
| N | 32 | F | Anaplastic | 9 | 7 |
| | | | astrocytoma | | |
| O | 62 | F | Glioblastoma | 1 | 1 |
| P | 30 | M | Anaplastic | 6 | 3 |
| | | | astrocytoma | | |
| R | 53 | F | Glioblastoma | 18 | 12 |
| S | 49 | F | Astrocytoma | 8 | 2 |
| U | 61 | F | Glioblastoma | 1 | 1 |
| V | 68 | F | Glioblastoma | 4 | 3 |
| | | | total | 92 | 52 |

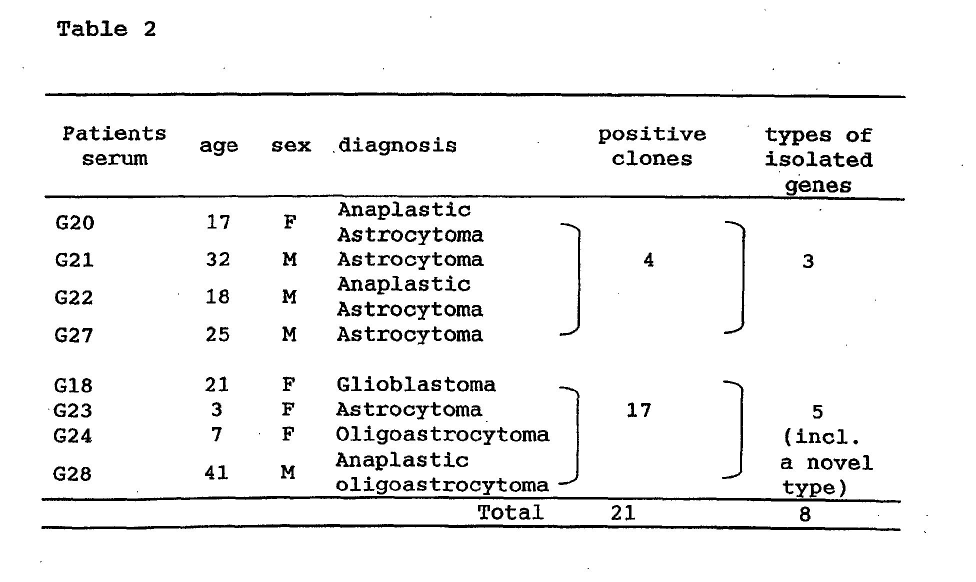

Said processed serum was reacted to recombinant protein blotted onto nitro cellulose filter at 37° C for 4 hours, and then recombinant protein which was reacted by antibodies within a serum was detected by using alkaline phosphatase-labeled anti-human IgG antibody (ICN Pharmaceuticals). Thereafter, positive clones which coincide with coloring reactive positive site was recovered from the aforementioned 150mm NZY agarose plate and dissolved into SM buffering solution ( 100mM of NaCl, 10mM of MgSO₄, 50mM of Tris-HC1, 0.01% of gelatin; ph7.5). This screening was further conducted for several times, and thus antibody reactivity of the positive clones was confirmed. As a result of the screening from 5 × 10⁵ phage clones per serum of one patient, 92 positive clones from the patients' sera shown in Table 1 and 21 positive clones from the patient' sera shown in Table 2 were isolated. Number of isolated positive clones by each patient are shown in Table 1 and 2. Among the 92 positive clones obtained from the patients' sera shown in Table 1, 18 clones were obtained from the patient K of Glioblastoma, 18 clones were from the patient R of Glioblastoma, and 15 clones were from the patient L of Anaplastic oligoastrocytoma. Herein a tendency wherein positive clones are concentrated to the specific patients' sera was acknowledged, however, this tendency appeared to have no apparent relationship with patients' age or malignancy.

### [Homology search of isolated antigen gene]

cDNA inserts which were integrated into 92 phages obtained by using the patients' sera shown in Table 1 and 21 phages obtained by using the patients' sera shown in Table 2 were amplified by PCR method and each base sequence was determined. ExTaq (TAKARA) was used as a reactive enzyme, T3 (5' -AATTAACCCTCACTAAAGGG-3'; SEQ ID NO: 13) was used as a sense primer, and T7 (5'-GTAATACGACTCACTATAGGGC-3' SEQ ID NO:14) was used as an anti-sense primer. Reaction condition was as follows: a cycle of denaturation for 5 minutes at 94°C at the beginning only by using thermal cycler (Perkin Elmer), followed by thermal denaturation for one minute at 94°C, annealing for 1 minute at 55° C, and extension for 2 minutes at 72° C was repeated 35 times, and extension for 7 minutes at 72°C was conducted lastly. Approximately 300-500 bp of the base sequence on 5' end were determined by DNA sequencing of the PCR products obtained herein with applications of Big Dye DNA Sequencing Kit (ABI) and ABI310 auto sequencer. The DNA determined herein was compared with gene information registered in the gene data base of National Center for Biotechnology Information or EST data base, and homology to the existing genes was searched. As a result, 92 positive clones obtained by using patients' sera shown in Table 1 were turned out to be 52 types of antigen cDNA comprising cDNA encoding 38 types of known proteins and cDNA encoding 14 types of new proteins. The number of 52 types of antigen cDNA present in each patient is shown in Table 1. Further, 21 positive clones obtained by using patients' sera shown in Table 2 were turned out to be 8 types of antigen cDNA comprising ODNA encoding 7 types of known proteins and cDNA encoding 1 type of new protein. The number of 8 types of antigen cDNA present in each patient is shown in Table 2.

### [Serum Screening]

In order to consider the application to a serum diagnosis of glioma by using each clone expressing 52 types of antigens isolated by using patients' sera as shown in Table 1, screening of each serum which was diluted for 100 times of the following was raised: 17 glioma patients (Glioblastoma 9, Anaplastic astrocytoma 5, Anaplastic oligoastrocytoma 2, diffuse Astrocytoma 1); for part of antigens, 29 glioma patients (Glioblastoma 12, Anaplastic astrocytoma 7, Anaplastic oligoastrocytoma 4, Astrocytoma 4, Oligoastrocytoma 2); 8 patients of other brain deseases (malignant lymphoma 2, brain abscess 1. meningioma 3, metastatic glioma 1, subarachnoid bleeding 1); 16 patients of other cancer (malignant melanoma 4, pancreatic cancer 4, renal cancer 4, esophageal cancer 4); and 16 healthy persons (26 persons or 54 persons for a part of antigens). It was investigated whether IgG antibody against each isolated glioma antigen can be detected in these sera. As a result, the following antigens were obtained: 7 types of glioma-specific antigens (glioma specific antigen genes) which react exclusively with the sera of glioma patients; 4 types of glioma non-specific antigens (glioma non-specific antigen genes) which react with the sera of glioma patients and patients of other cancers but do not react with sera of other healthy persons. 7 types of glioma specific antigens (glioma specific antigen genes) included Cytochrome-C, DEK oncogene, Nuclear Antigen SP100, Sarcolemmal associated protein, K1AA1014 protein in addition to the novel KU-GB-2, L13 as shown in Table 3. Besides, 4 types of glioma non-specific antigens (glioma non-specific antigen genes) included Mitotic centromere associated protein, human S5A, Minichromosome maintenance deficient-3, in addition to the novel KU-GB-1 as shown in Table 4. The sera of cancer patients other than glioma which react to said glioma non-specific antigens are as follow; melanoma and renal cancer patients' sera for KU-GB-1, pancreatic cancer patients' sera for Mitotic centromere associated protein, esophageal cancer patients' sera for human S5a, melanoma patients' sera for Minichromosome maintenance deficient-3.

**Table 3**

| Antigens (Antigen genes) | Glioma Patients Patients | Healthy Persons | Patients of Other Brain Diseases | Patients of other cancers |
|---|---|---|---|---|
| KU-GB-2 | 5/29 | 0/54 | 0/8 | 0/16 |
| L13 | 2/17 | 0/16 | 0/8 | 0/16 |
| Cytochrome-C | 1/17 | 0/16 | 0/8 | 0/16 |
| DEK oncogene . 1/17 | 1/17 | 0/16 | 0/8 | 0/16 |
| Nuclear Antigen SP100 | 1/17 | 0/16 | 0/8 | 0/16 |
| Sarcolemmal associated protein | 1/17 | 0/16 | 0/8 | 0/16 |
| KIAA1014 protein | 1/17 | 0/16 | 0/8 | 0/16 |

**Table 4**

| Antigens (Antigen genes) | Glioma Patients | Healthy Persons | Patients of Other Brain Diseases | Patients of other cancers |
|---|---|---|---|---|
| | | | | |
| KU-GB-1 | 1/29 | 0/54 | 0/8 | 2/16 |
| Mitotic centromere associated protein | 2/17 | 0/16 | 0/8 | 1/16 |
| human S5a | 3/17 | 0/26 | 0/8 | 1/16 |
| Minichromosome maintenance deficient-3 | 1/17 | 0/16 | 0/4 | 1/16 |

The aforementioned 7 types of glioma specific antigens and 4 types of glioma non-specific antigens are considered to be effective as serum diagnosis products or therapy products for glioma or cancer including glioma, since they do not show an antibody reaction against the sera of other brain disease patients or healthy persons. For example, as in the result of detecting the aforementioned IgG antibody, antibody reaction against human S5a was found in the sera of glioma patients and esophageal cancer patients, but no reaction was found in the sera of other brain disease patients and healthy persons. Therefore, human S5a is considered to be effective as serum diagnosis products or therapy products for cancers including glioma. In addition, this human S5a is registered by 3 types of the names to GenBank [Human proteasome 26S subunit, non-ATPase 4 (PSMD4): Accession No.NM_002810, Antisecretory factory 1: Accession No.U24704, 26S protease subunit S5a:Accession No.U51007]. It is reported (EMBO J.19, 4144-4153,2000) that one type of isoform showing neuro-specific expression is present in this human S5a. There is a high possibility that this isoform i.e. human pUb-R5 (Accession NO.AB033605) is also a glioma antigen as human S5a.

In order to consider the application to serum diagnosis of glioma by using each clone expressing 8 types of antigens isolated by using patients' sera as shown in Table 2, screenings for each serum diluted for 100 times of the following were raised: 29 glioma patients (Glioblastoma 12, Anaplastic astrocytoma 7, Anaplastic oligoastrocytoma 4, Astrocytoma 4, Oligoastrocytoma 2); 14 patients of other brain disease (malignant lymphoma 2, brain abscess 1, subarachnoid bleeding 1, Parkinson's disease 2, meningioma 4, neurocytoma 1, craniopharyngioma 1, metastatic brain tumor 2); and 37 healthy persons. It was investigated whether IgG antibody against each isolated antigen can be detected in these sera in a manner similar to the aforementioned. As a result, 2 types of glioma-specific antigens (glioma specific antigen genes) were found, one of which reacts exclusively with the sera of glioma patients, and the other one of which reacts with most of the sera of glioma patients but showed little reaction with the sera of healthy persons. These 2 types of glioma specific antigens (glioma specific antigen genes) were found to be a novel KU-GB-5 and Mutt as shown in Table 5 (E. coli) homolog1 (hMLH1) (GenBank Accession No.NM_000249). The aforementioned 2 types of glioma-specific antigens are considered to be effective as glioma serum diagnosis products or therapy products because these antigens react specifically with the sera of glioma patients or with the sera of most of glioma patients.

**Table 5**

| Genes | GeneBank GeneBank Accession NO. | Serum Screening | | |
|---|---|---|---|---|
| | | Glioma Patients | Healthy Persons | Patients of Patients of ether Brain |
| KU-GB-5 | None | 1/29 | 0/37 | 0/14 |
| MutL(E.coli) homolog1 (hMLH1) | NM000249 | 5/29 | 1/37 | 0/14 |

### [Determination of base sequence and the amino acid sequence of antigens]

As a result of investigating the base sequence of isolated clones by using the automatic DNA sequencer (ALF Express, Pharmacia Biotech), it was found that KU-GB-1 consisted of the base sequence (SEQ ID NO:1) with the full length of 3709 bp comprising the genes encoding 1120 amino acid sequences (SEQ ID NO: 2). On the contrary, it was found that 4 types of isoforms are present in KU-GB-2. Namely, KU-GB-2a which consists of the base sequence (SEQ ID NO: 3) with the full length of 3533 bp comprising the genes encoding 1154 amino acid sequences (SEQ ID NO:4), together with 3 types of isoforms i.e. KU-GB-2b, KU-GB-2c, and Ku-GB-2d were found. The base sequence and the amino acid sequence of KU-GB-2b are shown respectively in SEQ ID NOS:5 and 6, the base sequence and the amino acid sequence of KU-GB-2c are shown respectively in SEQ ID NOS:7 and.8, the base sequence and the amino acid sequence of KU-GB-2d are shown respectively in SEQ ID NO:9 and 10. It was further found that KU-GB-5 consists of the base sequence (SEQ ID NO: 11) with the full length of 3847 bp comprising the genes encoding 891 amino acid sequences (SEQ ID NO:12).

### [Northern Blot Method for detecting the mRNA expression of glioma antigens]

Northern Blot was conducted for KU-GB-1 by using RNA derived from normal tissues (brain, heart, kidney, spleen, liver, small intestine, lung, testis, placenta, stomach, RNA derived from human glioma cell line (GI-1. U87-MG, T98G), or human glioma tissues (GB13, GB17), RNA derived from the following cell lines: human malignant melanoma cell line (1362mel, 888mel); lung cancer cell line (LU99, RERF-LC-MA); esophageal cancer cell line (TE10); breast cancer cell line (MDA231); pancreatic cancer cell line (PK1); renal cell carcinoma cell line (RCC6); bladder carcinoma cell line (BC47); prostate cancer cell line (PC3); and leukemia cell line (HL60, Molt4). RNA (11µ/g each) was electrophoresed in agarose gel comprising formamid NOe and formaldehyde, and removed into nylon membrane filter (Hybond XL, Amersham).

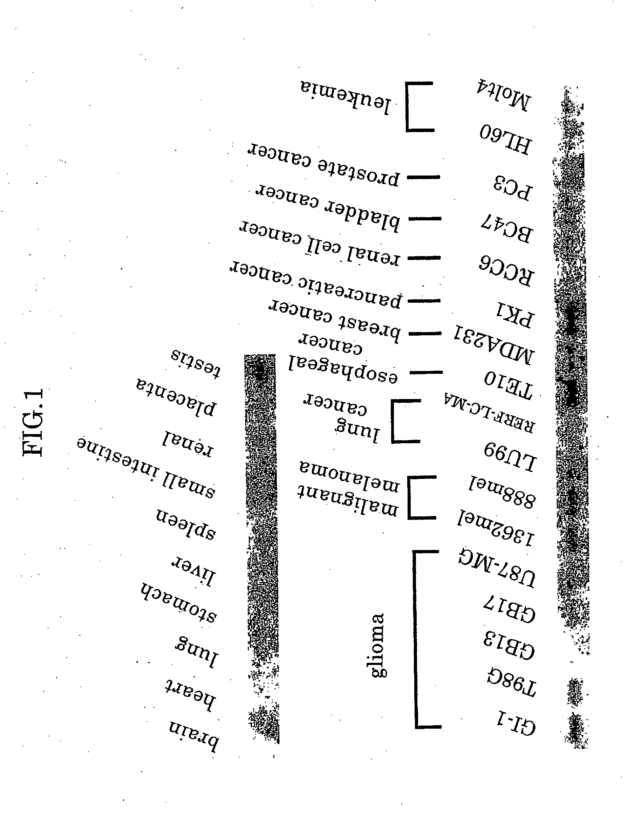

Secondly, a gene fragment comprising KU-GB-1 with the full length of 3709bp was labeled with ³²p as a probe by using High Prime DNA Labeling Kit (Boehringer). Nylon membrane filter was soaked in Quick Hyb (Stratagene) which is used as hyblidization buffer. Pre-hyblidization was conducted at 68° C for 30 minutes. Thereafter, hyblidization was conducted at 68°C for 2 hours in the Quick hyb to which the aforementioned probe was added. And then, said hyblidized nylon membrane filter was washed with the lavage fluid I (2 × SSC, 0 :1% of SDS) at the room temperature for 15 minutes for two times, and then with the lavage fluid II (0.1 × SSC, 0.1% of SDS) at 68°C for 15 minutes for two times. Detecting for radioactive signal was conducted with the washed nylon membrane filter by using Molecular Imager (BIORAD), of which result is shown in Figure 1.

As shown in Figure 1, gene expression of KU-GB-1 in the normal tissues was not found except in testis, but high-level expression was found only in the cancer cell lines including glioma. Therefore, KU-GB-1 gene is considered to be effective as genetic diagnosis products for cancer including glioma. Further, as described above, having considered the fact that antibody reaction against KU-GB-1 antigens was acknowledged with the sera of glioma and other cancer (malignant melanoma, renal cancer) patients, but no antibody reaction against other brain disease patients or healthy persons was acknowledged, KU-GB-1 antigens are considered to be effective as diagnostic drugs or therapy products for cancer including glioma. Regarding the expression in testis, there is considered to be little possibility that a damage of testis could be a problem when KU-GB-1 antigen is practically used for immunotherapy and the like since this tissue is insulated from immune-system. [RT-PCR method for detecting the expression of glioma antigens 1

Subsequently, the expression specificity of glioma antigens KU-GB-2 genes or KU-GB-5 genes were investigated by the RT-PCR method in each normal tissue, glioma cell lines and tissues, and other tumor cell lines. A panel of cDNA as a template of RT-PCR was generated in total reaction amount of 200 µl at 42°C by using each 5 µg of RNA derived from the following; normal tissues such as brain, heart, lung, stomach, liver, pancrea, small intestine, kidney, placenta, testis, colon, muscle, embryonic brain (purchased exclusively from Clontech), 4 types of glioma cell lines (GI-1, U251, U87MG, and T98G), 4 types of glioma tissues (GB13, GB16, GB17, GB4), cell lines such as malignant melanoma cell line (Skmel23), lung cancer cell line (LU99, EBC1), esophageal cancer cell line (TE10). pancreatic cancer cell line (KU7), breast cancer cell line (MDA231), and leukemia cell line (Molt4), and a avian myeloblastosis virus reverse transcriptase (TAKARA) and oligo (dT) as a primer

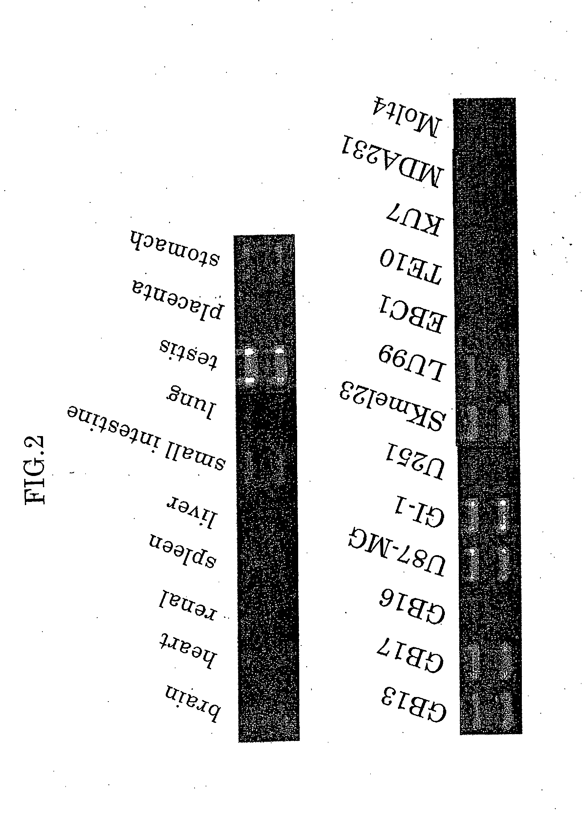

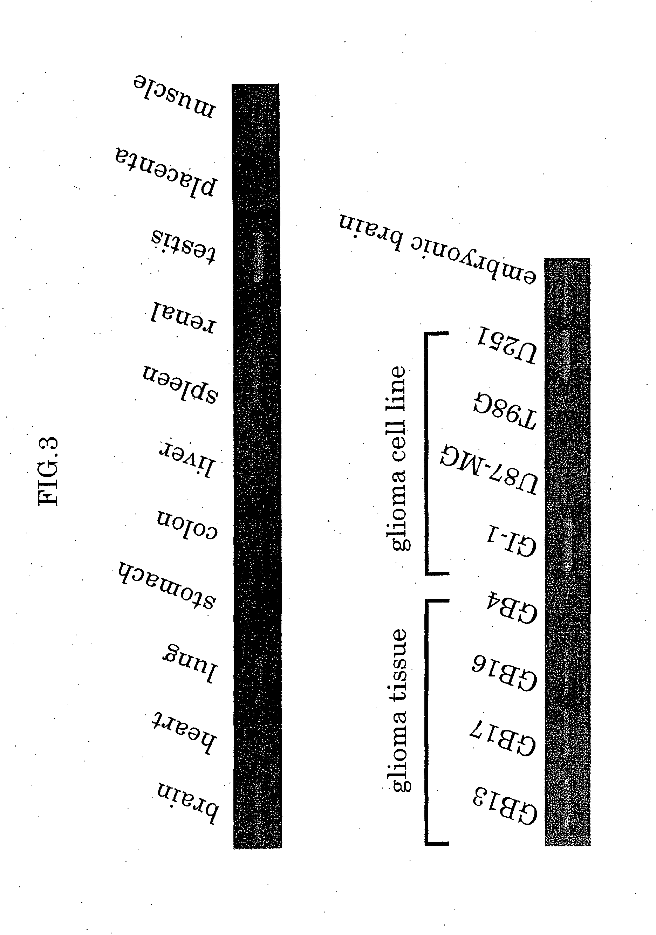

For detecting KU-GB-2, (5'-AGAGCGACCTTGAGACCAGAAA-3' ; SEQ ID NO:15) was used as a sense primer, and (5'-ACACAAGGCAGGAGATGGAAGT-3'; SEQ ID NO:16) was used as an anti-sense primer. For detecting KU-GB-5, (5'-AAAACTCGCCTTTCTGAACC-3'; SEQ ID NO: 17) was used as a sense primer, and (5' -AGCAAGTAACTGGAGAAGCA-3'; SEQ ID NO: 18) was used as an anti-sense primer. For detecting β actin (c551 bp) as a control, (5'-GTCGACAACGCATCCGGCATGTGCA-3'; SEQ ID NO:19) was used as a sense primer, and (5'-GGATCTTCATGAGGTAGTCAGTCAG-3'; SEQ ID NO:20) was used as an anti-sense primer. A cycle wherein a thermal denaturation is conducted at 94° C for one minute by using these PCR primers, EXTaq (TAKARA) as a reactive enzyme, and thermal cycler (Perkin-Elmer), and stretch reacting is held at 72° C for one minute after annealing was repeated for 30 times. Annealing temperature was set at Tm of the primers [62°C for KU-GB-2, 60° C for KU-GB-5, 68° C for β actin as a control], and conducted for 1 minute (30 seconds for KU-GB-5). PCR products obtained herein was electrophoresed in agarose gel (2.0%), and stained by ethidium bromide (EtBr), and then band was detected by ultraviolet irradiation. A result of RT-PCR for KU-GB-2 is shown in Figure 2, and a result of RT-PCR for KU-GB-5 is shown in Figure 3 respectively. As shown in Figure 2, strong expression of KU-GB-2 was found in U87-MG, GI-1 (human glioma cell line), GB13, GB17 (glioma tissue), Skmel23 (malignant melanoma cell line), and testis. Expression was weak in U251 (human glioma cell line), LU99 (lung cancer cell line). brain, small intestine, and stomach. Further, two bands are acknowledged in Figure 2. But as mentioned above, 4 types of isoforms are present in KU-GB-2, of which bigger bands (top lane; 628 bp) show the expression of KU-GB-2b and KU-GB-2d, and smaller bands (bottom lane; 4476 bp) show the expression of KU-GB-2a and KU-GB-2c. And Figure 3 shows that the strong expression of KU-GB-5 was acknowledged in GI-1, U251 (human glioma cell line), GB13, GB17 (glioma cell line), and testis, and weak expression can be seen in U87MG (human glioma cell line), GB16 (glioma tissue), brain, lung, spleen, and embryonic brain.

As shown in Figures.2 and 3, the high-level expression of genes cannot be seen in the normal tissues except for testis but only in glioma or Skmel 23 (malignant melanoma cell line) with KU-GB-2 and KU-GB-5 as is the case with KU-GB-1. Further, RT-PCR analysis was conducted respectively for 4 types of isoform which are present in KU-GB-2 as aforementioned, and as a result, it appeared that KU-GB-2a and KU-GB-2b show a expression pattern which is specific to nervous systems. Judging from these factors, individual genes belong to KU-GB-1 gene are also considered to be effective as genetic diagnosis products for cancer including glioma. Further, as described above, having considered the fact that antibody reaction against KU-GB-2 antigens was acknowledged specifically with the sera of glioma patients but not with the sera of other cancers and other brain disease patients or healthy persons, KU-GB-2 antigens are considered to be effective as diagnostic drugs or therapy products for glioma. And KU-GB-5 antigens are considered to be effective as serum diagnosis products or therapy products for glioma since they specifically react with the sera of glioma patients. Regarding for the expression in testis, there considered to be little possibility that impaired testis could be a problem when KU-GB-2 antigens or KU-GB-5 antigens are practically used for immunotherapy since testis is a tissue which is insulated from immune system.

### Industrial Applicability

A glioma antigen or a glioma antigen gene DNA obtained by a method for preparing a glioma antigen and/or a glioma antigen gene of the present invention is effective as for therapy and diagnosis of cancers such as glioma and the like, and for obtaining the basic knowledge of sideration of glioma.

## Claims

1. A method for preparing a glioma antigen and/or a glioma antigen gene comprising the following processes:
(1) A process of constructing λ phage cDNA library comprising the steps of: total RNA is extracted and purified from a glioma cell line; cDNA is synthesized with the purified mRNA, and this cDNA is introduced into λ phage vector to infect E. coli;
(2) A process of generating a reactive serum by eliminating reactants after applying the extract of E. coli to the diluted serum of a glioma patient;
(3) A process of detecting positive clones which are reacted by an antibody in the serum by using a labeled anti-IgG antibody after applying the aforementioned λ phage cDNA library to the reactive serum;
(4) A process of confirming antibody-reactivity after repeating several screenings for the detected positive clones;
(5) A process of a serum screening using the antigens isolated from the positive clones together with at least the sera of glioma patients and healthy persons.

2. A diagnostic drug for detecting glioma comprising an antibody which specifically binds to whole or part of one or more types of glioma antigens obtained by the method for preparing glioma antigens and/or glioma antigen genes as described in claim 1, and/or to whole or part of said glioma antigens.

3. A detecting and diagnostic probe for glioma comprising whole or part of antisense strand of DNA or RNA encoding one or more types of glioma antigens obtained by the method for preparing glioma antigens and/or glioma antigen genes as described in claim 1.

4. An anti-tumor agent with an antibody specifically binding to whole or part of one or more types of glioma antigens obtained by the method for preparing glioma antigens and/or a glioma antigen genes described in claim 1, and/or to whole or part of said glioma antigens as an effective ingredient.

5. DNA encoding the following protein (a) or (b).
(a) A protein comprising the amino acid sequence shown by SEQ ID NO:2.
(b) A protein which comprises an amino acid sequence wherein one or a few amino acids are deleted, substituted or added in the amino acid sequence shown by SEQ ID NO: 2, and which has the immunity-inducing activity.

6. DNA comprising the base sequence shown by SEQ ID NO:1 or its complementary sequence and part or whole of these sequences.

7. DNA which hybridizes under a stringent condition with DNA of claim 6, and which encodes a protein having the immunity-inducing activity.

8. DNA encoding the following protein (a) or (b).
(a) A protein comprising the amino acid sequence shown by SEQ ID NO:4, 6, 8, or 10.
(b) A protein which comprises an amino acid sequence wherein one or a few amino acids are deleted, substituted or added in the amino acid sequence shown by SEQ ID NO:4, 6, 8, or 10, and which has the immunity-inducing activity.

9. DNA comprising the base sequence shown by SEQ ID NO:3, 5, 7, or 9, or its complementary sequence and part or whole of these sequences.

10. DNA which hybridizes under a stringent condition with DNA of claim 9, and which encodes a protein having the immunity-inducing activity.

11. DNA encoding the following protein (a) or (b).
(a) A protein comprising the amino acid sequence shown by SEQ ID NO:12.
(b) A protein which comprises an amino acid sequence wherein one or a few amino acids are deleted, substituted or added in the amino acid sequence shown by SEQ ID NO:12, and which has the immunity-inducing activity.

12. DNA comprising the base sequence shown by SEQ ID NO:11 or its complementary sequence and part or whole of these sequences.

13. DNA which hybridizes under a stringent condition with DNA of claim 12, and which encodes a protein having the immunity-inducing activity.

14. A protein comprising the amino acid sequence shown by SEQ ID NO:2.

15. A protein which comprises an amino acid sequence wherein one or a few amino acids are deleted, substituted or added in the amino acid sequence shown by SEQ ID NO:2, and which has the immunity-inducing activity.

16. A protein comprising the amino acid sequence shown by SEQ ID NO:4, 6, 8, or 10.

17. A protein which comprises an amino acid sequence wherein one or a few amino acids are deleted, substituted or added in the amino acid sequence shown by SEQ ID NO: 4, 6, 8, or 10, and which has the immunity-inducing activity.

18. A protein comprising the amino acid sequence shown by SEQ ID NO:12.

19. A protein which comprises an amino acid sequence wherein one or a few amino acids are deleted, substituted or added in the amino acid sequence shown by SEQ ID NO:12, and which has the immunity-inducing activity.

20. A peptide comprising a partial protein of any of claims 14 to 19, and which has the immunity-inducing activity.

21. A fusion protein or a fusion peptide wherein the protein of any of claims 14 to 19 or the peptide of claim 20, and a marker protein and/or a peptide tag, are bound.

22. An antibody against the protein of any of claims 14 to 19 or the peptide of claim 20.

23. A host cell comprising an expression system capable of expressing the protein of any of claims 14 to 19 or the peptide of claim 20.

24. A non-human animal whose gene function to encode the protein of any of claims 14 to 19 or the peptide of claim 20 is deficient on its chromosome, or which over-expresses the protein of any of claims 14 to 19 or the peptide of claim 20.

25. A screening method for a promoter or a suppressor for the immunity-inducing activity wherein the immunity-inducing activity in T cell is measured and assessed by using the protein of any of claims 14 to 19 or the peptide of claim 20, a test substance, and T cell.
